# EUROPEAN PATENT APPLICATION

(11) **EP 4 148 046 A1**
(43) Date of publication of application: **15.03.2023**
(21) Application number: 21799652.9
(22) Date of filing: 07.05.2021
(51) Int. Cl.: C07D 237/32, A61K 31/502, A61P 35/00

(54) **NOVEL PHTHALAZINE DERIVATIVE HAVING ECTONUCLEOTIDE PYROPHOSPHATASE/PHOSPHODIESTERASE INHIBITORY ACTIVITY, AND USE THEREOF**

(30) Priority: 08.05.2020 KR 20200055335; 06.05.2021 KR 20210058828
(71) Applicant: Txinno Bioscience Inc., Gyeonggi-do 16942 (KR)
(72) Inventor: HAN, Seo Jung, Seoul 02792 (KR); CHOI, Jun Won, Seongnam-si Gyeonggi-do 13523 (KR); LEE, Sang Hee, Seoul 02792 (KR); PARK, Chan Sun, Seoul 02792 (KR); KIM, Sung Joon, Seoul 02792 (KR); CHEONG, Jae Eun, Seoul 02792 (KR); JEONG, Hee Jin, Seoul 02792 (KR); OH, Do Hee, Seoul 02792 (KR)
(74) Representative: Charrier Rapp & Liebau
(86) International application number: PCT/KR2021/005738
(87) International publication number: WO 2021/225407

(57) **Abstract**

The present invention relates to a compound for inhibiting ENPP1, a composition for inhibiting ENPP1, and a novel phthalazine derivative compound related to a method for inhibiting ENPP1, a pharmaceutically acceptable salt thereof, a hydrate thereof, or a stereoisomer thereof.

## Description

### [Technical Field]

The present invention relates to a compound selected from a novel phthalazine derivative compound having inhibitory activity against ectonucleotide pyrophosphatase-phosphodiesterase (ENPP), a pharmaceutically acceptable salt thereof, a hydrate thereof and a stereoisomer thereof, a method for preparing the compound, and a pharmaceutical composition for preventing, alleviating or treating cancer containing the compound.

### [Background Art]

### [Disclosure]

Cancer is a disease associated with uncontrolled cell proliferation that invades or proliferates to other organs, thus reducing quality of life and eventually leading to death. Although genetic aberrations are a direct cause of uncontrolled proliferation of cancer cells, failure of immune surveillance and/or lack of an adequate immune counterattack of the immune system against cancer cells is another cause of cancer cell proliferation. Also, this leads to the formation of a tumor microenvironment (TME) with suppressed anticancer immunity. Therapeutic agents for treating such fatal diseases may be broadly divided into two categories. The first category directly targets the cancer cells itself, and the second category targets the components of the tumor microenvironment (TME) to prevent further proliferation or survival of cancer cells.

Cancer immunotherapy is a therapeutic approach that targets immune factors present in the TME to cause immune cells to attack tumor cells. In some cases, cancer immunotherapy aims to promote recognition of tumor cells through the release of tumor-associated antigens in the TME (e.g., cancer vaccines). In other cases, cancer immunotherapy aims to promote attack on tumor cells by regulating the activity of innate and/or adaptive immune cells (e.g., immune checkpoint blockade).

Microbial infections cause a variety of diseases all over the world. Pathogenic microorganisms are diverse and include viruses, bacteria, fungi and protozoa. In some cases, a therapeutic agent is a chemical that directly prevents microbial growth. In other cases, therapeutic agents are therapeutic substances for enhancing or stimulating host immune function against pathogenic microorganisms.

The tumor microenvironment (TME) contains malignant tumor cells as well as various types of immune cells (e.g., macrophages, lymphocytes, NK cells and dendritic cells) and non-immune cells (e.g., cancer-associated fibroblasts, pericytes, endothelial cells, and adipocytes). Meanwhile, the presence of tumor-infiltrating lymphocytes has been reported to cause positive clinical outcomes in response to multiple pipelines of immunotherapy in different types of cancer. Preclinical studies reported that modulation or upregulation of other types of immune cells other than lymphocytes, particularly innate immune cells, modulates the responsiveness of anticancer therapies to tumors. The innate immune system is one of the two major components of the host immune defense system in vertebrates. The main functions of innate immunity include 1) to identify and remove foreign substances (e.g., bacteria, viruses) from body tissues, 2) to recruit immune cells to specific sites by producing cytokines and promoting adaptive immune responses, and 3) to activate the complement cascade. Such innate immunity may be activated by recognizing molecular patterns derived from microbial pathogens (pathogen-associated molecular patterns, PAMPs) or residues of destroyed cells (damage-associated molecular patterns, DAMPs).

Pattern recognition receptors (PRRs) are several different types of receptors that are mainly expressed by innate immune cells and can recognize specific PAMPs or DAMPs depending on the ligand specificity thereof. Cytoplasmic DNA is a type of molecular pattern recognized by cytoplasmic DNA sensors (a type of PRR) and triggers an innate immune response. The cGAS-STING pathway (cGAS, cyclic GMP-AMP synthase; STING, stimulator of interferon genes) is involved in 1) cytoplasmic DNA recognition resulting from microbial infection or self-DNA damage and 2) type 1 interferons (IFNs) by generation of chemical factors, mainly activation of the IRES transcription factor.

Type 1 IFN produced by TME due to transformed cancer cells promotes recruitment and activation of inflammatory cells, including NK cells, at the tumor site, and induces both tumor cell death and production of chemoattractants that promote adaptive immune responses.

Systemic administration of type 1 IFN showed verified efficacy in cancer environments based on tumor regression and improved survival rate by systemic injection of IFN-beta in a preclinical mouse model. However, systemic administration of type 1 IFN has a problem in that a high dose is required to reach a therapeutically effective amount to obtain therapeutic efficacy. In this case, tolerancea issues were reported.

Recently published clinical outcomes of exogenous STING agonists (modified cyclic dinucleotides) have shown lower disease control rates than expected despite an apparent increase in pro-inflammatory cytokine production.

Therefore, there is a need for research on novel therapeutic methods that can activate the cGAS-STING pathway.

### [Prior art literature]

### [Patent Literature]

(Patent Document 1) Chinese Patent Publication No. CN 110575458 A
(Patent Document 2) International Publication No. WO 2019/233300 A1
(Patent Document 3) International Publication No. WO 2018/119325 A1
(Patent Document 4) International Publication No. WO 2018/119328 A1
(Patent Document 5) International Publication No. WO 2019/046778 A1
(Patent Document 6) International Publication No. WO 2019/177971 A1
(Patent Document 7) Japanese Patent Publication No. JP 2020-15670
(Patent Document 8) International Publication No. WO 2019/051269 A1
(Patent Document 9) US Patent Publication No. US 2020/0039979 A1
(Patent Document 10) International Publication No. WO 2019/023635 A1
(Patent Document 11) International Publication No. WO 2019/051269 A1

### [Disclosure]

### [Technical Problem]

In order to solve the above problems, the present invention provides a method for increasing the activity of a cGAS-STING pathway.

It is one object of the present invention to provide a novel phthalazine derivative compound having inhibitory activity against ENPP1. It is another aspect of the present invention to provide a novel phthalazine derivative compound for improving and/or modulating the production of type 1 interferon (IFNs) *in vivo.*

It is another object of the present invention to provide a pharmaceutical composition useful for the treatment, prevention and alleviation of cancer containing, as an active ingredient, a novel phthalazine derivative compound, a pharmaceutically acceptable salt thereof, a hydrate thereof, a solvate thereof or a stereoisomer thereof.

It is another object of the present invention to provide a method for preventing, alleviating or treating cancer including administering the novel phthalazine derivative compound or the pharmaceutical composition containing the same to a patient or subject in need thereof.

It is another object of the present invention to provide a method for preventing, alleviating or treating an infectious disease including administering the novel phthalazine derivative compound or the pharmaceutical composition containing the same to a patient or subject in need thereof.

It is another object of the present invention to provide a method for preventing, alleviating or treating periodontal disease including administering the novel phthalazine derivative compound or the pharmaceutical composition containing the same to a patient or subject in need thereof.

It is another object of the present invention to provide a method for preventing, alleviating or treating pathological mineralization of soft tissue including administering the novel phthalazine derivative compound or the pharmaceutical composition containing the same to a patient or subject in need thereof.

It is another object of the present invention to implement the following mechanism as an active ingredient of a novel phthalazine derivative compound, a pharmaceutically acceptable salt thereof, a hydrate thereof, a solvate thereof, or an isomer thereof. In one aspect, the method includes priming cancer with an agent for inducing immunogenic cell death (ICD) prior to stimulation of the cGAS-STING pathway. In another aspect, the method includes blocking degradation of an endogenous STING ligand prior to priming the cancer with the agent for inducing ICD. In another aspect, the method includes the use of an inhibitor for 2',3'-cGAMP degrading polypeptide in combination with the agent for inducing ICD for the treatment of cancer. In one aspect, the present invention provides a method for designing an inhibitor for 2',3'-cGAMP-degrading polypeptide and a detailed assay method for evaluating the enzymatic activity of cGAMP-degrading polypeptide.

### [Technical Solution]

In order to solve the technical problems described above, in one aspect, the present invention provides a compound selected from a phthalazine derivative compound represented by the following Formula 1, a pharmaceutically acceptable salt thereof, a hydrate thereof, a solvate thereof and a stereoisomer thereof: 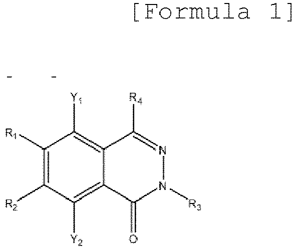 wherein
Y₁ and Y₂ are each independently hydrogen, a hydroxyl group, a halogen group, a C₁-C₁₃ alkyl group, a C₃-C₁₀ cyclic group, a C₁-C₆ alkoxy group, a C₃-C₁₀ cyclic group, or a C₃-C₁₀ heterocyclic group;
R₁ and R₂ are each independently hydrogen, a hydroxyl group, a halogen group, a C₁-C₁₃ alkyl group, a C₁-C₆ alkoxy group, a C₆-C₁₀ aryl group, a C₃-C₁₀ cyclic group, a C₃-C₁₀ heteroaryl group, a C₃-C₁₀ heterocyclic group, or -C(O)-(C₁-C₁₃ alkyl), or R₁, together with a nitrogen atom bonded to R₂, forms a 4- to 7-membered saturated, unsaturated or aromatic ring, which includes at least one of N, O, S, NH, C=N, C=O, -NHC(O)-, -NHC(O)NH-, -NHS(O)₂-, or SO₂, and is substituted with at least one of a C₁-C₁₃ alkyl group, a C₆-C₁₀ aryl group, a C₃-C₁₀ heteroaryl group, a hydroxyl group, a halide group or a cyano group;
R₃ is hydrogen, a hydroxyl group, a halogen group, a C₁-C₁₃ alkyl group, a C₁-C₆ alkoxy group, a C₆-C₁₀ aryl group, a C₃-C₁₀ cyclic group, a C₃-C₁₀ heteroaryl group, a C₃-C₁₀ heterocyclic group, or -C(O)-(C₁-C₁₃ alkyl);
R₄ is hydrogen, a hydroxyl group, a halogen group, a C₁-C₁₃ alkyl group, a C₁-C₆ alkoxy group, a C₁-C₆ alkenyl group, a C₆-C₁₀ aryl group, a C₃-C₁₀ cyclic group, a C₃-C₁₀ heteroaryl group, a C₃-C₁₀ heterocyclic group, an amino group, a nitro group, an amide group, a carboxylic group, a nitrile group, a urea group, a sulfonamide group, a sulfide group, a sulfonic group, or a phosphoryl group,
wherein the C₁-C₆ alkyl group, the C₁-C₁₃ alkyl group or the C₃-C₁₀ cyclic group includes at least one substituent selected from the group consisting of hydrogen, a hydroxyl group, a halogen group, a C₁-C₁₃ alkyl group, a C₁-C₆ alkoxy group, an amino group (-NR₅R₆), a nitro group (-N(O)₂), an amide group (-(C=O)NR₅R₆), a carboxylic group (-C(O)OH), a nitrile group (-CN), a urea group (-NR₅(C=O)NR₆-), a sulfonamide group (-NHS(O)₂-), a sulfide group (-S-), a sulfonyl group (-S(O)₂-), a phosphoryl group (-P(O)R₅R₆), a C₆-C₁₀ aryl group, a C₃-C₁₀ heteroaryl group, and a C₃-C₁₀ heterocyclic group, and
the C₆-C₁₀ aryl group, the C₃-C₁₀ heteroaryl group or the C₃-C₁₀ heterocyclic group includes at least one substituent selected from the group consisting of hydrogen, a hydroxyl group, a halogen group, a carbonyl group (-(C=O)R₅R₆), a C₁-C₃ alkyl group substituted or unsubstituted with halogen or a C₃-C₁₀ heterocyclic group, a C₁-C₃ alkoxy group substituted or unsubstituted with halogen or a C₃-C₁₀ heterocyclic group, C₆-C₁₀ phenoxy, an amino group (-NR₅R₆), a nitro group (-N(O)₂), an amide group (-(C=O)NR₅R₆), a carboxylic group (-C(O)OH), a nitrile group (-CN), a urea group (-NR₅(C=O)NR₆-), a sulfonamide group (-NHS(O)₂-), a sulfide group (-S-), a sulfonyl group (-S(O)₂-), a phosphoryl group (-P(O)R₅R₆), a C₆-C₁₀ aryl group, a C₃-C₁₀ heteroaryl group and a C₃-C₁₀ heterocyclic group; and
R₅ and R₆ are each independently hydrogen, a C₁-C₆ alkyl group, a C₁-C₆ alkenyl group, a C₁-C₆ alkynyl group, a C₆-C₁₀ aryl group, a C₃-C₁₀ heteroaryl group, a C₃-C₁₀ heterocyclic group, or R₅, together with a nitrogen or carbon atom bonded to R₆, forms a 3- to 7-membered saturated ring, which includes at least one of N, O, S, NH, C=N, C=O, -NHC(O)-, -NHC(O)NH-, -NHS(O)₂-, or SO₂, and is substituted with at least one of hydrogen, a C₁-C₁₃ alkyl group, a C₆-C₁₀ aryl group, a C₃-C₁₀ heteroaryl group, a hydroxyl group, a halide group or a cyano group,
wherein the C₃-C₁₀ heteroaryl group and the C₃-C₁₀ heterocyclic group include at least one heteroatom selected from the group consisting of N, O, and S.

### [Advantageous effects]

The compound according to the present invention is capable of effectively inhibiting the activity of ENPP1. Therefore, the compound may be used for the treatment, prevention and alleviation of cancer diseases caused by abnormal cell growth.

The compound according to the present invention, the pharmaceutically acceptable salt or the hydrate thereof, and the pharmaceutical composition for preventing or treating cancer containing the same as an active ingredient effectively inhibit ENPP1 to activate the STING pathway and are thus useful for the prevention or treatment of cancer.

Cancer diseases that can be treated, prevented and alleviated by the administration of the compound according to the present invention include gastric cancer, lung cancer, liver cancer, colorectal cancer, small intestine cancer, pancreatic cancer, brain cancer, bone cancer, melanoma, breast cancer, scleroderma, uterine cancer, cervical cancer, head and neck cancer, esophageal cancer, thyroid cancer, parathyroid cancer, kidney cancer, sarcoma, prostate cancer, urethral cancer, bladder cancer, blood cancer (including leukemia, multiple myeloma, and myelodysplastic syndrome), lymphoma (including Hodgkin's disease and non-Hodgkin's lymphoma), psoriasis, fibroadenoma, and the like.

In particular, the compound according to the present invention is effective in preventing, alleviating or treating diseases involving ENPP 1.

### [Best model

### Definitions

Unless the context clearly indicates otherwise, all numbers, figures and/or expressions that represent ingredients, reaction conditions, polymer compositions, and amounts of mixtures used in the specification are approximations that reflect various uncertainties of measurement occurring inherently in obtaining these figures, among other things. For this reason, it should be understood that, in all cases, the term "about" should be understood to modify all numbers, figures, and/or expressions. In addition, when numerical ranges are disclosed in the description, these ranges are continuous and include all numbers from the minimum to the maximum, including the maximum within each range, unless otherwise defined. Furthermore, when the range refers to an integer, it includes all integers from the minimum to the maximum, including the maximum within the range, unless otherwise defined.

It should be understood that, in the specification, when a range is referred to regarding a parameter, the parameter encompasses all figures including end points disclosed within the range. For example, the range of "5 to 10" includes figures of 5, 6, 7, 8, 9, and 10, as well as arbitrary sub-ranges, such as ranges of 6 to 10, 7 to 10, 6 to 9, and 7 to 9, and any figures, such as 5.5, 6.5, 7.5, 5.5 to 8.5, and 6.5 to 9, between appropriate integers that fall within the range. In addition, for example, the range of "10% to 30%" encompasses all integers, including numbers such as 10%, 11%, 12% and 13% as well as 30%, and any sub-ranges, such as ranges of 10% to 15%, 12% to 18%, or 20% to 30%, as well as any numbers, such as 10.5%, 15.5% and 25.5%, between appropriate integers that fall within the range.

As used herein, the terms "individual(s)," "subject (s)," and "patient (s) " mean any mammal (s) . In some embodiments, the mammal is a human. In some embodiments, the mammal is not a human. Any one of the terms does not require the situation characterized by the supervision (e.g., permanent or intermittent) of health care workers (e.g., physicians, regular nurses, apprentice nurses, physician's assistants, handymen or hospice workers) or is not limited to the situlation.

The term "treatment" is an attempt intended to prevent the development or alteration of a lesion of a disease. Accordingly, the term "treatment" refers to both therapeutic and prophylactic actions. Those in need of treatment include the state in which a disease has developed and the state in which the disease should be prevented. In the treatment of tumors, a therapeutic agent may function to directly reduce the pathology of tumor cells or make the tumor cells more susceptible to treatment through other therapeutic agents, for example, radiation, chemotherapy and/or immunotherapy. As used herein, the term "alleviated" or "treated" means a sign that approaches a normalized value measured by conventional statistical tests. The sign approaching a normalized value is, for example, a value obtained from a healthy patient or individual which has a difference from the normalized value by less than 50%, preferably by less than 25%, and more preferably by less than 10%, and even more preferably has a slight difference from the normalized value.

The term "treatment of cancer" means any one or more of the following effects: 1) inhibition of tumor growth, including i) slowing or ii) complete growth arrest; 2) reduction in the number of tumor cells; 3) maintenance of tumor size; 4) reduction in tumor size; 5) inhibition of infiltration of tumor cells into peripheral organs including i) reduction or ii) slowing or iii) complete prevention; 6) inhibition of metastasis, including i) reduction or ii) slowing or iii) complete prevention; and 7) enhancement of the anti-tumor immune response, which may cause i) maintenance of tumor size, ii) reduction in tumor size, iii) slowing of growth of a tumor, or iv) reduction, slowing or prevention of invasion.

As used herein, the term "effective amount" or "therapeutically effective amount" refers to a sufficient amount of the compound disclosed herein that relieves to some extent the symptoms of a disease or condition to be treated (e.g., cancer or inflammatory disease, periodontal diseases, or mineralization of soft tissue). In some embodiments, the outcome is 1) reduction and/or alleviation of the signs, symptoms or causes of a disease, or 2) any other desirable alteration of a biological system in clinical environments. In some embodiments, an appropriate "effective" amount for any individual case is determined using techniques such as dose escalation studies.

In some embodiments, the term "effective amount" refers to an effective amount to inhibit ENPP1 by about 20% (20% inhibition), at least about 30% (30% inhibition), at least about 40% (40% inhibition), at least about 50% (50% inhibition), at least about 60% (60% inhibition), at least about 70% (70% inhibition), at least about 80% (80% inhibition), or at least about 90% (90% inhibition), upon administration of an amount of the compound disclosed herein, i.e., one or more doses, in a single therapy or combination therapy, when compared to ENPP1 activity in a subject not treated with the compound, or when compared in ENPP1 activity before or after treatment with the compound.

In some embodiments, the term "therapeutically effective amount" refers to an effective amount to reduce a tumor burden of a subject by about 20%, at least about 30%, at least about 40%, at least about 50%, at least about 60%, at least about 70%, at least about 80%, or at least about 90%, upon administration of an amount of the compound disclosed herein, i.e., one or more doses, in a single therapy or combination therapy, when compared to a tumor burden of a subject not treated with the compound, or when compared in the tumor burden of a subject before or after treatment with the compound. As used herein, the term "tumor burden" means the total mass of tumor tissue of a subject with cancer.

In some embodiments, the term "therapeutically effective amount" refers to a dose used for radiotherapy required to observe tumor shrinkage in a subject by about 20%, at least about 30%, at least about 40%, at least about 50%, at least about 60%, at least about 70%, at least about 80%, or at least about 90%, upon administration of an amount of the compound disclosed herein, i.e., one or more doses, in a single therapy or combination therapy, when compared to the dose of radiotherapy required to observe tumor shrinkage in a subject not treated with the compound.

Hereinafter, the present invention will be described in detail.

As a result of continuous research to solve the above problems, the present inventors developed a compound for inhibiting ENPP1, a composition for inhibiting ENPP1, and a method for inhibiting ENPP1. In one aspect, the present inventors developed the novel phthalazine derivative compound for inhibiting ENPP1, a pharmaceutically acceptable salt thereof, a hydrate and a stereoisomer thereof, a method for preparing the same, and a pharmaceutical composition for preventing or treating cancer containing the same as an active ingredient. In one embodiment, the method includes treating a sample with a cell-permeable ENPP1 inhibitor to inhibit cGAMP hydrolysis attributable to ENPP1. In one embodiment, the method includes administering to the patient or subject a therapeutically effective amount of cell-permeable ENPP1 inhibitor to treat cancer. The compound according to one aspect of the present invention, a composition containing the same, or the compound and the composition may be used for various applications or diseases requiring inhibition of ENPP1.

### cGAS-STING pathway, immunogenic apoptosis, and production of type 1 IFNs

Cytoplasmic double-stranded DNA may be introduced from outside cells through microbial infection or vesicular transfer from adjacent dead cells. Cytoplasmic DNA may also be derived from damaged genomic DNA or mitochondrial DNA inside cells. Once cytoplasmic DNA is present, it may be detected by various DNA sensors such as RNA polymerase III, DDX41, DAI, IFI6, cGAS, LEEFIP1, DHX9, DHX36, Ku70 and AIM2.

Cyclic-GMP-AMP synthase (cGAS) is a cytoplasmic protein present as a dimer and consists of two DNA-binding domains and a nucleotidyltransferase domain (that converts ATP and GTP to cyclic dinucleotide 2',3-cGAMP having 2',5' and 3',5' phosphodiesters bonds). In addition, cGAMP acts as a secondary messenger and binds to STING with high affinity (Kd ~ 4 nM) to induce type 1 IFN expression.

STING (also known as TMEM173, MITA, or MPYS) is an endoplasmic reticulum (ER) anchor protein containing four transmembrane domains at the N-terminus and a dimerization domain at the C-terminus. Upon binding to cGAMP, STING forms a tetramer and translocates from the ER to the ER-Golgi intermediate compartment. In the Golgi apparatus, STING attracts and activates tank binding kinase1 (TBK1), the activated TBK1 phosphorylates the C-terminal domain of STING, and STING recruits and activates interferon regulator factor 3 (IRF3). Then, the activated IRF3 migrates to the nucleus and promotes the expression of immune-stimulated genes (ISG) and type 1 IFN. After activation, STING is transferred to endolysosomes and is then degraded. At this time, cGAS-STING pathway activation is terminated.

### Immunogenic cell death (ICD)

Immunogenic cell death (ICD), which is a type of cell death, refers to a cellular phenomenon that induces activation of a controlled immune response. The cell death is characterized by an apoptotic morphology and maintains the integrity of the biological membrane. ICD is also characterized by the secretion of DAMPs (e.g., calreticulin, high mobility group box1 (HMGB1), ATP and Hsp70/90 proteins) as well as exposure of polypeptides formed by native or mutated proteins to the cells. The exposed polypeptides (acting as antigens) are recognized by dendritic cells (DCs) and subsequently prime effector T-cell lymphocytes to activate an adaptive immune response.

ICD may also be further classified based on different types of ICD inducers, such as 1) radiation (e.g., UV radiation or gamma radiation), 2) small chemotherapy molecules (e.g., doxorubicin or paclitaxel), and 3) biological agents (e.g., polypeptides, oligosaccharides, lipids or nucleic acids).

### Radiation therapy

Radiation therapy is well known and used for the treatment of patients suffering from a variety of diseases. Radiation therapy is typically used to inhibit the death or growth of undesirable tissue (e.g., cancerous tissue). The determined amount of high-energy electromagnetic radiation and/or high-energy particles aims to minimize unintended damage to desirable or healthy tissue while directly damaging undesirable tissues or lesions on the path through which the radiation is passed.

The results of research so far showed that, since the effect on normal tissue depends more greatly on the fraction size than the dose, the 1.8-2.0-Gy fractional dose is regarded as the standard of the conventional radiation therapy and thus the treatment time is longer. In fact, a small dose per fraction induces the tumor effect by mitotic death of cancer cells and restores sublethal damage to normal tissues after administration. Stereotactic body radiation therapy (SBRT) is an improved radiation therapy that uses sophisticated image guidance to pinpoint the three-dimensional location of tumors so that radiation can be delivered more accurately to cancer cells. In addition to direct cytotoxicity, SBRT has substantial effects on tumor cell death at high doses accompanying microvascular damage, thus adding a novel mechanism of radiation-induced damage. However, a recent report has shown that high-dose radiation induces the expression of nuclease enzymes to attenuate the effects of radiation on STING-mediated innate immune activation.

### Pathogen

As described above, intracellular invasion of nucleic acids derived from pathogens activates the cGAS-STING pathway, thereby increasing the immune response to pathogens. In some cases, the pathogen is a virus, such as a DNA virus or RNA virus. In some cases, the pathogen is a retrovirus. Examples of viruses that activate the cGAS-STING pathway include, but are not limited to, herpes simplex virus 1 (HSV-1), Kaposi's sarcoma-associated herpesvirus (KSHV), vaccinia virus (VACV), adenovirus, human papilloma virus (HPV), hepatitis B virus (HBV), hepatitis C virus (HCV), dengue virus (DENV), Zika virus (ZIKV), influenza A virus (IAV), human immunodeficiency virus (HIV), or human cytomegalovirus (HCMV). In other embodiments, the pathogen is a bacterium. Examples of bacteria include, but are not limited to, *Listeria monocytogenes, Mycobacterium tuberculosis, Francisella novicida, Legionella pneumophila, Chlamydia trachomatis, Streptococcus pneumoniae,* and *Neisseria gonorrhoeae.*

### Phosphodiesterases

Phosphodiesterases (PDEs) include cyclic nucleotide phosphodiesterases, phospholipases C and D, autotaxins, sphingomyelin phosphodiesterases, DNases, RNases, restriction endonucleases, and a number of less well known small molecule phosphodiesterases. A group of examplary PDE enzymes are important for hydrolysis of the cyclic nucleotides adenosine 3',5'-cyclic monophosphate (cAMP) and guanosine 3',5'-cyclic monophosphate (cGMP) into inactive 5' monophosphate thereof.

Cyclic nucleotide phosphodiesterases contain a group of enzymes that cleave phosphodiester bonds of the cyclic nucleotide secondary messenger molecules, namely, cAMP and cGMP. They regulate the localization, persistence and amplification of cyclic nucleotide signals in subcellular domains.

### Ecto-nucleotide pyrophosphatases/phosphodiesterase

The class of phosphodiesterases also includes ectonucleotide pyrophosphatases/phosphodiesterases. Ectonucleotide pyrophosphatases/phosphodiesterase (ENPP) or nucleotide pyrophosphatase/phosphodiesterase (NPP) is a group of enzymes of ectonucleotidases that hydrolyze the pyrophosphate and phosphodiester bonds of substrates thereof to form nucleotide 5'-monophosphates (or phospholipids and phosphocholines). In some embodiments, the ENPP enzyme group includes seven enzymes of ectonucleotidases (ENPP-1, ENPP-2, ENPP-3, ENPP-4, ENPP- 5, ENPP-6 and ENPP-7) having a similar protein structure on the cell surface.

ENPP enzymes usually have a modular structure including a catalytic domain of 400 amino acids. This catalytic domain is not related to phospholipase, Nudix hydrolase or ectonucleoside triphosphate diphosphohydrolase, although it exhibits partially overlapping activities. ENPPs 1 and 3 are predicted to have a transmembrane domain at the N-terminus thereof and a nuclease-like domain at the C-terminus thereof and to be type 2 single-spanning transmembrane proteins whose catalytic domains are directed towards the extracellular domain. ENPP 2, which lacks a transmembrane domain at either the N- or C-terminus thereof, is expected to have a signal peptide at the N-terminus thereof and to be secreted extracellularly. ENPPs 4, 5, 6, and 7 containing putative N-terminal signal peptides and C-terminal transmembrane domains are also predicted to be type 1 single-spanning transmembrane proteins whose catalytic domains are directed to the extracellular domain.

ENPPs 1, 2 and 3 are known to generate nucleoside monophosphates (ENPPs 1, 2 and 3) or nucleoside diphosphates (ENPPs 1 and 2) using nucleotides and derivatives thereof as substrates. It is known that only ENPP2 uses lysophospholipids. ENPPs 6 and 7 are known to produce choline phosphates using choline phosphate esters as substrates. For ENPPs 4 and 5, there is no known substrate.

ENPP1, which is also called NPP1 or PC-1, is a type 2 transmembrane glycoprotein and is expressed in a veriaty of tissues (pancreas, kidney, bladder and liver). ENPP1 is essential for purinergic signaling, which plays an important role in the regulation of cardiovascular, neuronal, immune and blood functions in mammals. ENPP1 catalyzes the hydrolysis of ATP or GTP to AMP or GMP to produce inorganic pyrophosphate (PPi). In general, since inorganic pyrophosphate regulates the mineralization of bone and cartilage, the production of PPi by ENPP1 makes ENPP1 a central regulator of bone and cartilage development. In contrast to the inhibitory effect of excessive PPi produced by ENPP1 in joint tissue, formation of calcium phosphate from PPi produced by ENPP1 is essential for mineralization of bone tissue. ENPP1 has broad specificity and hydrolyzes a variety of substrates, including phosphodiester linkages of nucleotides and nucleotide sugars and pyrophosphate linkages of nucleotides and nucleotide sugars.

Recently, ENPP1 has been found to play an important role in the immunological response to various extrinsic signals that activate the cGAS-STING pathway. An exploratory study of enzymatic activity degrading cGAMP molecules revealed that ENPP1 functions as a major hydrolase of cGAMP. Consistent with these findings, it was reported that the half-life of cGAMP was greatly dependent on ENPP1 based on verification of a much longer cGAMP half-life in ENPP1 knockout mice.

The bisphosphothionate analogue of cGAMP, which is resistant to ENPP1 hydrolysis, was shown to activate STING 10-fold more than cGAMP, which indicates that delay or reduction of cGAMP hydrolysis by inhibition of ENPP1 can greatly increase the activation of STING. It has been reported that inhibition of ENPP1 induces the presence of persistent cGAMP to activate the STING pathway, thereby attenuating pseudorabies virus infection and reducing *Mycobacterium tuberculosis* infection.

Accordingly, in one aspect, the present invention provides an inhibitor of ENPP1, which is a cGAMP-degrading polypeptide.

In one aspect, the ENPP1 inhibitor is a reversible inhibitor.

In one aspect, the ENPP1 inhibitor is a competitive inhibitor.

In one aspect, the ENPP1 inhibitor is an allosteric inhibitor.

In one aspect, the ENPP1 inhibitor is an irreversible inhibitor.

In one aspect, the inhibitor of ENPP1 binds to a phosphodiesterase (PDE) catalytic domain to which AMP or GMP is bound.

In one aspect, the inhibitor of ENPP1 binds to the PDE catalytic domain but weakly binds thereto, when AMP is bound thereto.

In another aspect, the inhibitor of ENPP1 does not inhibit the ATP hydrolytic activity of the catalytic domain or weakly inhibits the ATP hydrolytic activity thereof.

### Method for inhibiting ENPP1

As mentioned above, the present invention includes the following configurations: 1) an ENPP1 inhibitor; 2) a method of inhibiting an ENPP1 enzyme using the ENPP1 inhibitor; 3) a method of inhibiting the hydrolase activity of ENPP1 to cGAMP; 4) a method of improving the signal output of STING path activation; and 5) a method of inhibiting tumor growth in an appropriate mouse tumor model in a single or combination therapy setting.

In some embodiments, inhibition of ENPP1 means that the activity of ENPP1 is reduced by 10% or greater, for example 20% or greater, 30% or greater, 40% or greater, 50% or greater, 60% or greater, 70% or greater, 80% or greater, 90% or greater, or 95% or greater, compared to a control not treated with the compound. In some embodiments, inhibition of ENPP1 means that the activity of ENPP1 is reduced by at least 2 times, for example at least 3 times, at least 5 times, at least 10 times, at least 100 times, or at least 1,000 times, compared to a control not treated with the compound.

In some embodiments, the cell-permeable ENPP1 inhibitor is an inhibitor as described herein. In some embodiments, the cell-penetrable ENPP1 inhibitor is an inhibitor for any one compound selected from the phthalazine derivative compound represented by Formula 1, the pharmaceutically acceptable salt thereof, the hydrate thereof, and the stereoisomer thereof.

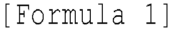

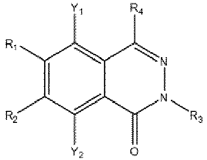

In some embodiments, the cell-permeable ENPP1 inhibitor is any one of the following compounds:
Compound No. 1: 4-phenylphthalazin-1(2H)-one;
Compound No. 2: tert-butyl (3-(4-oxo-3,4-dihydrophthalazin-1-yl)phenyl)carbamate;
Compound No. 3: tert-butyl (3(3-methyl-4-oxo-3,4-dihydrophthalazin-1-yl)phenyl)carbamate;
Compound No. 4: tert-butyl (3(3-benzyl-4-oxo-3,4-dihydrophthalazin-1-yl)phenyl)carbamate;
Compound No. 5: tert-butyl(3-(4-oxo-3,4-dihydrophthalazin-1-yl)carbamate;
Compound No. 6: tert-butyl 3-(3-methyl-4-oxo-3,4-dihydrophthalazin-1-yl)benzyl carbamate;
Compound No. 7: tert-butyl (3-(3-benzyl-4-oxo-3,4-dihydrophthalazin-1-yl)benzyl)carbamate;
Compound No. 8: tert-butyl (4-(4-oxo-3,4-dihydrophthalazin-1-yl)benzyl)carbamate; Compound No. 9: tert-butyl (4-3-methyl-4-oxo-3,4-dihydrophthalazin-1-yl)benzyl)carbamate;
Compound No. 10: tert-butyl (4-(3-ethyl-4-oxo-3,4-dihydrophthalazin-1-yl)benzyl)carbamate;
Compound No. 11: tert-butyl 4-(3-isopropyl-4-oxo-3,4-dihydrophthalazin-1-yl)benzyl carbamate;
Compound No. 12: tert-butyl (4-(4-oxo-3-phenyl-3,4-dihydrophthalazin-1-yl)benzyl)carbamate;
Compound No. 13: tert-butyl (4-3-benzyl-4-oxo-3,4-dihydrophthalazin-1-yl)benzyl)carbamate;
Compound No. 14: tert-butyl (4-(3-(4-fluorobenzyl)-4-oxo-3,4-dihydrophthalazin-1-yl)benzyl)carbamate;
Compound No. 15: tert-butyl (4-(7-methoxy-4-oxo-3,4-dihydrophthalazin-1-yl)benzyl)carbamate;
Compound No. 16: tert-butyl 4-(7-methoxy-3-methyl-4-oxo-3,4-dihydrophthalazin-1-yl)benzyl carbamate;
Compound No. 17: tert-butyl 4-(6-methoxy-4-oxo-3,4-dihydrophthalazin-1-yl)benzyl carbamate;
Compound No. 18: tert-butyl (4-(6-methoxy-3-methyl-4-oxo-3,4-dihydrophthalazin-1-yl)benzyl)carbamate; Compound No. 19: tert-butyl (4-(6-methoxy-4-oxo-3,4-dihydrophthalazin-1-yl)benzyl)carbamate;
Compound No. 20: tert-butyl (4-(6,7-dimethoxy-3-methyl-4-oxo-3,4-dihydrophthalazin-1-yl)benzyl)carbamate;
Compound No. 21: tert-butyl (1-(4-(4-oxo-3,4-dihydrophthalazin-1-yl)phenyl)ethyl)carbamate;
Compound No. 22: tert-butyl (R)-(1-(4-(4-oxo-3,4-dihydrophthalazin-1-yl)phenyl)ethyl)carbamate;
Compound No. 23: tert-butyl (2-(4-(4-oxo-3,4-dihydrophthalazin-1-yl)phenyl)propan-2-yl)carbamate;
Compound No. 24: tert-butyl 4-(4-oxo-3,4-dihydrophthalazin-1-yl)-2-(trifluoromethyl)benzyl carbamate;
Compound No. 25: tert-butyl methyl(4-(4-oxo-3,4-dihydrophthalazin-1-yl)benzyl)carbamate;
Compound No. 26: tert-butyl 6-(4-oxo-3,4-dihydrophthalazin-1-yl)-3,4-dihydroisoquinoline-2(1H)-carboxylate;
Compound No. 27: tert-butyl-6-(3-methyl-4-oxo-3,4-dihydrophthalazin-1-yl)-3,4-dihydroisoquinoline-2(1H)-carboxylate;
Compound No. 28: tert-butyl 6-(6,7-dimethoxy-4-oxo-3,4-dihydrophthalazin-1-yl)-3,4-dihydroisoquinoline-2(1H)-carboxylate;
Compound No. 29: tert-butyl-6-(6,7-dimethoxy-3-methyl-4-oxo-3,4-dihydrophthalazin-1-yl)-3,4-dihydroisoquinoline-2(1H)-carboxylate;
Compound No. 30: 4-(3-aminophenyl)phthalazin-1(2H)-one hydrochloride;
Compound No. 31: 4-(3-aminophenyl)-2-methylphthalazin-1(2H)-one hydrochloride;
Compound No. 32: 4-(3-aminophenyl)-2-benzylphthalazin-1(2H)-one hydrochloride;
Compound No. 33: 4-(3-(aminomethyl)phenyl)phthalazin-1(2H)-one hydrochloride;
Compound No. 34: 4-(3-(aminomethyl)phenyl)-2-methylphthalazin-1-(2H)-one hydrochloride;
Compound No. 35: 4-(3-(aminomethyl)phenyl)-2-benzylphthalazin-1(2H)-one hydrochloride;
Compound No. 36: 4-(4-(aminomethyl)phenyl)phthalazin-1(2H)-one hydrochloride;
Compound No. 37: 4-(4-aminomethyl)phenyl)-2-methylphthalazin-1(2H)-one hydrochloride;
Compound No. 38: 4-(4-(aminomethyl)phenyl)-2-ethylphthalazin-1(2H)-one hydrochloride;
Compound No. 39: 4-(4-(aminomethyl)phenyl)-2-isopropylphthalazin-1-(2H)-one hydrochloride;
Compound No. 40: 4-(4-aminomethyl)phenyl)-2-phenylphthalazin-1(2H)-one hydrochloride;
Compound No. 41: 4-(4-aminomethyl)phenyl)-2-benzylphthalazin-1(2H)-one hydrochloride;
Compound No. 42: 4-(4-(aminomethyl)phenyl)-2-(4-fluorobenzyl)phthalazin-1(2H)-one hydrochloride;
Compound No. 43: 4-(4-(aminomethyl)phenyl)-6-methoxyphthalazin-1(2H)-one hydrochloride;
Compound No. 44: (4-(7-methoxy-3-methyl-4-oxo-3,4-dihydrophthalazin-1-yl)phenyl)methanaminium chloride;
Compound No. 45: (4-(6-methoxy-4-oxo-3,4-dihydrophthalazin-1-yl)phenyl)methanaminium chloride;
Compound No. 46: 4-(4-(aminomethyl)phenyl)-7-methoxy-2-methylphthalazin-1(2H)-one hydrochloride;
Compound No. 47: 4-(4-(aminomethyl)-phenyl)-7-methoxyphthalazin-1(2H)-one hydrochloride;
Compound No. 48: 4-(4-(aminomethyl)phenyl)-6,7-dimethoxyphthalazin-1(2H)-one hydrochloride;
Compound No. 49: 4-(4-(aminomethyl)phenyl)-6,7-dimethoxy-2-methylphthalazin-1(2H)-one hydrochloride;
Compound No. 50: 4-(4-(1-aminoethyl)phenyl)phthalazin-1(2H)-one hydrochloride;
Compound No. 51: (R)-4-(4-(1-aminoethyl)phenyl)phthalazin-1(2H)-one hydrochloride;
Compound No. 52: 4-(4-(2-aminopropan-2-yl)phenyl)phthalazin-1(2H)-one hydrochloride;
Compound No. 53: 4-(4-(aminomethyl)-3-(trifluoromethyl)phenyl)phthalazin-1(2H)-one hydrochloride;
Compound No. 54: 4-(4-((methylamino)methyl)phenyl)phthalazin-1(2H)-one hydrochloride;
Compound No. 55: 4-(1,2,3,4-tetrahydroisoquinolin-6-yl)phthalazin-1(2H)-one hydrochloride;
Compound No. 56: 2-methyl-4-(1,2,3,4-tetrahydroisoquinolin-6-yl)phthalazin-1(2H)-one hydrochloride;
Compound No. 57: 6,7-dimethoxy-4-(1,2,3,4-tetrahydroisoquinolin-6-yl)phthalazin-1(2H)-one hydrochloride;
Compound No. 58: 6,7-dimethoxy-2-methyl-4-(1,2,3,4-tetrahydroisoquinolin-6-yl)phthalazin-1(2H)-one hydrochloride;
Compound No. 59: 6,7-dimethoxy-4-(1,2,3,4-tetrahydroisoquinolin-7-yl) phthalazin-1(2H)-one;
Compound No. 60: tert-butyl (N-(3-(4-oxo-3,4-dihydrophthalazin-1-yl)phenyl)sulfamoyl)carbamate;
Compound No. 61: tert-butyl (N-(3-(3-methyl-4-oxo-3,4-dihydrophthalazin-1-yl)phenyl)sulfamoyl)carbamate;
Compound No. 62: tert-butyl (N-(3-(3-benzyl-4-oxo-3,4-dihydrophthalazin-1-yl)phenyl)sulfamoyl)carbamate;
Compound No. 63: tert-butyl (N-(3-(4-oxo-3,4-dihydrophthalazin-1-yl)benzyl)sulfamoyl)carbamate;
Compound No. 64: tert-butyl N-(3-(3-methyl-4-oxo-3,4-dihydrophthalazin-1-yl)benzyl)sulfamoyl carbamate;
Compound No. 65: tert-butyl (N-(3-(3-benzyl-4-oxo-3,4-dihydrophthalazin-1-yl)benzyl)sulfamoyl)carbamate;
Compound No. 66: tert-butyl (N-(4-(3-methyl-4-oxo-3,4-dihydrophthalazin-1-yl)benzyl)sulfamoyl)carbamate;
Compound No. 67: tert-butyl (N-(4-(3-ethyl-4-oxo-3,4-dihydrophthalazin-1-yl)benzyl)sulfamoyl)carbamate;
Compound No. 68: tert-butyl N-(4-(3-isopropyl-4-oxo-3,4-dihydrophthalazin-1-yl)benzyl)sulfamoyl carbamate; Compound No. 69: tert-butyl (N-(4-(4-oxo-3-phenyl-3,4-dihydrophthalazin-1-yl)benzyl)sulfamoyl)carbamate;
Compound No. 70: tert-butyl (N-(4-3-benzyl-4-oxo-3,4-dihydrophthalazin-1-yl)benzyl)sulfamoyl)carbamate;
Compound No. 71: tert-butyl (N-(4-(3-(4-fluorobenzyl)-4-oxo-3,4-dihydrophthalazin-1-yl)benzyl)sulfamoyl)carbamate;
Compound No. 72: tert-butyl (4-(7-methoxy-4-oxo-3,4-dihydrophthalazin-1-yl)benzyl)sulfamoyl)carbamate;
Compound No. 73: tert-butyl N-(4-(7-methoxy-3-methyl-4-oxo-3,4-dihydrophthalazin-1-yl)benzyl)sulfamoyl carbamate;
Compound No. 74: tert-butyl N-(4-(6-methoxy-4-oxo-3,4-dihydrophthalazin-1-yl)benzyl)sulfamoyl carbamate;
Compound No. 75: tert-butyl (N-(4-(6-methoxy-3-methyl-4-oxo-3,4-dihydrophthalazin-1-yl)benzyl)sulfamoyl)carbamate;
Compound No. 76: tert-butyl (N-(4-(6,7-dimethoxy-4-oxo-3,4-dihydrophthalazin-1-yl)benzyl)sulfamoyl)carbamate;
Compound No. 77: tert-butyl (N-(4-(6,7-dimethoxy-3-methyl-4-oxo-3,4-dihydrophthalazin-1-yl)benzyl)sulfamoyl)carbamate;
Compound No. 78: tert-butyl (N-(1-(4-(4-oxo-3,4-dihydrophthalazin-1-yl)phenyl)ethyl)sulfamoyl)carbamate;
Compound No. 79: tert-butyl (R)-(N-(1-(4-(4-oxo-3,4-dihydrophthalazin-1-yl)phenyl)ethyl)sulfamoyl)carbamate;
Compound No. 80: tert-butyl (N-(2-(4-(4-oxo-3,4-dihydrophthalazin-1-yl)phenyl)propan-2-yl)sulfamoyl)carbamate;
Compound No. 81: tert-butyl N-(4-(4-oxo-3,4-dihydrophthalazin-1-yl)-2-(trifluoromethyl)benzyl)sulfamoyl carbamate;
Compound No. 82: tert-butyl (N-methyl-N-(4-(4-oxo-3,4-dihydrophthalazin-1-yl)benzyl)sulfamoyl)carbamate;
Compound No. 83: tert-butyl ((6-(4-oxo-3,4-dihydrophthalazin-1-yl)-3,4-dihydroisoquinolin-2(1H)-yl)sulfonyl)carbamate;
Compound No. 84: tert-butyl ((6-(3-methyl-4-oxo-3,4-dihydrophthalazin-1-yl)-3,4-dihydroisoquinolin-2(1H)-yl)sulfonyl)carbamate;
Compound No. 85: tert-butyl ((6-(6,7-dimethoxy-4-oxo-3,4-dihydrophthalazin-1-yl)-3,4-dihydroisoquinolin-2(1H)-yl)sulfonyl)carbamate;
Compound No. 86: tert-butyl ((6-(6,7-dimethoxy-3-methyl-4-oxo-3,4-dihydrophthalazin-1-yl)-3,4-dihydroisoquinolin-2(1H)-yl)sulfonyl)carbamate;
Compound No. 87: N-(4-(4-oxo-3,4-dihydrophthalazin-1-yl)phenyl)sulfamide;
Compound No. 88: N-(3-(3-methyl-4-oxo-3,4-dihydrophthalazin-1-yl)phenyl)sulfamide hydrochloride;
Compound No. 89: N-(3-(3-benzyl-4-oxo-3,4-dihydrophthalazin-1-yl)phenyl)sulfamide hydrochloride;
Compound No. 90: N-(3-(3-benzyl-4-oxo-3,4-dihydrophthalazin-1-yl)benzyl)sulfamide hydrochloride;
Compound No. 91: N-(4-(4-oxo-3,4-dihydrophthalazin-1-yl)phenyl)sulfamide
Compound No. 92: N-(4-(3-methyl-4-oxo-3,4-dihydrophthalazin-1-yl)phenyl)sulfamide;
Compound No. 93: N-(4-(3-benzyl-4-oxo-3,4-dihydrophthalazin-1-yl)phenyl)sulfamide;
Compound No. 94: N-(3-(3-methyl-4-oxo-3,4-dihydrophthalazin-1-yl)benzyl)sulfamide trifluoroacetic acid;
Compound No. 95: N-(3-(3-oxo-3,4-dihydrophthalazin-1-yl)benzyl)sulfamide hydrochloride;
Compound No. 96: N-(4-(4-oxo-3,4-dihydrophthalazin-1-yl)benzyl) sulfamide hydrochloride;
Compound No. 97: N-(4-(3-methyl-4-oxo-3,4-dihydrophthalazin-1-yl)benzyl)sulfamide hydrochloride;
Compound No. 98: N-(4-(3-ethyl-4-oxo-3,4-dihydrophthalazin-1-yl)benzyl)sulfamide hydrochloride;
Compound No. 99: N-(4-(3-isopropyl-4-oxo-3,4-dihydrophthalazin-1-yl)benzyl)sulfamide trifluoroacetic acid;
Compound No. 100: N-(4-(4-oxo-3-phenyl-3,4-dihydrophthalazin-1-yl)benzyl)sulfamide hydrochloride;
Compound No. 101: N-(4-(3-benzyl-4-oxo-3,4-dihydrophthalazin-1-yl)benzyl)sulfamide hydrochloride;
Compound No. 102: N-(4-(3-(4-fluorobenzyl)-4-oxo-3,4-dihydrophthalazin-1-yl)benzyl)sulfamide hydrochloride;
Compound No. 103: N-(4-(7-methoxy-4-oxo-3,4-dihydrophthalazin-1-yl)benzyl)sulfamide hydrochloride;
Compound No. 104: N-(4-(6,7-dimethoxy-4-oxo-3,4-dihydrophthalazin-1-yl)benzyl)sulfamide hydrochloride;
Compound No. 105: N-(4-(7-methoxy-3-methyl-4-oxo-3,4-dihydrophthalazin-1-yl)benzyl)sulfamide hydrochloride
Compound No. 106: N-(4-(6-methoxy-4-oxo-3,4-dihydrophthalazin-1-yl)benzyl)sulfamide hydrochloride
Compound No. 107: N-(4-(6-methoxy-3-methyl-4-oxo-3,4-dihydrophthalazin-1-yl)benzyl)sulfamide hydrochloride
Compound No. 108: N-(4-(6,7-dimethoxy-3-methyl-4-oxo-3,4-dihydrophthalazin-1-yl)benzyl)sulfamide hydrochloride;
Compound No. 109: N-(3-(6,7-dimethoxy-4-oxo-3,4-dihydrophthalazin-1-yl)-benzyl) sulfamide;
Compound No. 110: N-(3-(6,7-dimethoxy-3-methyl-4-oxo-3,4-dihydrophthalazin-1-yl)-benzyl) sulfamide;
Compound No. 111: N-(4-(6,7-dimethoxy-4-oxo-3,4-dihydrophthalazin-1-yl)-2-fluorobenzyl)sulfamide hydrochloride;
Compound No. 112: N-(4-(6,7-dimethoxy-4-oxo-3,4-dihydrophthalazin-1-yl)-3-fluorobenzyl)sulfamide hydrochloride;
Compound No. 113: N-(4-(6,7-dimethoxy-4-oxo-3,4-dihydrophthalazin-1-yl)-2-fluorobenzyl)-N-methyl sulfamide hydrochloride;
Compound No. 114: N-(4-(6,7-dimethoxy-4-oxo-3,4-dihydrophthalazin-1-yl)-3-fluorobenzyl)-N-methyl sulfamide hydrochloride;
Compound No. 115: N-(4-(6,7-dimethoxy-4-oxo-3,4-dihydrophthalazin-1-yl)-2,3-difluorobenzyl)sulfamide hydrochloride;
Compound No. 116: N-(4-(6,7-dimethoxy-4-oxo-3,4-dihydrophthalazin-1-yl)-3-(trifluorobenzyl methyl)benzyl) sulfamide hydrochloride;
Compound No. 117: N-(4-(6,7-dimethoxy-4-oxo-3,4-dihydrophthalazin-1-yl)-2,3-difluorobenzyl)-N-methyl sulfamide hydrochloride;
Compound No. 118: N-(4-(6,7-dimethoxy-4-oxo-3,4-dihydrophthalazin-1-yl)-3-(trifluorobenzyl methyl)benzyl)-N-methyl sulfamide hydrochloride;
Compound No. 119: N-(4-(6,7-dimethoxy-4-oxo-3,4-dihydrophthalazin-1-yl)-2-(trifluorobenzyl methyl)benzyl)sulfamide hydrochloride;
Compound No. 120: N-(4-(6,7-dimethoxy-4-oxo-3,4-dihydrophthalazin-1-yl)-2-(trifluorobenzyl methyl)benzyl)-N-methyl sulfamide hydrochloride;
Compound No. 121: 7-(4-oxo-3,4-dihydrophthalazin-1-yl)-3,4-dihydroisoquinoline-2(1H) sulfamide hydrochloride;
Compound No. 122: 7-(6,7-dimethoxy-4-oxo-3,4-dihydrophthalazin-1-yl)-3,4-dihydroisoquinoline-2(1H)-sulfamide;
Compound No. 123: N-(1-(4-(4-oxo-3,4-dihydrophthalazin-1-yl)phenyl)ethyl)sulfamide hydrochloride;
Compound No. 124: (R)-N-(1-(4-(4-oxo-3,4-dihydrophthalazin-1-yl)phenyl)ethyl)sulfamide hydrochloride;
Compound No. 125: N-(2-(4-(4-oxo-3,4-dihydrophthalazin-1-yl)phenyl)propan-2-yl)sulfamide hydrochloride;
Compound No. 126: N-(4-(4-oxo-3,4-dihydrophthalazin-1-yl)-2-(trifluoromethyl)benzyl)sulfamide hydrochloride;
Compound No. 127: N-methyl-N-(4-(4-oxo-3,4-dihydrophthalazin-1-yl)benzyl)sulfamide;
Compound No. 128: 6-(4-oxo-3,4-dihydrophthalazin-1-yl-3,4-dihydroisoquinoline-2(1H)-sulfonamide hydrochloride;
Compound No. 129: 6-(3-methyl-4-oxo-3,4-dihydrophthalazin-1-yl)-3,4-dihydroisoquinoline-2(1H)-sulfonamide hydrochloride;
Compound No. 130: 6-(6,7-dimethoxy-4-oxo-3,4-dihydrophthalazin-1-yl)-3,4-dihydroisoquinoline-2(1H)-sulfonamide hydrochloride;
Compound No. 131: 6-(6,7-dimethoxy-3-methyl-4-oxo-3,4-dihydrophthalazin-1-yl)-3,4-dihydroisoquinoline-2(1H)-sulfonamide hydrochloride;
Compound No. 132: tert-butyl ((1-(4-oxo-3,4-dihydrophthalazin-1-yl)piperidin-4-yl)methyl)carbamate;
Compound No. 133: tert-butyl ((1-(6,7-dimethoxy-4-oxo-3,4-dihydrophthalazin-1-yl)piperidin-4-yl)methyl)carbamate;
Compound No. 134: 4-(4-(aminomethyl)piperidin-1-yl)phthalazin-1(2H)-one hydrochloride;
Compound No. 135: 4-(4-(aminomethyl)piperidin-1-yl)-6,7-dimethoxyphthalazin-1(2H)-one hydrochloride;
Compound No. 136: tert-butyl N-((1-(4-oxo-3,4-dihydrophthalazin-1-yl)piperidin-4-yl)methyl)sulfamoyl carbamate;
Compound No. 137: tert-butyl N-((1-(6,7-dimethoxy-4-oxo-3,4-dihydrophthalazin-1-yl)piperidin-4-yl)methyl)sulfamoyl carbamate;
Compound No. 138: N-((1-(4-oxo-3,4-dihydrophthalazin-1-yl)piperidin-4-yl)methyl)sulfamide hydrochloride;
Compound No. 139: N-((1-(6,7-dimethoxy-4-oxo-3,4-dihydrophthalazin-1-yl)piperidin-4-yl)methyl)sulfamide hydrochloride;
Compound No. 140: N-(4-(6,7-dimethoxy-3-methyl-4-oxo-3,4-dihydrophthalazin-1-yl)-amino)phenyl)sulfamide hydrochloride;
Compound No. 141: N-(3-(6,7-dimethoxy-3-methyl-4-oxo-3,4-dihydrophthalazin-1-yl)-amino)phenyl)sulfamide hydrochloride;
Compound No. 142: N-(2-(1-(4-oxo-3,4-dihydrophthalazin-1-yl)piperidin-4-yl)ethyl)sulfamide trifluoroacetic acid;
Compound No. 143: 4-benzylphthalazin-1(2H)-one;
Compound No. 144: 4-benzyl-6,7-dimethoxyphthalazin-1(2H)-one;
Compound No. 145: tert-butyl (3-((6,7-dimethoxy-4-oxo-3,4-dihydrophthalazin-1-yl)methyl)phenyl)carbamate;
Compound No. 146: tert-butyl (3-((6,7-dimethoxy-3-methyl-4-oxo-3,4-dihydrophthalazin-1-yl)methyl)phenyl)carbamate;
Compound No. 147: tert-butyl (4-((4-oxo-3,4-dihydrophthalazin-1-yl)methyl)phenyl)carbamate;
Compound No. 148: tert-butyl (4-((3-methyl-4-oxo-3,4-dihydrophthalazin-1-yl)methyl)phenyl)carbamate;
Compound No. 149: tert-butyl (4-((6,7-dimethoxy-4-oxo-3,4-dihydrophthalazin-1-yl)methyl)phenyl)carbamate;
Compound No. 150: tert-butyl (4-((6,7-dimethoxy-3-methyl-4-oxo-3,4-dihydrophthalazin-1-yl)methyl)phenyl)carbamate;
Compound No. 151: tert-butyl (4-((4-oxo-3,4-dihydrophthalazin-1-yl)methyl)phenyl) (methyl)carbamate;
Compound No. 152: tert-butyl (4-((6,7-dimethoxy-4-oxo-3,4-dihydrophthalazin-1-yl)methyl)phenyl) (methyl) carbamate;
Compound No. 153: tert-butyl (4-((6,7-dimethoxy-3-methyl-4-oxo-3,4-dihydrophthalazin-1-yl)methyl)phenyl) (methyl) carbamate;
Compound No. 154: tert-butyl (5-((6,7-dimethoxy-4-oxo-3,4-dihydrophthalazin-1-yl)methyl)indoline-1-carboxylate;
Compound No. 155: tert-butyl (5-((6,7-dimethoxy-3-methyl-4-oxo-3,4-dihydrophthalazin-1-yl)methyl)indoline-1-carboxylate;
Compound No. 156: 4-(3-aminobenzyl)-6,7-dimethoxyphthalazin-1(2H)-one hydrochloride;
Compound No. 157: 4-(3-aminobenzyl)-6,7-dimethoxy-2-methylphthalazin-1(2H)-one hydrochloride;
Compound No. 158: 4-(4-aminobenzyl)-phthalazin-1(2H)-one hydrochloride;
Compound No. 159: 4-(4-aminobenzyl)-2-methylphthalazin-1(2H)-one hydrochloride;
Compound No. 160: 4-(4-aminobenzyl)-6,7-dimethoxyphthalazin-1(2H)-one hydrochloride;
Compound No. 161: 4-(4-aminobenzyl)-6,7-dimethoxy-2-methylphthalazin-1(2H)-one hydrochloride;
Compound No. 162: 4-(4-(methylamino)benzyl)phthalazin-1(2H)-one hydrochloride;
Compound No. 163: 6,7-dimethoxy-4-(4-(methylamino)benzyl)phthalazin-1(2H)-one hydrochloride;
Compound No. 164: 6,7-dimethoxy-2-methyl-4-(4-(methylamino)benzyl)phthalazin-1(2H)-one hydrochloride;
Compound No. 165: 4-(indolin-5-yl-methyl)-6,7-dimethoxyphthalazin-1(2H)-one hydrochloride;
Compound No. 166: 4-(indolin-5-yl-methyl)-6,7-dimethoxy-2-methylphthalazin-1(2H)-one hydrochloride;
Compound No. 167: tert-butyl (N-(3-((6,7-dimethoxy-4-oxo-3,4-dihydrophthalazin-1-yl)methyl)phenyl)sulfamoyl)carbamate;
Compound No. 168: tert-butyl (N-(3-((6,7-dimethoxy-3-methyl-4-oxo-3,4-dihydrophthalazin-1-yl)methyl)phenyl)sulfamoyl)carbamate;
Compound No. 169: tert-butyl (N-(4-((4-oxo-3,4-dihydrophthalazin-1-yl)methyl)phenyl)sulfamoyl)carbamate;
Compound No. 170: tert-butyl (N-(4-((3-methyl-4-oxo-3,4-dihydrophthalazin-1-yl)methyl)phenyl)sulfamoyl)carbamate;
Compound No. 171: tert-butyl (N-(4-((6,7-dimethoxy-4-oxo-3,4-dihydrophthalazin-1-yl)methyl)phenyl)sulfamoyl)carbamate;
Compound No. 172: tert-butyl (N-(4-((6,7-dimethoxy-3-methyl-4-oxo-3,4-dihydrophthalazin-1-yl)methyl)phenyl)sulfamoyl)carbamate;
Compound No. 173: tert-butyl N-methyl-N-(4-((4-oxo-3,4-dihydrophthalazin-1-yl)methyl)phenyl)sulfamoyl carbamate;
Compound No. 174: tert-butyl N-(4-((6,7-dimethoxy-4-oxo-3,4-dihydrophthalazin-1-yl)methyl)phenyl)-N-methylsulfamoyl carbamate;
Compound No. 175: tert-butyl N-(4-((6,7-dimethoxy-3-methyl-4-oxo-3,4-dihydrophthalazin-1-yl)methyl)phenyl)-N-methylsulfamoyl carbamate;
Compound No. 176: tert-butyl (5-((6,7-dimethoxy-4-oxo-3,4-dihydrophthalazin-1-yl)methyl)indolin-1-yl)sulfonyl carbamate;
Compound No. 177: tert-butyl (5-((6,7-dimethoxy-3-methyl-4-oxo-3,4-dihydrophthalazin-1-yl)methyl)indolin-1-yl)sulfonyl carbamate;
Compound No. 178: N-(3-((6,7-dimethoxy-4-oxo-3,4-dihydrophthalazin-1-yl)methyl)phenyl)sulfamide hydrochloride;
Compound No. 179: N-(3-((6,7-dimethoxy-3-methyl-4-oxo-3,4-dihydrophthalazin-1-yl)methyl)phenyl)sulfamide hydrochloride;
Compound No. 180: N-(4-((4-oxo-3,4-dihydrophthalazin-1-yl)methyl)phenyl)sulfamide hydrochloride;
Compound No. 181: N-(4-((3-methyl-4-oxo-3,4-dihydrophthalazin-1-yl)methyl)phenyl)sulfamide hydrochloride;
Compound No. 182: N-(4-((6,7-dimethoxy-4-oxo-3,4-dihydrophthalazin-1-yl)methyl)phenyl)sulfamide hydrochloride;
Compound No. 183: N-(4-((6,7-dimethoxy-3-methyl-4-oxo-3,4-dihydrophthalazin-1-yl)methyl)phenyl)sulfamide hydrochloride;
Compound No. 184: N-methyl-N-(4-((4-oxo-3,4-dihydrophthalazin-1-yl)methyl)phenyl)sulfamide hydrochloride;
Compound No. 185: N-(4-((6,7-dimethoxy-4-oxo-3,4-dihydrophthalazin-1-yl)methyl)phenyl)-N-methylsulfamide hydrochloride;
Compound No. 186: N-(4-((6,7-dimethoxy-3-methyl-4-oxo-3,4-dihydrophthalazin-1-yl)methyl)phenyl)-N-methylsulfamide hydrochloride;
Compound No. 187: 5-((6,7-dimethoxy-4-oxo-3,4-dihydrophthalazin-1-yl)methyl)indoline-1-sulfonamide hydrochloride;
Compound No. 188: 5-((6,7-dimethoxy-3-methyl-4-oxo-3,4-dihydrophthalazin-1-yl)methyl)indoline-1-sulfonamide hydrochloride;
Compound No. 189: N-(3-fluoro-4-((4-oxo-3,4-dihydrophthalazin-1-yl)methyl)phenyl)sulfamide hydrochloride;
Compound No. 190: N-(2-fluoro-4-((4-oxo-3,4-dihydrophthalazin-1-yl)methyl)phenyl)sulfamide hydrochloride;
Compound No. 191: N-(3-(trifluorobenzyl methyl)-4-((4-oxo-3,4-dihydrophthalazin-1-yl)methyl)phenyl)sulfamide hydrochloride;
Compound No. 192: N-(2-(trifluorobenzyl methyl)-4-((4-oxo-3,4-dihydrophthalazin-1-yl)methyl)phenyl)sulfamide hydrochloride;
Compound No. 193: 4-(3-bromobenzyl)-6,7-dimethoxyphthalazin-1(2H)-one;
Compound No. 194: 4-(4-bromobenzyl)-6,7-dimethoxyphthalazin-1(2H)-one;
Compound No. 195: N-(4-(4-oxo-3,4-dihydrophthalazin-1-yl)benzyl)methanesulfonamide;
Compound No. 196: N-(4-(4-oxo-3,4-dihydrophthalazin-1-yl)benzyl)cyclopropanesulfonamide;
Compound No. 197: 4-methyl-N-(4-(4-oxo-3,4-dihydrophthalazin-1-yl)benzyl)benzenesulfonamide;
Compound No. 198: N-(4-(4-oxo-3,4-dihydrophthalazin-1-yl)benzyl)thiophene-2-sulfonamide;
Compound No. 199: (4-(3-methyl-4-oxo-3,4-dihydrophthalazin-1-yl)phenyl)sulfamide;
Compound No. 200: N-methyl-1-(4-(3-methyl-4-oxo-3,4-dihydrophthalazin-1-yl)phenyl)methanesulfonamide;
Compound No. 201: 2-methyl-4-(4-((morpholinosulfonyl)methyl)phenyl)phthalazin-1(2H)-one;
Compound No. 202: 2-(4-(3-methyl-4-oxo-3,4-dihydrophthalazin-1-yl)phenyl)ethane-1-sulfonamide; and
Compound No. 203: 4-(4-(aminomethyl)-3-(trifluoromethyl)phenyl-6,7-dimethoxy phthalazin-1(2H)-one hydrochloride.

In some embodiments, the compounds of the present invention have an ENPP1 inhibition profile that reflects activity against additional enzymes. In some embodiments, the compounds of the present invention specifically inhibit ENPP1 without unintended inhibition of one or more other enzymes.

In some embodiments, the compounds of the present invention inhibit ENPP 1, which can be determined through assay for detecting the activity of enzymes in cell-free or cellular systems after treatment with the compounds of the present invention, compared to a control group by inhibition analysis, for example, by measurement of IC₅₀ or EC₅₀. In some embodiments, the compounds of the present invention have an IC₅₀ (or EC₅₀₎ of 10 µM or less, such as 3 µM or less, 1 µM or less, 500 nM or less, 300 nM or less, 200 nM or less, 100 nM or less, 50 nM or less, 30 nM or less, 10 nM or less, 5 nM or less, 3 nM or less, 1 nM or less, or an even lower value.

The procedure of the assay that can be used to determine the activity of ENPP1 may include, but is not limited to, the following assay procedures including cell-free assay systems, such as avidity assays, assays using purified enzymes, cell assays for determining phenotypes of cells, such as gene expression assays and in *vivo* assays involving specific animals.

In some embodiments, the method of the present invention is a method of suppressing cancer cell proliferation, the method including treating the cells with an effective amount of the compound of the present invention to suppress cancer cell proliferation. In some embodiments, the method may be performed in combination with chemotherapy. Any cancer cells may be used as long as they are available.

### Treatment method

As mentioned above, the present invention includes methods for inhibiting ENPP1 activity against cGAMP to cause increased levels of cGAMP and/or modulation of downstream factors of the STING pathway. A recent report has shown that ENPP1 inhibition can modulate STING activity *in vivo,* and thus can be used for the treatment of various diseases, such as cancer immunotherapy or treatment of infectious diseases. As described above, the method of the present invention is a method of increasing the STING-mediated response and regulating the immune response in a subject.

In some embodiments, the STING-mediated response includes increasing production of interferon (e.g., type 1 interferon, type 3 interferon) in the subject. Interferons (IFNs) are a group of signaling proteins produced and released by host cells in response to the presence of one or more pathogens to enhance the defenses of surrounding cells against the pathogens. IFNs also have a variety of other functions of 1) activating immune cells such as NK cells and macrophages, and of 2) promoting host defense by upregulating antigen presentation through increased expression of major histocompatibility complex (MHC) antigens. IFNs are generally classified into three classes: type 1 IFNs, type 2 IFNs, and type 3 IFNs. Mammalian type 1 INFs include IFN-α (alpha), IFN-β (beta), IFN-δ (delta), IFN-ε (epsilon), IFN-κ (kappa), IFN-τ (tau), IFN-ω (omega), and IFN-ζ (zeta). All type 1 IFNs bind to a specific cell surface receptor complex known as an IFN-α/β receptor (IFNAR), which consists of IFNAR1 and IFNAR2 chains.

Interferons have been studied as cancer therapeutics due to the antitumor activity thereof, which exhibit two different effects, namely, tumor-intrinsic and/or immunomodulatory effects. IFNs regulate the expression of a number of genes that directly affect tumor cell growth, proliferation, differentiation, survival, migration and other specific functions. In some cases, in *vitro* treatment using type 1 IFN has a direct antiproliferative effect due to the prolonged all phases of the cell cycle by IFN. In some cases, CRKL, which is a type of CRK proteins activated by type 1 IFN, interacts with the tumor suppressor, small G-protein RAP1A to inhibit RAS family GTPase, resulting in growth arrest of cancer cells. IFNs are known to regulate two major apoptotic responses. The two apoptotic responses are extrinsic (apoptotic receptor-mediated pathway) and intrinsic (mitochondrial) pathways. In response to type 1 IFN, upregulation of sensor protein activation (e.g., DR receptor) occurs, which causes apoptosis of tumor cells.

IFNs have extrinsic effects on tumors through regulation of processes such as angiogenesis, osteoclastogenesis and immunity. Both endogenous and exogenous type 1 IFNs play a major role in the activity of anticancer immunity. For example, type 1 IFNs enhance the activity of α/β T cells, γ/δ T cells, NK cells and dendritic cells, and inhibit the activity of immune-suppressive cells, for example, conversion of regulatory T cells, bone marrow-derived suppressor cells and tumor-associated macrophages. Type 1 IFN also acts directly on tumor cells to improve antigen expression and up-regulate numerous immune-interacting molecules (e.g., stress ligands recognized by major histocompatibility complex class 1 (MHCI)) and germline-encoded immunoreceptors.

In one aspect, the method includes administering a therapeutically effective amount of the ENPP1 inhibitor to a subject having cancer to treat the cancer of the subject. In one embodiment, the subject may be a subject diagnosed with or suspected of having cancer. Any suitable ENPP1 inhibitor may be used in the subject. In one embodiment, the cancer is selected from adrenal gland cancer, liver cancer, kidney cancer, bladder cancer, breast cancer, colon cancer, stomach cancer, ovarian cancer, cervical cancer, uterus cancer, esophageal cancer, colorectal cancer, prostate cancer, pancreatic cancer, lung (small and non-small cell) cancer, thyroid cancer, carcinoma, sarcoma, glioblastoma, melanoma, and head and neck cancer. In one embodiment, the cancer is lymphoma.

In one embodiment, the effective amount of the compound ranges from about 10 ng to about 100 mg, e.g., from about 10 ng to about 50 ng, from about 50 ng to about 150 ng, from about 150 ng to about 250 ng, from about 250 ng to about 500 ng, from about 500 ng to about 750 ng, from about 750 ng to about 1 ug, from about 1 ug to about 10 ug, from about 10 ug to about 50 ug, from about 50 ug to about 150 ug, from about 150 ug to about 250 ug, from about 250 ug to about 500 ug, from about 500 ug to about 750 ug, from about 750 ug to about 1 mg, from about 1 mg to about 50 mg, from about 1 mg to about 100 mg, or from about 50 mg to about 100 mg. The amount may be a single dose or a total daily dose. The total daily amount may range from 10 ng to 100 mg, or from 100 mg to about 500 mg, or from 500 mg to about 1,000 mg.

In one embodiment, the compound is administered in a single dose. In other embodiments, multiple doses are administered. When the multiple doses are administered over a period of time, the compound is administered twice a day (bid), once a day (qd), every other day (qod), every 3 days, 3 times a week (tiw), or twice a week (biw).

### Combination therapy

The ENPP1 inhibitor compound of the present invention may be administered to a subject alone or in combination with an additional active agent or additional therapy, e.g., radiation therapy. The terms "agent", "compound" and "drug" are used interchangeably herein. In one embodiment, the method of the present invention further includes administering to the subject an additional agent, e.g., a small molecule, a chemotherapeutic agent, an antibody, an antibody-drug conjugate, an aptamer, a protein, an immune checkpoint inhibitor, or radiotherapy concomitantly or sequentially in combination.

The term "co-administration" or "in combination with" includes the administration of two or more therapeutic agents simultaneously, concurrently, or sequentially, without any specific time limit. In some embodiments, agonists or agents are concurrently present in cells or subjects or simultaneously exert biological or therapeutic effects. In some embodiments, the therapeutic agents are in the form of an identical composition or unit dosage form. In some embodiments, the therapeutic agents are separate compositions or unit dosage forms. In some embodiments, the first therapeutic agent is administered prior to administration of the second therapeutic agent (e.g., before 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, 12 weeks), concomitantly with the administration of the second therapeutic agent, or after the administration of the second therapeutic agent (e.g., after 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks).

The "concomitant administration" of additional therapy based on a known therapeutic agent or the composition including the disclosed compound of the present invention means a combination of the compound and the second agent or additional therapy such that both the known agent and the composition including the disclosed compound have therapeutic effects. The concomitant administration may include simultaneous or previous administration of drugs in conjunction with administration of the compound of the present invention. The routes of administration of the two agents may vary and representative routes of administration are described in detail below. Those skilled in the art will have no difficulty determining the appropriate administration time, sequence and dosage for the drug or therapy and the compound disclosed herein.

For the treatment of cancer, the ENPP1 inhibitor compounds may be administered in combination with a chemotherapeutic agent selected from the group consisting of alkylating agents, anti-metabolites, anti-tumor antibiotics, plant alkaloids, taxanes, nucleoside analogues, anthracyclines, thymidylate-targeting drugs, apoptosis modulators, cell cycle control inhibitors, colony stimulation factor-1 receptor inhibitors, CD47 inhibitors and the other.

For the treatment of cancer, the ENPP1 inhibitor compound may be administered in combination with an immunotherapeutic agent. The immunotherapeutic agent is an agonist suitable for the treatment of cancer by inducing, enhancing, or inhibiting an immune response. In some embodiments, the immunotherapeutic agent is an immune checkpoint inhibitor. Any immunosuppressant may be used. For example, the immune checkpoint inhibitor may be a cytotoxic T-lymphocyte-associated antigen 4 (CTLA-4) inhibitor, a programmed death 1 (PD-1) inhibitor or programmed cell death ligand 1 (PD-L1) inhibitor, but is not limited thereto. Examples of the immune checkpoint inhibitor include, but are not limited to, ipilimumab, pembrolizumab, nivolumab, atezolizumab, avelumab, durvalumab, cemiplimab and the like. In some embodiments, the immunotherapeutic agent is immune cell therapy. Any suitable cell therapy may be used and examples thereof include, but not limited to, chimeric antigen receptor T cell therapy, chimeric antigen receptor NK cell therapy, and other cell therapies.

For the treatment of cancer, the ENPP1 inhibitor compound may be administered in combination with a suitable cancer vaccine regimen, for example, a dendritic cell vaccine that promotes Th1/Th17 immunity. In some cases, the ENPP1 inhibitor compound is suitable for use as an adjuvant therapy in combination with Th-17-inducing vaccination.

### Combination with radiation therapy

For cancer treatment methods, the ENPP1 inhibitor compound may be administered in combination with radiation therapy. In some embodiments, the ENPP1 inhibitor compound may be administered before or after radiation therapy. The combination of radiation therapy and administration of the compound of the present invention may provide a synergistic therapeutic effect. When the subject is subjected to radiation therapy (RT) at an appropriate dose and/or frequency during radiation therapy (RT), cGAMP may be produced in the subject. The ENPP1 inhibitor compound can enhance therapeutic efficacy on the subject by preventing degradation of the produced cGAMP, compared to the level of cGAMP produced by, for example, RT alone. As such, the details of the methods of the present invention include administration of the reduced dose and/or frequency and/or regime of radiation therapy as compared to the dosage and/or frequency and/or reduction of the therapeutic effect of radiation therapy alone. In some embodiments, radiation therapy is administered in combination with the compound of the present invention at a dosage and/or frequency effective to reduce the risk of radiation damage, e.g., the risk of radiation damage to the subject, expected by a therapeutically effective amount.

In one embodiment, the method includes administering to the subject an ENPP1 inhibitor prior to radiation therapy. In one embodiment, the method includes administering an ENPP1 inhibitor to a subject after exposure of the subject to radiation therapy. In another embodiment, the method includes sequentially administering to a subject in need thereof radiation therapy, followed by an ENPP1 inhibitor, followed by an immune checkpoint inhibitor.

### Pharmaceutical composition

Pharmaceutically acceptable excipients such as vehicles, adjuvants, carriers or diluents are easily obtainable to those skilled in the art. Pharmaceutically acceptable auxiliary substances such as pH modifiers, buffers, isotonicity modifiers, stabilizers, wetting agents and the like are easily obtainable to those skilled in the art.

In some embodiments, the compound of the present invention is formulated in an aqueous buffer. Suitable aqueous buffers include, but are not limited to, acetate, succinate, citrate and phosphate buffers at various concentrations ranging from 5 mM to 1,000 mM. In some embodiments, aqueous buffers include reagents that provide isotonic solutions. Such reagents include, but are not limited to, sodium chloride, sugars such as mannitol, dextrose, and sucrose, and the like. In some embodiments, the aqueous buffer further includes a nonionic surfactant such as polysorbate 20 or 80. Optionally, the formulation may further include a preservative. Suitable preservatives include, but are not limited to, benzyl alcohol, phenol, chlorobutanol, benzalkonium chloride, and the like. In another embodiment, the formulation is stored at about 4°C. The formulation may also be lyophilized and generally contains a cryoprotectant such as sucrose, trehalose, lactose, maltose, or mannitol. Freeze-dried preparations may be stored even at room temperature for a long period of time.

Here, the pharmaceutical composition may include or essentially consist of the compound disclosed herein, or a pharmaceutically acceptable salt thereof, an isomer thereof, or a tautomer thereof, and further includes or essentially consists of one or more activators of interest. Any beneficial activator may be used in the present method in combination with the compound of the present invention. In one embodiment, the compound of the present invention and the immune checkpoint inhibitor, as well as the additional therapeutic agent as described herein for combination therapy, may be administered orally, subcutaneously, intramuscularly, intranasally, parenterally or via other routes. In another embodiment, the compound of the present invention, the chemotherapeutic agent (especially a chemotherapeutic agent capable of inducing cGAMP production *in vivo*), and the additional therapeutic agent described herein for combination therapy may be administered orally, subcutaneously, intramuscularly, intranasally, parenterally or by other routes. The compound of the present invention and the second activator (if present) may be administered via identical or different routes. The therapeutic agent may be administered by any suitable means including, but not limited to, oral, rectal, nasal, topical, vaginal, parenteral, intravenous, intranasal, and intratumoral injection into the organ affected.

The compounds of the present invention may be administered in a unit dosage form and may be prepared by any method well known in the art. Such methods include combining the compound of the present invention with a pharmaceutically acceptable carrier or diluent which constitutes one of one or more auxiliary ingredients. The pharmaceutically acceptable carrier is selected based on the selected route of administration and standard pharmaceutical practice. Each carrier should be "pharmaceutically acceptable" in that it is compatible with the other ingredients of the formulation and does not harm the subject or patient. This carrier may be solid or liquid, the type of which is generally selected according to the route of administration.

Examples of suitable solid carriers include lactose, sucrose, gelatin, agar and bulk powder. Examples of suitable liquid carriers include water, pharmaceutically acceptable fats and oils, alcohols or other organic solvents such as esters, emulsions, syrups or elixirs, suspensions and solutions and/or suspensions reconstituted from non-foaming granules. Such liquid carriers may contain, for example, suitable solvents, preservatives, emulsifiers, suspending agents, diluents, sweetening agents, thickeners and melting agents.

**Hereinafter, various aspects of the present invention will be described.**

In one aspct, the present invention provides a compound selected from a phthalazine derivative compound represented by the following Formula 1, a pharmaceutically acceptable salt thereof, a hydrate thereof, a solvate thereof and a stereoisomer thereof: 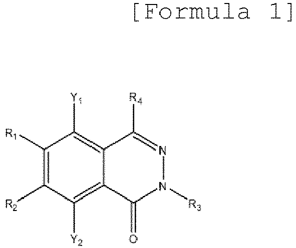 wherein
Y₁ and Y₂ are each independently hydrogen, a hydroxyl group, a halogen group, a C₁-C₁₃ alkyl group, a C₃-C₁₀ cyclic group, a C₁-C₆ alkoxy group, a C₃-C₁₀ cyclic group, or a C₃-C₁₀ heterocyclic group;
R₁ and R₂ are each independently hydrogen, a hydroxyl group, a halogen group, a C₁-C₁₃ alkyl group, a C₁-C₆ alkoxy group, a C₆-C₁ aryl group, a C₃-C₁₀ cyclic group, a C₃-C₁₀ heteroaryl group, a C₃-C₁₀ heterocyclic group, or -C(O)-(C₁-C₁₃ alkyl), or R₁, together with the nitrogen atom bonded to R₂, forms a 4- to 7-membered saturated, unsaturated or aromatic ring, which includes at least one of N, O, S, NH, C=N, C=O, -NHC(O)-, -NHC(O)NH-, -NHS(O)₂-, or SO₂, and is substituted with at least one of a C₁-C₁₃ alkyl group, a C₆-C₁₀ aryl group, a C₃-C₁₀ heteroaryl group, a hydroxyl group, a halide group or a cyano group;
R₃ is hydrogen, a hydroxyl group, a halogen group, a C₁-C₁₃ alkyl group, a C₁-C₆ alkoxy group, a C₆-C₁₀ aryl group, a C₃-C₁₀ cyclic group, a C₃-C₁₀ heteroaryl group, a C₃-C₁₀ heterocyclic group, or -C(O)-(C₁-C₁₃ alkyl);
R₄ is hydrogen, a hydroxyl group, a halogen group, a C₁-C₁₃ alkyl group, a C₁-C₆ alkoxy group, a C₁-C₅ alkenyl group, a C₆-C₁ aryl group, a C₃-C₁₀ cyclic group, a C₃-C₁₀ heteroaryl group, a C₃-C₁₀ heterocyclic group, an amino group, a nitro group, an amide group, a carboxylic group, a nitrile group, a urea group, a sulfonamide group, a sulfide group, a sulfonic group, or a phosphoryl group,
wherein the C₁-C₆ alkyl group, the C₁-C₁₃ alkyl group or the C₃-C₁₀ cyclic group includes at least one substituent selected from the group consisting of hydrogen, a hydroxyl group, a halogen group, a C₁-C₁₃ alkyl group, a C₁-C₆ alkoxy group, an amino group (-NR₅R₆), a nitro group (-N(O)₂), an amide group (-(C=O)NR₅R₆), a carboxylic group (-C(O)OH), a nitrile group (-CN), a urea group (-NR₅(C=O)NR₆-), a sulfonamide group (-NHS(O)₂-), a sulfide group (-S-), a sulfonyl group (-S(O)e-), a phosphoryl group (-P(O)R₅R₆), a C₆-C₁₀ aryl group, a C₃-C₁₀ heteroaryl group, and a C₃-C₁₀ heterocyclic group, and
the C₆-C₁₀ aryl group, the C₃-C₁₀ heteroaryl group or the C₃-C₁₀ heterocyclic group includes at least one substituent selected from the group consisting of hydrogen, a hydroxyl group, a halogen group, a carbonyl group (-(C=O)R₅R₆), a C₁-C₃ alkyl group substituted or unsubstituted with halogen or a C₃-C₁₀ heterocyclic group, a C₁-C₃ alkoxy group substituted or unsubstituted with halogen or a C₃-C₁₀ heterocyclic group, C₆-C₁₀ phenoxy, an amino group (-NR₅R₆), a nitro group (-N(O)₂), an amide group (-(C=O)NR₅R₆), a carboxylic group (-C(O)OH), a nitrile group (-CN), a urea group (-NR₅(C=O)NR₆-), a sulfonamide group (-NHS(O)₂-), a sulfide group (-S-), a sulfonyl group (-S(O)s-), a phosphoryl group (-P(O)R₅R₆), a C₆-C₁₀ aryl group, a C₃-C₁₀ heteroaryl group, and a C₃-C₁₀ heterocyclic group; and
R₅ and R₆ are each independently hydrogen, a C₁-C₆ alkyl group, a C₁-C₆ alkenyl group, a C₁-C₆ alkynyl group, a C₆-C₁ aryl group, a C₃-C₁₀ heteroaryl group, a C₃-C₁₀ heterocyclic group, or R₅, together with the nitrogen or carbon atom bonded to R₆, forms a 3- to 7-membered saturated ring, which includes at least one of N, O, S, NH, C=N, C=O, -NHC(O)-, -NHC(O)NH-, -NHS(O)₂-, or SO₂, and is substituted with at least one of hydrogen, a C₁-C₁₃ alkyl group, a C₆-C₁₀ aryl group, a C₃-C₁₀ heteroaryl group, a hydroxyl group, a halide group, or a cyano group,
wherein the C₃-C₁₀ heteroaryl group and the C₃-C₁₀ heterocyclic group include at least one heteroatom selected from the group consisting of N, O, and S.

In the definition of a substituent in the present invention, the term "alkyl" means an aliphatic hydrocarbon radical. Alkyl may be "saturated alkyl" containing no alkenyl or alkynyl moiety, or "unsaturated alkyl" containing at least one alkenyl or alkynyl moiety. The term "alkenyl" means a group containing at least one carbon-carbon double bond, and the term "alkynyl" means a group containing at least one carbon-carbon triple bond. The alkyl may be cyclic, branched or straight-chain when used alone or in combination.

The term "aryl" means a C6 aromatic monocarbocyclic group which may be further fused, singly or in combination with another radical, with a 5- or 6-membered carbocyclic group which may be aromatic, and may be saturated or unsaturated. Examples of the aryl may include, but are not limited to, phenyl, indanyl, 1-naphthyl, 2-naphthyl, tetrahydronaphthyl, and the like. Aryl may be linked with other groups at appropriate positions on the aromatic ring.

The term "alkoxy" refers to an alkyl group linked to another group through an oxygen atom (i.e., -O-alkyl). The alkoxy group may be unsubstituted or substituted with one or more suitable substituents. Examples of the alkoxy group include, but are not limited to, a (C1-C6)alkoxy group, such as -O-methyl, -O-ethyl, -O-propyl, -O-isopropyl, -O-2-methyl-1-propyl, -O-2-methyl-2-propyl, -O-2-methyl-1-butyl, -O-3-methyl-1-butyl, -O-2-methyl-3-butyl, -O-2,2-dimethyl-1-propyl, -O-2-methyl-1-pentyl, 3-O-methyl-1-pentyl, -O-4-methyl-1-pentyl, -O-2-methyl-2-pentyl, -O-3-methyl-2-pentyl, -O-4-methyl-2-pentyl, -O-2,2-dimethyl-1-butyl, -O-3,3-dimethyl-butyl, -O-2-ethyl-1-butyl, -O-butyl, -O-isobutyl, -O-t-butyl, -O-pentyl, -O-isopentyl, -O-neopentyl and -O-hexyl.

The term "phenoxy" refers to a phenyl group linked to another group through an oxygen atom (i.e., -O-aryl). The phenoxy is unsubstituted or substituted with at least one of halogen, alkyl, aryl or heteroaryl group, but is not limited thereto.

The term "amino group" refers to an alkyl group linked to another group through a nitrogen atom (i.e., -NH- or -N-alkyl). The amine group may be unsubstituted or substituted with one or more suitable substituents. Examples of the amine group include, but are not limited to, (C₁-C₆)amino, for example, -NH-methyl, -NH-ethyl, -NH-propyl, -NH-isopropyl, -NH-2-methyl-1-propyl, -NH-2-methyl-2-propyl, -NH-2-methyl-1-butyl, -NH-3-methyl-1-butyl, -NH-2-methyl-3-butyl, -NH-2,2-dimethyl-1-propyl, -NH-2-methyl-1-pentyl, 3-NH-methyl-1-pentyl, -NH-4-methyl-1-pentyl, -NH-2-methyl-2-pentyl, -NH-3-methyl-2-pentyl, -NH-4-methyl-2-pentyl, -NH-2,2-dimethyl-1-butyl, -NH-3,3-dimethyl-butyl, - NH-2-ethyl-1-butyl, -NH-butyl, -NH-isobutyl, -NH-t-butyl, - NH-pentyl, -NH-isopentyl, -NH-neopentyl, -NH-hexyl, -N,N-dimethyl, -N-methyl-N-ethyl, -N-methyl-N-propyl, -N-methyl-isopropyl, -N-methyl-N-butyl, -N-methyl-N-isobutyl, -N-methyl-N-pentyl, -N-methyl-N-isopentyl, N-methyl-N-hexyl, N-methyl-N-isohexyl, -N,N-diethyl, -N-ethyl-N-propyl, -N-ethyl-N-isopropyl, -N-ethyl-N-butyl, -N-ethyl-N-isobutyl, - N-ethyl-N-pentyl, -N-ethyl-N-isopentyl, -N-ethyl-N-hexyl, -N-ethyl-N-isohexyl, -N,N-dipropyl, -N-propyl-N-isopropyl, -N-propyl-N-butyl, -N-propyl-N-isobutyl, -N-propyl-N-pentyl, -N-propyl-N-isopentyl, -N-propyl-N-hexyl, -N-propyl-N-isohexyl, -N,N-dibutyl, -N-butyl-N-isobutyl, -N-butyl-N-pentyl, -N-butyl-N-isopentyl, -N-butyl-N-hexyl, - N-butyl-N-isohexyl, -N,N-dipentyl, -N-pentyl-N-hexyl, -N-pentyl-N-isohexyl, and -N,N-dihexyl.

The term "halogen group" means fluorine, chlorine, bromine or iodine.

The term "heterocyclic group" means a heteroaromatic compound containing at least one heteroatom selected from the group consisting of N, O, and S, unless otherwise mentioned. Preferably, the heterocyclic group may include, without being limited to, pyrrolidine, furan, morpholine, piperazine and piperidine groups, more preferably pyrrolidine, piperidine, piperazine and morpholine groups.

The term "heteroaryl group" refers to a heteroaromatic compound containing at least one heteroatom selected from the group consisting of N, O, and S, unless otherwise mentioned. Preferably, the heteroaryl group may include, without being limited to, pyridine, pyrazine, pyrimidine, pyridazine, pyrazole, imidazole, triazole, indole, oxadiazole, thiadiazole, quinoline, isoquinoline, isoxazole, oxazole, thiazole and pyrrole groups.

In one aspect, the present invention provides a compound selected from a phthalazine derivative compound represented by Formula 1, a pharmaceutically acceptable salt thereof, a hydrate thereof, a solvate thereof and a stereoisomer thereof, wherein Y₁ and Y₂ are each independently hydrogen, or a C₁-C₁₃ alkyl group, R₁ and R₂ are each independently hydrogen, a hydroxyl group, a C₁-C₁₃ alkyl group, or a C₁-C₆ alkoxy group, R₃ is hydrogen, a C₁-C₁₃ alkyl group, or a C₆-C₁₀ aryl group, and R₄ is a C₁-C₁₃ alkyl group, a C₆-C₁₀ aryl group, a C₃-C₁₀ cyclic group, a C₃-C₁₀ heteroaryl group, a C₃-C₁₀ heterocyclic group, an amino group, a nitro group, an amide group, a carboxylic group, a nitrile group, a urea group, a sulfonamide group, a sulfide group, a sulfonic group, or a phosphoryl group.

In one aspect, the present invention provides a compound selected from a phthalazine derivative compound represented by Formula 1, a pharmaceutically acceptable salt thereof, a hydrate thereof, a solvate thereof and a stereoisomer thereof, wherein Y₁ and Y₂ are each hydrogen, R₁ and R₂ are each independently hydrogen, a C₁-C₄ alkyl group, or a C₁-C₄ alkoxy group, R₃ is hydrogen, a C₁-C₄ alkyl group, or a C₆-C₁₀ aryl group, and R₄ is a substituted or unsubstituted C₁-C₆ alkyl group, substituted or unsubstituted benzene, substituted or unsubstituted hexane, substituted or unsubstituted furan, substituted or unsubstituted thiophene, substituted or unsubstituted pyridine, substituted or unsubstituted benzofuran, substituted or unsubstituted naphthalene, substituted or unsubstituted anthracene, substituted or unsubstituted phenanthrene, substituted or unsubstituted pyridazine, substituted or unsubstituted piperidine, substituted or unsubstituted morpholine, substituted or unsubstituted pyrrolidine, substituted or unsubstituted pyrazine, substituted or unsubstituted imidazole, substituted or unsubstituted pyrazole, substituted or unsubstituted quinoline, substituted or unsubstituted pyrimidine, substituted or unsubstituted pyrrole, substituted or unsubstituted indole, substituted or unsubstituted purine, substituted or unsubstituted cyclopropane, or substituted or unsubstituted cyclobutane.

In one aspect, the present invention provides a compound selected from a phthalazine derivative compound represented by Formula 1, a pharmaceutically acceptable salt thereof, a hydrate thereof, a solvate thereof and a stereoisomer thereof, wherein Y₁ and Y₂ are each hydrogen, R₁ and R₂ are each independently hydrogen, or a methoxy group, R₃ is hydrogen, a C₁-C₄ alkyl group, a phenyl group, or a benzyl group, and R₄ is a substituted or unsubstituted phenyl group, a substituted or unsubstituted benzyl group,

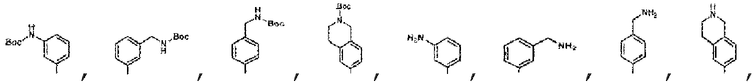

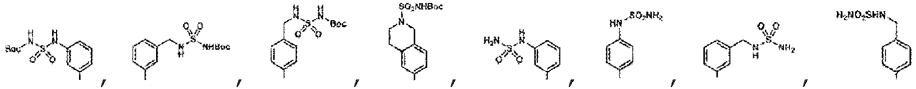

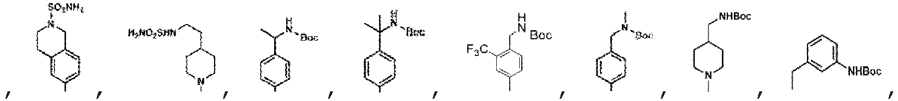

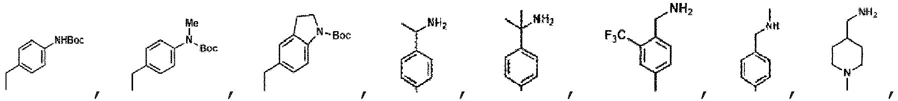

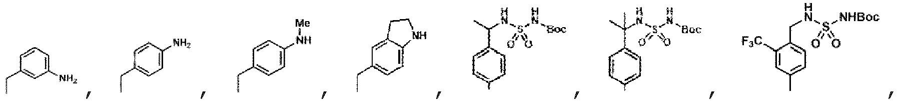

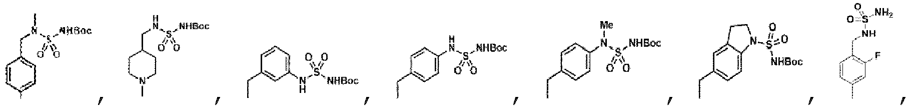

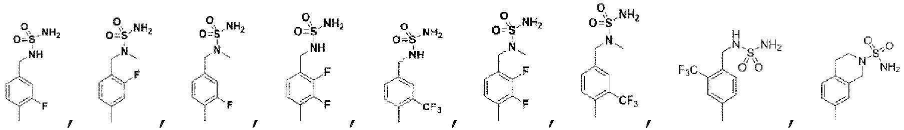

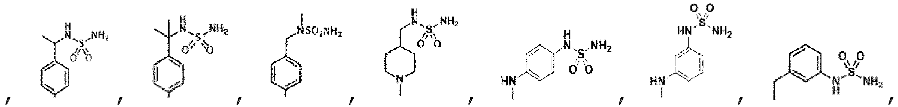

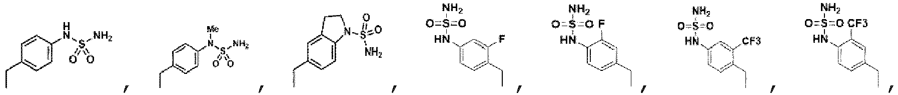

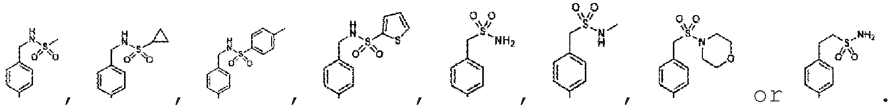

In one aspect, the present invention provides a compound selected from a phthalazine derivative compound represented by Formula 1, a pharmaceutically acceptable salt thereof, a hydrate thereof, a solvate thereof and a stereoisomer thereof, wherein the compound is selected from the group consisting of the following Compounds Nos. 1 to 203:
Compound No. 1: 4-phenylphthalazin-1(2H)-one;
Compound No. 2: tert-butyl(3-(4-oxo-3,4-dihydrophthalazin-1-yl)phenyl)carbamate;
Compound No. 3: tert-butyl (3(3-methyl-4-oxo-3,4-dihydrophthalazin-1-yl)phenyl)carbamate;
Compound No. 4: tert-butyl (3(3-benzyl-4-oxo-3,4-dihydrophthalazin-1-yl)phenyl)carbamate;
Compound No. 5: tert-butyl(3-(4-oxo-3,4-dihydrophthalazin-1-yl)carbamate;
Compound No. 6: tert-butyl 3-(3-methyl-4-oxo-3,4-dihydrophthalazin-1-yl)benzyl carbamate;
Compound No. 7: tert-butyl (3-(3-benzyl-4-oxo-3,4-dihydrophthalazin-1-yl)benzyl)carbamate;
Compound No. 8: tert-butyl (4-(4-oxo-3,4-dihydrophthalazin-1-yl)benzyl)carbamate; Compound No. 9: tert-butyl (4-3-methyl-4-oxo-3,4-dihydrophthalazin-1-yl)benzyl)carbamate;
Compound No. 10: tert-butyl (4-(3-ethyl-4-oxo-3,4-dihydrophthalazin-1-yl)benzyl)carbamate;
Compound No. 11: tert-butyl 4-(3-isopropyl-4-oxo-3,4-dihydrophthalazin-1-yl)benzyl carbamate;
Compound No. 12: tert-butyl (4-(4-oxo-3-phenyl-3,4-dihydrophthalazin-1-yl)benzyl)carbamate;
Compound No. 13: tert-butyl (4-3-benzyl-4-oxo-3,4-dihydrophthalazin-1-yl)benzyl)carbamate;
Compound No. 14: tert-butyl (4-(3-(4-fluorobenzyl)-4-oxo-3,4-dihydrophthalazin-1-yl)benzyl)carbamate;
Compound No. 15: tert-butyl (4-(7-methoxy-4-oxo-3,4-dihydrophthalazin-1-yl)benzyl)carbamate;
Compound No. 16: tert-butyl 4-(7-methoxy-3-methyl-4-oxo-3,4-dihydrophthalazin-1-yl)benzyl carbamate;
Compound No. 17: tert-butyl 4-(6-methoxy-4-oxo-3,4-dihydrophthalazin-1-yl)benzyl carbamate;
Compound No. 18: tert-butyl (4-(6-methoxy-3-methyl-4-oxo-3,4-dihydrophthalazin-1-yl)benzyl)carbamate; Compound No. 19: tert-butyl (4-(6-methoxy-4-oxo-3,4-dihydrophthalazin-1-yl)benzyl)carbamate;
Compound No. 20: tert-butyl (4-(6,7-dimethoxy-3-methyl-4-oxo-3,4-dihydrophthalazin-1-yl)benzyl)carbamate;
Compound No. 21: tert-butyl (1-(4-(4-oxo-3,4-dihydrophthalazin-1-yl)phenyl)ethyl)carbamate;
Compound No. 22: tert-butyl (R)-(1-(4-(4-oxo-3,4-dihydrophthalazin-1-yl)phenyl) ethyl)carbamate;
Compound No. 23: tert-butyl (2-(4-(4-oxo-3,4-dihydrophthalazin-1-yl)phenyl)propan-2-yl)carbamate;
Compound No. 24: tert-butyl 4-(4-oxo-3,4-dihydrophthalazin-1-yl)-2-(trifluoromethyl)benzyl carbamate;
Compound No. 25: tert-butyl methyl(4-(4-oxo-3,4-dihydrophthalazin-1-yl)benzyl)carbamate;
Compound No. 26: tert-butyl 6-(4-oxo-3,4-dihydrophthalazin-1-yl)-3,4-dihydroisoquinoline-2(1H)-carboxylate;
Compound No. 27: tert-butyl-6-(3-methyl-4-oxo-3,4-dihydrophthalazin-1-yl)-3,4-dihydroisoquinoline-2(1H)-carboxylate;
Compound No. 28: tert-butyl 6-(6,7-dimethoxy-4-oxo-3,4-dihydrophthalazin-1-yl)-3,4-dihydroisoquinoline-2(1H)-carboxylate;
Compound No. 29: tert-butyl-6-(6,7-dimethoxy-3-methyl-4-oxo-3,4-dihydrophthalazin-1-yl)-3,4-dihydroisoquinoline-2(1H)-carboxylate;
Compound No. 30: 4-(3-aminophenyl)phthalazin-1(2H)-one hydrochloride;
Compound No. 31: 4-(3-aminophenyl)-2-methylphthalazin-1(2H)-one hydrochloride;
Compound No. 32: 4-(3-aminophenyl)-2-benzylphthalazin-1(2H)-one hydrochloride;
Compound No. 33: 4-(3-(aminomethyl)phenyl)phthalazin-1(2H)-one hydrochloride;
Compound No. 34: 4-(3-(aminomethyl)phenyl)-2-methylphthalazin-1-(2H)-one hydrochloride;
Compound No. 35: 4-(3-(aminomethyl)phenyl)-2-benzylphthalazin-1(2H)-one hydrochloride;
Compound No. 36: 4-(4-(aminomethyl)phenyl)phthalazin-1(2H)-one hydrochloride;
Compound No. 37: 4-(4-aminomethyl)phenyl)-2-methylphthalazin-1(2H)-one hydrochloride;
Compound No. 38: 4-(4-(aminomethyl)phenyl)-2-ethylphthalazin-1(2H)-one hydrochloride ;
Compound No. 39: 4-(4-(aminomethyl)phenyl)-2-isopropylphthalazin-1-(2H)-one hydrochloride;
Compound No. 40: 4-(4-aminomethyl)phenyl)-2-phenylphthalazin-1(2H)-one hydrochloride;
Compound No. 41: 4-(4-aminomethyl)phenyl)-2-benzylphthalazin-1(2H)-one hydrochloride;
Compound No. 42: 4-(4-(aminomethyl)phenyl)-2-(4-fluorobenzyl)phthalazin-1(2H)-one hydrochloride;
Compound No. 43: 4-(4-(aminomethyl)phenyl)-6-methoxyphthalazin-1(2H)-one hydrochloride;
Compound No. 44: (4-(7-methoxy-3-methyl-4-oxo-3,4-dihydrophthalazin-1-yl)phenyl)methanaminium chloride;
Compound No. 45: (4-(6-methoxy-4-oxo-3,4-dihydrophthalazin-1-yl)phenyl)methanaminium chloride;
Compound No. 46: 4-(4-(aminomethyl)phenyl)-7-methoxy-2-methylphthalazin-1(2H)-one hydrochloride;
Compound No. 47: 4-(4-(aminomethyl)-phenyl)-7-methoxyphthalazin-1(2H)-one hydrochloride;
Compound No. 48: 4-(4-(aminomethyl)phenyl)-6,7-dimethoxyphthalazin-1(2H)-one hydrochloride;
Compound No. 49: 4-(4-(aminomethyl)phenyl)-6,7-dimethoxy-2-methylphthalazin-1(2H)-one hydrochloride;
Compound No. 50: 4-(4-(1-aminoethyl)phenyl)phthalazin-1(2H)-one hydrochloride;
Compound No. 51: (R)-4-(4-(1-aminoethyl)phenyl)phthalazin-1(2H)-one hydrochloride;
Compound No. 52: 4-(4-(2-aminopropan-2-yl)phenyl)phthalazin-1(2H)-one hydrochloride;
Compound No. 53: 4-(4-(aminomethyl)-3-(trifluoromethyl)phenyl)phthalazin-1(2H)-one hydrochloride;
Compound No. 54 : 4-(4-((methylamino)methyl)phenyl)phthalazin-1(2H)-one hydrochloride;
Compound No. 55: 4-(1,2,3,4-tetrahydroisoquinolin-6-yl)phthalazin-1(2H)-one hydrochloride;
Compound No. 56: 2-methyl-4-(1,2,3,4-tetrahydroisoquinolin-6-yl)phthalazin-1(2H)-one hydrochloride;
Compound No. 57: 6,7-dimethoxy-4-(1,2,3,4-tetrahydroisoquinolin-6-yl)phthalazin-1(2H)-one hydrochloride;
Compound No. 58: 6,7-dimethoxy-2-methyl-4-(1,2,3,4-tetrahydroisoquinolin-6-yl)phthalazin-1(2H)-one hydrochloride;
Compound No. 59: 6,7-dimethoxy-4-(1,2,3,4-tetrahydroisoquinolin-7-yl) phthalazin-1(2H)-one;
Compound No. 60: tert-butyl (N-(3-(4-oxo-3,4-dihydrophthalazin-1-yl)phenyl) sulfamoyl)carbamate;
Compound No. 61: tert-butyl (N-(3-(3-methyl-4-oxo-3,4-dihydrophthalazin-1-yl)phenyl)sulfamoyl)carbamate;
Compound No. 62: tert-butyl (N-(3-(3-benzyl-4-oxo-3,4-dihydrophthalazin-1-yl)phenyl)sulfamoyl)carbamate;
Compound No. 63: tert-butyl (N-(3-(4-oxo-3,4-dihydrophthalazin-1-yl)benzyl)sulfamoyl)carbamate;
Compound No. 64: tert-butyl N-(3-(3-methyl-4-oxo-3,4-dihydrophthalazin-1-yl)benzyl)sulfamoyl carbamate;
Compound No. 65: tert-butyl (N-(3-(3-benzyl-4-oxo-3,4-dihydrophthalazin-1-yl)benzyl)sulfamoyl)carbamate;
Compound No. 66: tert-butyl (N-(4-(3-methyl-4-oxo-3,4-dihydrophthalazin-1-yl)benzyl)sulfamoyl)carbamate;
Compound No. 67: tert-butyl (N-(4-(3-ethyl-4-oxo-3,4-dihydrophthalazin-1-yl)benzyl)sulfamoyl)carbamate;
Compound No. 68: tert-butyl N-(4-(3-isopropyl-4-oxo-3,4-dihydrophthalazin-1-yl)benzyl)sulfamoyl carbamate; Compound No. 69: tert-butyl (N-(4-(4-oxo-3-phenyl-3,4-dihydrophthalazin-1-yl)benzyl)sulfamoyl)carbamate;
Compound No. 70: tert-butyl (N-(4-3-benzyl-4-oxo-3,4-dihydrophthalazin-1-yl)benzyl)sulfamoyl)carbamate;
Compound No. 71: tert-butyl (N-(4-(3-(4-fluorobenzyl)-4-oxo-3,4-dihydrophthalazin-1-yl)benzyl)sulfamoyl)carbamate;
Compound No. 72: tert-butyl (4-(7-methoxy-4-oxo-3,4-dihydrophthalazin-1-yl)benzyl)sulfamoyl)carbamate;
Compound No. 73: tert-butyl N-(4-(7-methoxy-3-methyl-4-oxo-3,4-dihydrophthalazin-1-yl)benzyl)sulfamoyl carbamate;
Compound No. 74: tert-butyl N-(4-(6-methoxy-4-oxo-3,4-dihydrophthalazin-1-yl)benzyl)sulfamoyl carbamate;
Compound No. 75: tert-butyl (N-(4-(6-methoxy-3-methyl-4-oxo-3,4-dihydrophthalazin-1-yl)benzyl)sulfamoyl)carbamate;
Compound No. 76: tert-butyl (N-(4-(6,7-dimethoxy-4-oxo-3,4-dihydrophthalazin-1-yl)benzyl)sulfamoyl)carbamate;
Compound No. 77: tert-butyl (N-(4-(6,7-dimethoxy-3-methyl-4-oxo-3,4-dihydrophthalazin-1-yl)benzyl)sulfamoyl)carbamate;
Compound No. 78: tert-butyl (N-(1-(4-(4-oxo-3,4-dihydrophthalazin-1-yl)phenyl) ethyl)sulfamoyl)carbamate;
Compound No. 79: tert-butyl (R)-(N-(1-(4-(4-oxo-3,4-dihydrophthalazin-1-yl)phenyl)ethyl)sulfamoyl)carbamate;
Compound No. 80: tert-butyl (N-(2-(4-(4-oxo-3,4-dihydrophthalazin-1-yl)phenyl)propan-2-yl)sulfamoyl)carbamate;
Compound No. 81: tert-butyl N-(4-(4-oxo-3,4-dihydrophthalazin-1-yl)-2-(trifluoromethyl)benzyl)sulfamoyl carbamate;
Compound No. 82: tert-butyl (N-methyl-N-(4-(4-oxo-3,4-dihydrophthalazin-1-yl)benzyl)sulfamoyl)carbamate;
Compound No. 83: tert-butyl ((6-(4-oxo-3,4-dihydrophthalazin-1-yl)-3,4-dihydroisoquinolin-2(1H)-yl)sulfonyl)carbamate;
Compound No. 84: tert-butyl ((6-(3-methyl-4-oxo-3,4-dihydrophthalazin-1-yl)-3,4-dihydroisoquinolin-2(1H)-yl)sulfonyl)carbamate;
Compound No. 85: tert-butyl ((6-(6,7-dimethoxy-4-oxo-3,4-dihydrophthalazin-1-yl)-3,4-dihydroisoquinolin-2(1H)-yl)sulfonyl)carbamate;
Compound No. 86: tert-butyl ((6-(6,7-dimethoxy-3-methyl-4-oxo-3,4-dihydrophthalazin-1-yl)-3,4-dihydroisoquinolin-2(1H)-yl)sulfonyl)carbamate;
Compound No. 87: N-(4-(4-oxo-3,4-dihydrophthalazin-1-yl)phenyl)sulfamide;
Compound No. 88: N-(3-(3-methyl-4-oxo-3,4-dihydrophthalazin-1-yl)phenyl)sulfamide hydrochloride;
Compound No. 89: N-(3-(3-benzyl-4-oxo-3,4-dihydrophthalazin-1-yl)phenyl)sulfamide hydrochloride;
Compound No. 90: N-(3-(3-benzyl-4-oxo-3,4-dihydrophthalazin-1-yl)benzyl)sulfamide hydrochloride;
Compound No. 91: N-(4-(4-oxo-3,4-dihydrophthalazin-1-yl)phenyl)sulfamide
Compound No. 92: N-(4-(3-methyl-4-oxo-3,4-dihydrophthalazin-1-yl)phenyl)sulfamide;
Compound No. 93: N-(4-(3-benzyl-4-oxo-3,4-dihydrophthalazin-1-yl)phenyl)sulfamide;
Compound No. 94: N-(3-(3-methyl-4-oxo-3,4-dihydrophthalazin-1-yl)benzyl)sulfamide trifluoroacetic acid;
Compound No. 95: N-(3-(3-oxo-3,4-dihydrophthalazin-1-yl)benzyl)sulfamide hydrochloride;
Compound No. 96: N-(4-(4-oxo-3,4-dihydrophthalazin-1-yl)benzyl) sulfamide hydrochloride;
Compound No. 97: N-(4-(3-methyl-4-oxo-3,4-dihydrophthalazin-1-yl)benzyl)sulfamide hydrochloride;
Compound No. 98: N-(4-(3-ethyl-4-oxo-3,4-dihydrophthalazin-1-yl)benzyl)sulfamide hydrochloride;
Compound No. 99: N-(4-(3-isopropyl-4-oxo-3,4-dihydrophthalazin-1-yl)benzyl)sulfamide trifluoroacetic acid;
Compound No. 100: N-(4-(4-oxo-3-phenyl-3,4-dihydrophthalazin-1-yl)benzyl)sulfamide hydrochloride;
Compound No. 101: N-(4-(3-benzyl-4-oxo-3,4-dihydrophthalazin-1-yl)benzyl)sulfamide hydrochloride;
Compound No. 102: N-(4-(3-(4-fluorobenzyl)-4-oxo-3,4-dihydrophthalazin-1-yl)benzyl)sulfamide hydrochloride;
Compound No. 103: N-(4-(7-methoxy-4-oxo-3,4-dihydrophthalazin-1-yl)benzyl)sulfamide hydrochloride;
Compound No. 104: N-(4-(6,7-dimethoxy-4-oxo-3,4-dihydrophthalazin-1-yl)benzyl)sulfamide hydrochloride;
Compound No. 105: N-(4-(7-methoxy-3-methyl-4-oxo-3,4-dihydrophthalazin-1-yl)benzyl)sulfamide hydrochloride
Compound No. 106: N-(4-(6-methoxy-4-oxo-3,4-dihydrophthalazin-1-yl)benzyl)sulfamide hydrochloride
Compound No. 107: N-(4-(6-methoxy-3-methyl-4-oxo-3,4-dihydrophthalazin-1-yl)benzyl)sulfamide hydrochloride
Compound No. 108: N-(4-(6,7-dimethoxy-3-methyl-4-oxo-3,4-dihydrophthalazin-1-yl)benzyl)sulfamide hydrochloride;
Compound No. 109: N-(3-(6,7-dimethoxy-4-oxo-3,4-dihydrophthalazin-1-yl)-benzyl) sulfamide;
Compound No. 110: N-(3-(6,7-dimethoxy-3-methyl-4-oxo-3,4-dihydrophthalazin-1-yl)-benzyl) sulfamide;
Compound No. 111: N-(4-(6,7-dimethoxy-4-oxo-3,4-dihydrophthalazin-1-yl)-2-fluorobenzyl)sulfamide hydrochloride;
Compound No. 112: N-(4-(6,7-dimethoxy-4-oxo-3,4-dihydrophthalazin-1-yl)-3-fluorobenzyl)sulfamide hydrochloride;
Compound No. 113: N-(4-(6,7-dimethoxy-4-oxo-3,4-dihydrophthalazin-1-yl)-2-fluorobenzyl)-N-methyl sulfamide hydrochloride;
Compound No. 114: N-(4-(6,7-dimethoxy-4-oxo-3,4-dihydrophthalazin-1-yl)-3-fluorobenzyl)-N-methyl sulfamide hydrochloride;
Compound No. 115: N-(4-(6,7-dimethoxy-4-oxo-3,4-dihydrophthalazin-1-yl)-2,3-difluorobenzyl)sulfamide hydrochloride;
Compound No. 116: N-(4-(6,7-dimethoxy-4-oxo-3,4-dihydrophthalazin-1-yl)-3-(trifluorobenzyl methyl)benzyl) sulfamide hydrochloride;
Compound No. 117: N-(4-(6,7-dimethoxy-4-oxo-3,4-dihydrophthalazin-1-yl)-2,3-difluorobenzyl)-N-methyl sulfamide hydrochloride;
Compound No. 118: N-(4-(6,7-dimethoxy-4-oxo-3,4-dihydrophthalazin-1-yl)-3-(trifluorobenzyl methyl)benzyl)-N-methyl sulfamide hydrochloride;
Compound No. 119: N-(4-(6,7-dimethoxy-4-oxo-3,4-dihydrophthalazin-1-yl)-2-(trifluorobenzyl methyl)benzyl)sulfamide hydrochloride;
Compound No. 120: N-(4-(6,7-dimethoxy-4-oxo-3,4-dihydrophthalazin-1-yl)-2-(trifluorobenzyl methyl)benzyl)-N-methyl sulfamide hydrochloride;
Compound No. 121: 7-(4-oxo-3,4-dihydrophthalazin-1-yl)-3,4-dihydroisoquinoline-2(1H) sulfamide hydrochloride;
Compound No. 122: 7-(6,7-dimethoxy-4-oxo-3,4-dihydrophthalazin-1-yl)-3,4-dihydroisoquinoline-2(1H)-sulfamide;
Compound No. 123: N-(1-(4-(4-oxo-3,4-dihydrophthalazin-1-yl)phenyl) ethyl)sulfamide hydrochloride;
Compound No. 124: (R)-N-(1-(4-(4-oxo-3,4-dihydrophthalazin-1-yl)phenyl) ethyl)sulfamide hydrochloride;
Compound No. 125: N-(2-(4-(4-oxo-3,4-dihydrophthalazin-1-yl)phenyl)propan-2-yl)sulfamide hydrochloride;
Compound No. 126: N-(4-(4-oxo-3,4-dihydrophthalazin-1-yl)-2-(trifluoromethyl)benzyl)sulfamide hydrochloride;
Compound No. 127: N-methyl-N-(4-(4-oxo-3,4-dihydrophthalazin-1-yl)benzyl)sulfamide;
Compound No. 128: 6-(4-oxo-3,4-dihydrophthalazin-1-yl-3,4-dihydroisoquinoline-2(1H)-sulfonamide hydrochloride;
Compound No. 129: 6-(3-methyl-4-oxo-3,4-dihydrophthalazin-1-yl)-3,4-dihydroisoquinoline-2(1H)-sulfonamide hydrochloride;
Compound No. 130: 6-(6,7-dimethoxy-4-oxo-3,4-dihydrophthalazin-1-yl)-3,4-dihydroisoquinoline-2(1H)-sulfonamide hydrochloride;
Compound No. 131: 6-(6,7-dimethoxy-3-methyl-4-oxo-3,4-dihydrophthalazin-1-yl)-3,4-dihydroisoquinoline-2(1H)-sulfonamide hydrochloride;
Compound No. 132: tert-butyl ((1-(4-oxo-3,4-dihydrophthalazin-1-yl)piperidin-4-yl)methyl)carbamate;
Compound No. 133: tert-butyl ((1-(6,7-dimethoxy-4-oxo-3,4-dihydrophthalazin-1-yl)piperidin-4-yl)methyl)carbamate;
Compound No. 134: 4-(4-(aminomethyl)piperidin-1-yl)phthalazin-1(2H)-one hydrochloride;
Compound No. 135: 4-(4-(aminomethyl)piperidin-1-yl)-6,7-dimethoxyphthalazin-1(2H)-one hydrochloride;
Compound No. 136: tert-butyl N-((1-(4-oxo-3,4-dihydrophthalazin-1-yl)piperidin-4-yl)methyl)sulfamoyl carbamate;
Compound No. 137: tert-butyl N-((1-(6,7-dimethoxy-4-oxo-3,4-dihydrophthalazin-1-yl)piperidin-4-yl)methyl)sulfamoyl carbamate;
Compound No. 138: N-((1-(4-oxo-3,4-dihydrophthalazin-1-yl)piperidin-4-yl)methyl)sulfamide hydrochloride;
Compound No. 139: N-((1-(6,7-dimethoxy-4-oxo-3,4-dihydrophthalazin-1-yl)piperidin-4-yl)methyl)sulfamide hydrochloride;
Compound No. 140: N-(4-(6,7-dimethoxy-3-methyl-4-oxo-3,4-dihydrophthalazin-1-yl)-amino)phenyl)sulfamide hydrochloride;
Compound No. 141: N-(3-(6,7-dimethoxy-3-methyl-4-oxo-3,4-dihydrophthalazin-1-yl)-amino)phenyl)sulfamide hydrochloride;
Compound No. 142: N-(2-(1-(4-oxo-3,4-dihydrophthalazin-1-yl)piperidin-4-yl)ethyl)sulfamide trifluoroacetic acid;
Compound No. 143: 4-benzylphthalazin-1(2H)-one;
Compound No. 144: 4-benzyl-6,7-dimethoxyphthalazin-1(2H)-one;
Compound No. 145: tert-butyl (3-((6,7-dimethoxy-4-oxo-3,4-dihydrophthalazin-1-yl)methyl)phenyl)carbamate;
Compound No. 146: tert-butyl (3-((6,7-dimethoxy-3-methyl-4-oxo-3,4-dihydrophthalazin-1-yl)methyl)phenyl)carbamate;
Compound No. 147: tert-butyl (4-((4-oxo-3,4-dihydrophthalazin-1-yl)methyl)phenyl)carbamate;
Compound No. 148: tert-butyl (4-((3-methyl-4-oxo-3,4-dihydrophthalazin-1-yl)methyl)phenyl)carbamate;
Compound No. 149: tert-butyl (4-((6,7-dimethoxy-4-oxo-3,4-dihydrophthalazin-1-yl)methyl)phenyl)carbamate;
Compound No. 150: tert-butyl (4-((6,7-dimethoxy-3-methyl-4-oxo-3,4-dihydrophthalazin-1-yl)methyl)phenyl)carbamate;
Compound No. 151: tert-butyl (4-((4-oxo-3,4-dihydrophthalazin-1-yl)methyl)phenyl) (methyl)carbamate;
Compound No. 152: tert-butyl (4-((6,7-dimethoxy-4-oxo-3,4-dihydrophthalazin-1-yl)methyl)phenyl) (methyl) carbamate;
Compound No. 153: tert-butyl (4-((6,7-dimethoxy-3-methyl-4-oxo-3,4-dihydrophthalazin-1-yl)methyl)phenyl) (methyl) carbamate;
Compound No. 154: tert-butyl (5-((6,7-dimethoxy-4-oxo-3,4-dihydrophthalazin-1-yl)methyl)indoline-1-carboxylate;
Compound No. 155: tert-butyl (5-((6,7-dimethoxy-3-methyl-4-oxo-3,4-dihydrophthalazin-1-yl)methyl)indoline-1-carboxylate;
Compound No. 156: 4-(3-aminobenzyl)-6,7-dimethoxyphthalazin-1(2H)-one hydrochloride;
Compound No. 157: 4-(3-aminobenzyl)-6,7-dimethoxy-2-methylphthalazin-1(2H)-one hydrochloride;
Compound No. 158: 4-(4-aminobenzyl)-phthalazin-1(2H)-one hydrochloride;
Compound No. 159: 4-(4-aminobenzyl)-2-methylphthalazin-1(2H)-one hydrochloride;
Compound No. 160: 4-(4-aminobenzyl)-6,7-dimethoxyphthalazin-1(2H)-one hydrochloride;
Compound No. 161: 4-(4-aminobenzyl)-6,7-dimethoxy-2-methylphthalazin-1(2H)-one hydrochloride;
Compound No. 162: 4-(4-(methylamino)benzyl)phthalazin-1(2H)-one hydrochloride;
Compound No. 163: 6,7-dimethoxy-4-(4-(methylamino)benzyl)phthalazin-1(2H)-one hydrochloride;
Compound No. 164: 6,7-dimethoxy-2-methyl-4-(4-(methylamino)benzyl)phthalazin-1(2H)-one hydrochloride;
Compound No. 165: 4-(indolin-5-yl-methyl)-6,7-dimethoxyphthalazin-1(2H)-one hydrochloride;
Compound No. 166: 4-(indolin-5-yl-methyl)-6,7-dimethoxy-2-methylphthalazin-1(2H)-one hydrochloride;
Compound No. 167: tert-butyl (N-(3-((6,7-dimethoxy-4-oxo-3,4-dihydrophthalazin-1-yl)methyl)phenyl)sulfamoyl)carbamate;
Compound No. 168: tert-butyl (N-(3-((6,7-dimethoxy-3-methyl-4-oxo-3,4-dihydrophthalazin-1-yl)methyl)phenyl)sulfamoyl)carbamate;
Compound No. 169: tert-butyl (N-(4-((4-oxo-3,4-dihydrophthalazin-1-yl)methyl)phenyl)sulfamoyl)carbamate;
Compound No. 170: tert-butyl (N-(4-((3-methyl-4-oxo-3,4-dihydrophthalazin-1-yl)methyl)phenyl)sulfamoyl)carbamate;
Compound No. 171: tert-butyl (N-(4-((6,7-dimethoxy-4-oxo-3,4-dihydrophthalazin-1-yl)methyl)phenyl)sulfamoyl)carbamate;
Compound No. 172: tert-butyl (N-(4-((6,7-dimethoxy-3-methyl-4-oxo-3,4-dihydrophthalazin-1-yl)methyl)phenyl)sulfamoyl)carbamate;
Compound No. 173: tert-butyl N-methyl-N-(4-((4-oxo-3,4-dihydrophthalazin-1-yl)methyl)phenyl)sulfamoyl carbamate;
Compound No. 174: tert-butyl N-(4-((6,7-dimethoxy-4-oxo-3,4-dihydrophthalazin-1-yl)methyl)phenyl)-N-methylsulfamoyl carbamate;
Compound No. 175: tert-butyl N-(4-((6,7-dimethoxy-3-methyl-4-oxo-3,4-dihydrophthalazin-1-yl)methyl)phenyl)-N-methylsulfamoyl carbamate;
Compound No. 176: tert-butyl (5-((6,7-dimethoxy-4-oxo-3,4-dihydrophthalazin-1-yl)methyl)indolin-1-yl)sulfonyl carbamate;
Compound No. 177: tert-butyl (5-((6,7-dimethoxy-3-methyl-4-oxo-3,4-dihydrophthalazin-1-yl)methyl)indolin-1-yl)sulfonyl carbamate;
Compound No. 178: N-(3-((6,7-dimethoxy-4-oxo-3,4-dihydrophthalazin-1-yl)methyl)phenyl)sulfamide hydrochloride;
Compound No. 179: N-(3-((6,7-dimethoxy-3-methyl-4-oxo-3,4-dihydrophthalazin-1-yl)methyl)phenyl)sulfamide hydrochloride;
Compound No. 180: N-(4-((4-oxo-3,4-dihydrophthalazin-1-yl)methyl)phenyl)sulfamide hydrochloride;
Compound No. 181: N-(4-((3-methyl-4-oxo-3,4-dihydrophthalazin-1-yl)methyl)phenyl)sulfamide hydrochloride;
Compound No. 182: N-(4-((6,7-dimethoxy-4-oxo-3,4-dihydrophthalazin-1-yl)methyl)phenyl)sulfamide hydrochloride;
Compound No. 183: N-(4-((6,7-dimethoxy-3-methyl-4-oxo-3,4-dihydrophthalazin-1-yl)methyl)phenyl)sulfamide hydrochloride;
Compound No. 184: N-methyl-N-(4-((4-oxo-3,4-dihydrophthalazin-1-yl)methyl)phenyl)sulfamide hydrochloride;
Compound No. 185: N-(4-((6,7-dimethoxy-4-oxo-3,4-dihydrophthalazin-1-yl)methyl)phenyl)-N-methylsulfamide hydrochloride;
Compound No. 186: N-(4-((6,7-dimethoxy-3-methyl-4-oxo-3,4-dihydrophthalazin-1-yl)methyl)phenyl)-N-methylsulfamide hydrochloride;
Compound No. 187: 5-((6,7-dimethoxy-4-oxo-3,4-dihydrophthalazin-1-yl)methyl)indoline-1-sulfonamide hydrochloride;
Compound No. 188: 5-((6,7-dimethoxy-3-methyl-4-oxo-3,4-dihydrophthalazin-1-yl)methyl)indoline-1-sulfonamide hydrochloride;
Compound No. 189: N-(3-fluoro-4-((4-oxo-3,4-dihydrophthalazin-1-yl)methyl)phenyl)sulfamide hydrochloride;
Compound No. 190: N-(2-fluoro-4-((4-oxo-3,4-dihydrophthalazin-1-yl)methyl)phenyl)sulfamide hydrochloride;
Compound No. 191: N-(3-(trifluorobenzyl methyl)-4-((4-oxo-3,4-dihydrophthalazin-1-yl)methyl)phenyl)sulfamide hydrochloride;
Compound No. 192: N-(2-(trifluorobenzyl methyl)-4-((4-oxo-3,4-dihydrophthalazin-1-yl)methyl)phenyl)sulfamide hydrochloride;
Compound No.193: 4-(3-bromobenzyl)-6,7-dimethoxyphthalazin-1(2H)-one;
Compound No.194: 4-(4-bromobenzyl)-6,7-dimethoxyphthalazin-1(2H)-one;
Compound No. 195: N-(4-(4-oxo-3,4-dihydrophthalazin-1-yl)benzyl)methanesulfonamide;
Compound No. 196: N-(4-(4-oxo-3,4-dihydrophthalazin-1-yl)benzyl)cyclopropanesulfonamide;
Compound No. 197: 4-methyl-N-(4-(4-oxo-3,4-dihydrophthalazin-1-yl)benzyl)benzenesulfonamide;
Compound No. 198: N-(4-(4-oxo-3,4-dihydrophthalazin-1-yl)benzyl)thiophene-2-sulfonamide;
Compound No. 199: (4-(3-methyl-4-oxo-3,4-dihydrophthalazin-1-yl)phenyl)sulfamide;
Compound No. 200: N-methyl-1-(4-(3-methyl-4-oxo-3,4-dihydrophthalazin-1-yl)phenyl)methanesulfonamide;
Compound No. 201: 2-methyl-4-(4-((morpholinosulfonyl)methyl)phenyl)phthalazin-1(2H)-one;
Compound No. 202: 2-(4-(3-methyl-4-oxo-3,4-dihydrophthalazin-1-yl)phenyl)ethane-1-sulfonamide; and
Compound No. 203: 4-(4-(aminomethyl)-3-(trifluoromethyl)phenyl-6,7-dimethoxy phthalazin-1(2H)-one hydrochloride.

The compound of Formula 1 according to the present invention may be used in the form of a pharmaceutically acceptable salt derived from an inorganic or organic acid and the pharmaceutically acceptable salt preferably includes at least one selected from the group consisting of hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, hydroxymaleic acid, benzoic acid, hydroxybenzoic acid, phenylacetic acid, cinnamic acid, salicylic acid, methanesulfonic acid, benzenesulfonic acid, and toluenesulfonic acid.

The compound of Formula 1 according to the present invention or a pharmaceutically acceptable salt thereof may include a hydrate and a solvate. The hydrate may be formed by bonding the compound of Formula 1 with a water molecule.

In another aspect, the present invention provides a pharmaceutical composition for preventing, alleviating or treating cancer containing the compound according to one aspect of the present invention as an active ingredient.

In another aspect, the present invention provides an ENPP 1 inhibitor containing the compound according to one aspect of the present invention as an active ingredient.

In another aspect, the present invention provides a STING pathway activator containing the compound according to one aspect of the present invention as an active ingredient.

In another aspect, the present invention provides a pharmaceutical composition for preventing, alleviating or treating cancer wherein the cancer is associated with inhibition of ENPP 1.

In another aspect, the present invention provides a pharmaceutical composition for preventing, alleviating or treating cancer, containing the compound selected from the compound of Formula 1 according to the present invention, the pharmaceutically acceptable salt thereof, the hydrate thereof or the stereoisomer thereof as an active ingredient.

The pharmaceutical composition according to the present invention has excellent ability to inhibit the activity of ENPP 1.

Therefore, the pharmaceutical composition of the present invention may be used for the treatment, prevention and alleviation of cancer diseases caused by abnormal cell growth. The cancer diseases that can be prevented, treated or alleviated by administration of the pharmaceutical composition of the present invention include gastric cancer, lung cancer, liver cancer, colorectal cancer, small intestine cancer, pancreatic cancer, brain cancer, bone cancer, melanoma, breast cancer, sclerosis, uterine cancer, cervical cancer, head and neck cancer, esophageal cancer, thyroid cancer, parathyroid cancer, kidney cancer, sarcoma, prostate cancer, urethral cancer, bladder cancer, blood cancer (including leukemia, multiple myeloma, myelodysplastic syndrome), lymphoma (including Hodgkin's disease and non-Hodgkin's lymphoma), psoriasis, and fibroadenoma.

In another aspect, the present invention provides an ENPP 1 inhibitor containing any one of the compounds described above as an active ingredient.

In another aspect, the present invention provides a STING pathway activator containing any one of the compounds described above as an active ingredient.

The pharmaceutical composition may be applied to experimental animals such as mice, rabbits, rats, guinea pigs or hamsters, preferably primates including humans, more preferably humans, without being limited thereto.

As used herein, the term "treatment" includes alleviating or ameliorating symptoms, reducing the extent of a disease, delaying or alleviating disease progression, ameliorating, alleviating, or stabilizing a disease condition, partial or complete recovery, prolonging survival, and other beneficial treatment results.

In addition, as used herein, treatment of cancer means treatment of all cancer cells, and the cancer includes angiogenesis of endothelial cells and mitosis thereof (solid tumors, tumor metastasis, and benign tumors). For example, the cancer includes, but is not limited to, breast cancer, ovarian cancer, cervical cancer, prostate cancer, testicular cancer, genitourinary cancer, esophageal cancer, laryngeal cancer, glioblastoma, stomach cancer, skin cancer, keratoacanthomas, lung cancer, squamous cell carcinoma, large cell carcinoma, small cell carcinoma, lung adenocarcinoma, bone cancer, colon cancer, adenoma, pancreatic cancer, adenocarcinoma, carcinogenic cancer, follicular carcinoma, undifferentiated cancer, papillary cancer, normal hematoma, melanoma, sarcoma, bladder cancer, liver cancer, bile duct cancer, kidney cancer, myeloid diseases, lymphoid diseases, Hodgkin's disease, hair cell cancer, oral cavity cancer, pharyngeal (oral) cancer, lip cancer, tongue cancer, small intestine cancer, colorectal cancer, colon cancer, rectal cancer, brain cancer, central nervous system cancer, leukemia, hemangioma, trachoma, and purulent sarcoma.

The content of the active ingredient, namely, the compound represented by Formula 1, the pharmaceutically acceptable salt thereof, the hydrate thereof, or the solvate thereof is appropriately adjusted through selection by those skilled in the art depending on the use mode and use method of the pharmaceutical composition of the present invention.

For example, the compound represented by Formula 1, the pharmaceutically acceptable salt thereof, the hydrate thereof, or the solvate thereof may be present in an amount of 0.1 to 10% by weight, more preferably 0.5 to 5% by weight, based on the total weight of the pharmaceutical composition.

The compound represented by Formula 1, the pharmaceutically acceptable salt thereof, the hydrate thereof, or the solvate thereof may be present alone or in combination with a pharmacologically acceptable carrier, excipient, diluent, or an accessory ingredient in the pharmaceutical composition.

For example, the pharmaceutically acceptable carrier, excipient, and diluent include, but are not limited to, one or more selected from the group consisting of lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, mineral oil, dextrin, calcium carbonate, propylene glycol, liquid paraffin, and physiological saline, and a common carrier, excipient, or diluent may be used. In addition, the pharmaceutical composition may further include a conventional filler, extender, binder, disintegrating agent, anticoagulant, lubricant, wetting agent, pH adjusting agent, nutrient, vitamin, electrolyte, alginic acid or salt thereof, pectic acid or salt thereof, protective colloid, glycerin, fragrance, emulsifier, preservative, or the like.

When administered in combination with another anticancer drug for treating cancer or tumors, the compound represented by Formula 1 or the pharmaceutically acceptable salt thereof can exert a synergistic therapeutic effect with the anticancer drug.

Specifically, the pharmaceutical composition may further include at least one other anti-cancer agent or therapeutic agent known to be effective for treating or preventing cancer, in addition to the active ingredient, and thus may be used as a simultaneously or separately applied combination therapy. Other anti-cancer agents or therapeutic agents that may be applied to combination therapy may include, for example, at least one compound selected from the group consisting of Gleevec^{®} (imatinib), Sutent^{®} (sunitinib), Herceptin^{®} (trastuzumab), Velcade^{®} (bortezomib), dexamethasone, Nexavar^{®} (sorafenib), aromatase inhibitors, and kinase inhibitors, but are not limited thereto.

The pharmaceutical composition may be administered orally or parenterally, and for example, may be administered through various routes including oral, transdermal, subcutaneous, intravenous, or intramuscular routes. In addition, the formulation of the composition may vary depending on the method of use, and may be formulated using methods well known in the art to provide rapid, sustained, or delayed release of the active ingredient after administration to mammals. In general, solid preparations for oral administration include tablets, troches, soft or hard capsules, pills, powders, granules, and the like. These preparations can be prepared, for example, by mixing starch, calcium carbonate, sucrose, lactose, gelatin, or the like. In addition, lubricants such as magnesium stearate and talc may also be used, in addition to simple excipients. Liquid preparations for oral administration include suspensions, liquids and solutions, emulsions, syrups, and the like. In addition to water and liquid paraffin, which are common simple diluents, various excipients such as wetting agents, sweeteners, fragrances, and preservatives may be included. Formulations for parenteral administration include creams, lotions, ointments, plasters, liquids and solutions, aerosols, fluid extracts, elixirs, infusions, sachets, patches, injections, and the like. Injection formulations are preferably in the form of an isotonic aqueous solution or suspension.

The pharmaceutical composition may further include an adjuvant such as a sterilizer, a preservative, a stabilizer, a hydrating agent or an emulsifying accelerator, a salt and/or a buffer for controlling osmotic pressure, and other therapeutically useful substances. Alternatively, the pharmaceutical composition may be formulated in accordance with an ordinary mixing, granulation, or coating method or using a suitable method known in the art.

In addition, the dosage of the pharmaceutical composition may be determined in consideration of the administration method, the age, gender, disease severity, and condition of the patient, the rate of absorption of the active ingredient in the body, and the inactivation rate of the active ingredient and drugs used in combination therewith, and the pharmaceutical composition may be administered once or multiple times in a portionwise manner. The active ingredient of the pharmaceutical composition is preferably orally or parenterally administered to a mammal including a human in an amount of 0.001 to 100 mg/kg body weight, preferably 0.01 to 35 mg/kg body weight, on a daily basis, and may be administered once or divided into multiple administrations throughout the day.

In another aspect, the present invention provides a method of treating cancer including administering a therapeutically effective amount of the compound represented by Formula 1, the pharmaceutically acceptable salt thereof, or the hydrate thereof.

Preferably, the treatment method may further include identifying a patient in need of prevention or treatment of cancer before the administration.

As used herein, the term "therapeutically effective amount" means an amount of an active ingredient which is effective for the prevention or treatment of cancer in a mammal, and the therapeutically effective amount may be controlled by a variety of factors such as the type of disease, the severity of the disease, the type and content of the active ingredient and other ingredients contained in the composition, the type of formulation, the age, weight, general state of health, gender, and diet of the patient, the time of administration, the route of administration, clearance rate of the composition in blood, the duration of treatment, and drugs used simultaneously therewith. Preferably, as described above, the compound may be administered in an amount of 0.001 to 100 mg/kg body weight, preferably 0.01 to 35 mg/kg body weight, on a daily basis, once or multiple times in a portionwise manner a day, by oral or parenteral routes.

In another aspect, the present invention provides a method of preparing the 7-amino-3,4-dihydropyrimidopyrimidin-2-one derivative compound represented by Formula 1, the pharmaceutically acceptable salt thereof, or the hydrate thereof.

Meanwhile, the present invention provides a method for preparing the compound represented by Formula 1. The preparation method according to the present invention will be described in detail as follows.

In one embodiment, the compound represented by Formula 1 according to the present invention may be prepared by at least one of the following Preparation Methods 1 to 4.

### Preparation Method 1

The phthalazine derivative compound of Formula 1 according to one aspect of the present invention may be prepared in accordance with the preparation methods based on the following Reaction Schemes 1-1 to 1-3.

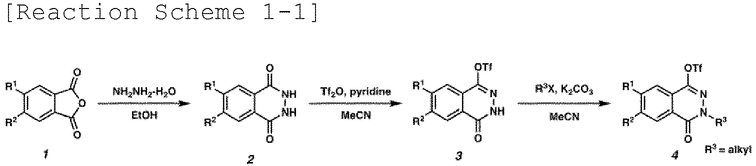

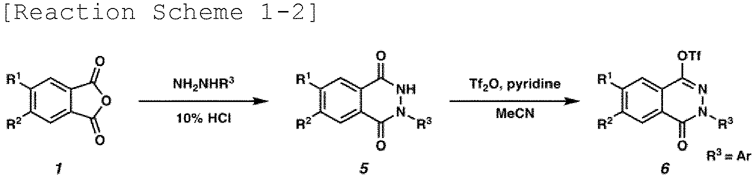

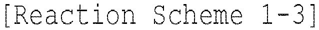

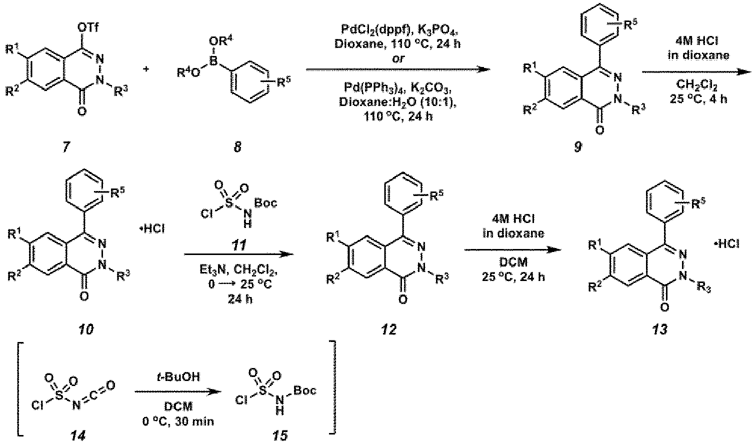

### Preparation Method 2

The phthalazine derivative compound of Formula 1 according to one aspect of the present invention may be prepared in accordance with the preparation methods based on the following Reaction Scheme 2.

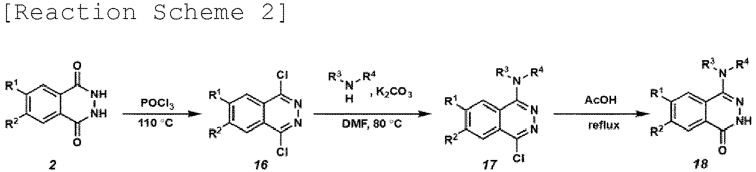

### Preparation Method 3

The phthalazine derivative compound of Formula 1 according to one aspect of the present invention may be prepared in accordance with the preparation methods based on the following Reaction Schemes 3-1 to 3-2.

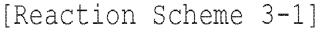

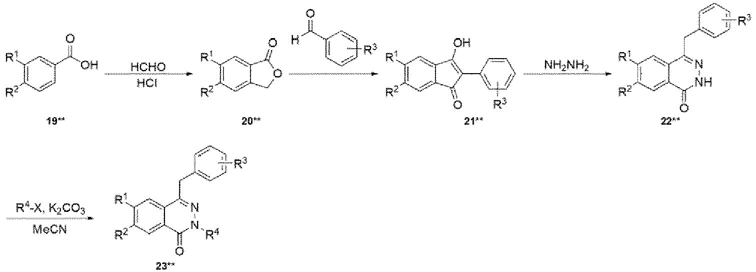

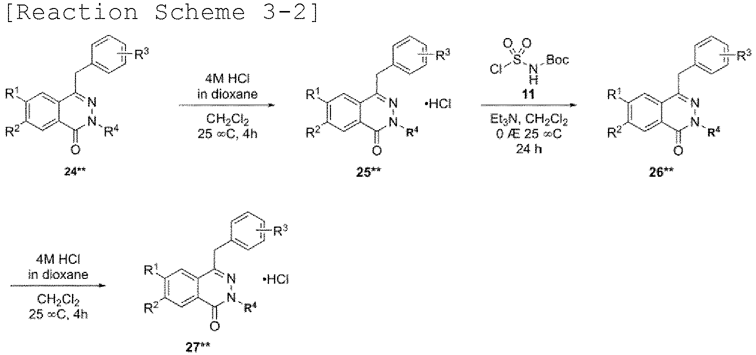

### [Mode for Invention]

Hereinafter, the present invention will be described in more detail with reference to Synthesis Examples, Preparation Examples, Examples, Experimental examples and Formulation Examples. However, the following Synthesis Examples, Preparation Examples, Examples, Experimental examples and Formulation Examples are provided only for illustration and should not be construed as limiting the scope of the present invention.

### [Preparation Example 1]

Preparation Example 1 includes a process of preparing Product 4 (Preparation Example 1-1) or Product 6 (Preparation Example 1-2). Then, a phthalazine derivative was prepared through Preparation Example 1-3 using the product 4 or 6 as Reactant 7.

### Preparation Example 1-1: Preparation of Reactant 4

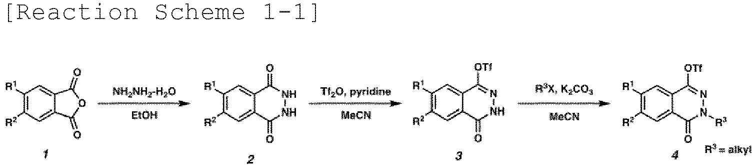

**Step 1:** Reactant **1** (1.0 eq.) and hydrazine-hydrate (1.7 eq.) were added to ethanol in a round-bottom flask, followed by refluxing for 2 hours. The reaction product was cooled to room temperature and filtered to obtain a solid 2 (71-94% yield).

Examples of Reactant 1 used in step 1 are as follows.

### Reactant 1-1: 2,3-dihydrophthalazine-1,4-dione

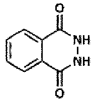

¹H NMR (400 MHz, DMSO) δ 11.51 (s, 2H), 8.15-7.95 (m, 2H), 7.92-7.79 (m, 2H).

### Reactant 1-2: 6,7-dimethoxy-2,3-dihydrophthalazine-1,4-dione

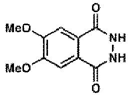

¹H NMR (400 MHz, DMSO) δ 11.25 (br s, 2H), 7.42 (s, 2H), 3.93 (s, 6H).

### Reactant 1-3: 6-methoxy-2,3-dihydrophthalazine-1,4-dione

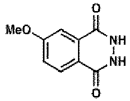

¹H NMR (400 MHz, DMSO) δ 11.44 (s, 2H), 8.00 (d, *J* = 9.2 Hz, 1H), 7.60-7.20 (m, 2H), 3.92 (s, 3H).

**Step 2-1:** Reactant **2** (1.0 eq.) obtained in step 1 and anhydrous pyridine (5.0 eq.) were dissolved in anhydrous acetonitrile (0.61 M) under a nitrogen atmosphere in a round bottom flask and the resulting solution was cooled to 0°C, followed by stirring for 10 minutes. Triflic anhydride (1.05 eq.) was added thereto at 0°C and then the resulting mixture was warmed to room temperature and stirred for 6 hours. The mixture was poured into ethyl acetate, the result was washed with a 5% aqueous hydrochloric acid solution, and the aqueous layer was extracted with ethyl acetate. The organic layer was dried over anhydrous sodium sulfate, concentrated under reduced pressure, and recrystallized using ethyl acetate to obtain **3** (yield: 67-79%).

### Reactant 3-1: 4-oxo-3,4-dihydrophthalazin-1-yl trifluoromethanesulfonate

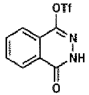

¹H NMR (400 MHz, DMSO) δ 12.91 (s, 1H), 8.36-8.29 (m, 1H), 8.12 (td, *J* = 7.6, 1.4 Hz, 1H), 8.05 (td, *J* = 7.6, 1.3 Hz, 1H), 7.88 (apparent d, *J* = 7.7 Hz 1H).

### Reactant 3-2: 6-methoxy-4-oxo-3,4-dihydrophthalazin-1-yl trifluoromethanesulfonate

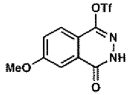

¹H NMR (400 MHz, DMSO) δ 12.82 (s, 1H), 7.82 (d, *J* = 8.8 Hz, 1H), 7.70 (d, *J* = 2.6 Hz, 1H), 7.67 (dd, *J* = 8.8, 2.7 Hz, 1H), 3.99 (s, 3H).

### Reactant 3-3: 7-methoxy-4-oxo-3,4-dihydrophthalazin-1-yl trifluoromethanesulfonate

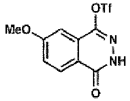

¹H NMR (400 MHz, DMSO) δ 12.80 (s, 1H), 8.24 (d, *J =* 8.9 Hz, 1H), 7.61 (dd, *J* = 8.8, 2.6 Hz, 1H), 7.13 (d, *J* = 2.5 Hz, 1H), 3.97 (s, 3H).

### Reactant 3-4: 6,7-dimethoxy-4-oxo-3,4-dihydrophthalazin-1-yl trifluoromethanesulfonate

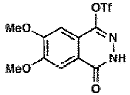

¹H NMR (400 MHz, DMSO) δ 12.77 (s, 1H), 7.67 (s, 1H), 7.13 (s, 1H), 4.00 (s, 3H), 3.99 (s, 3H).

**Step 2-2:** Reactant **3** (1.0 eq.) obtained in step 2-1 above was dissolved in anhydrous acetonitrile (0.2 M) in a round bottom flask, and K₂CO₃ (3.0 eq.) and alkyl halide (3.0 eq.) were added thereto, followed by stirring at 80°C for 4 hours. The resulting product was washed with ethyl acetate and filtered through celite to remove solids, and the compound was purified by column chromatography (60-87% yield) .

### Reactant 3-5: 3-methyl-4-oxo-3,4-dihydrophthalazin-1-yl-trifluoromethanesulfonate

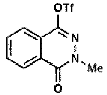

¹H NMR (400 MHz, DMSO) δ 8.36 (dt, *J* = 8.0, 1.1 Hz, 1H), 8.16 - 8.02 (m, 2H), 7.90 (d, *J* = 7.8 Hz, 1H), 3.69 (s, 3H).

### Reactant 3-6: 3-ethyl-4-oxo-3,4-dihydrophthalazin-1-yl-trifluoromethanesulfonate

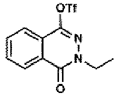

¹H NMR (400 MHz, CDCl₃) δ 8.47 (dt, *J* = 6.9, 1.7 Hz, 1H), 7.96 - 7.80 (m, 3H), 7.25 (dd, *J =* 5.2, 2.8 Hz, 1H), 4.24 (q, *J =* 7.2 Hz, 2H), 1.41 (td, *J* = 7.2 Hz, 3H).

### Reactant 3-7: 3-isopropyl-4-oxo-3,4-dihydrophthalazin-1-yl-trifluoromethanesulfonate

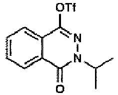

¹H NMR (400 MHz, CDCl₃) δ 8.46 (dd, *J* = 7.8, 1.5 Hz, 1H), 8.04 (dd, *J* = 7.8, 1.4 Hz, 1H), 7.80 (dtd, *J* = 15.0, 7.6, 1.5 Hz, 2H), 5.18 (p, *J* = 6.2 Hz, 1H), 1.43 - 1.41 (m, 3H), 1.41 - 1.39 (m, 3H).

### Reactant 3-8: 3-benzyl-4-oxo-3,4-dihydrophthalazin-1-yl-trifluoromethanesulfonate

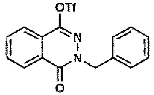

¹H NMR (600 MHz, CDCl₃) δ 8.48 - 8.44 (m, 1H), 7.94 - 7.84 (m, 2H), 7.83 (dd, *J* = 8.0, 1.3 Hz, 1H), 7.49 - 7.45 (m, 2H), 7.44 - 7.26 (m, 3H), 5.31 (s, 2H).

### Reactant 3-9: 6,7-dimethoxy-3-methyl-4-oxo-3,4-dihydrophthalazin-1-yl-trifluoromethanesulfonate

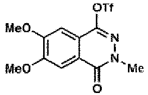

¹H NMR (600 MHz, DMSO) δ 7.67 (s, 1H), 7.13 (s, 1H), 3.99 (d, *J* = 7.4 Hz, 6H), 3.68 (s, 3H).

### Preparation Example 1-2: Preparation of Reactant 6

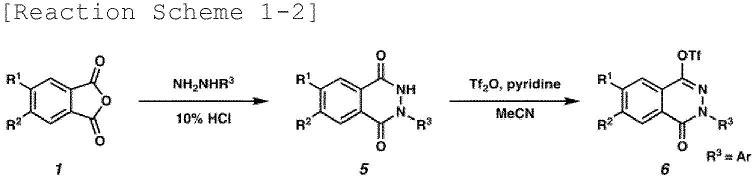

**Step 1: 1** (1.0 eq.) and arylhydrazine (3.0 eq.) were refluxed in the presence of a 10% HCl aqueous solution in a round bottom flask for 10 hours. The reaction product was cooled to room temperature, a 1M aqueous Na₂CO₃ solution was added to the residue against to remove solids, and the residue was filtered to obtain a solid **5** (65% yield).

### Reactant 5-1: 2-phenyl-2,3-dihydrophthalazine-1,4-dione

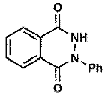

¹H NMR (400 MHz, DMSO) δ 11.83 (s, br, 1H), 8.30 (dd, *J* = 7.5, 1.6 Hz, 1H), 8.06 - 7.89 (m, 3H), 7.66 - 7.61 (m, 2H), 7.49 (dd, *J* = 8.6, 7.1 Hz, 2H), 7.40 - 7.34 (m, 1H).

**Step 2:** Reactant **5** (1.0 eq.) prepared in Step 1 above and anhydrous pyridine (5.0 eq.) were dissolved in anhydrous acetonitrile (0.61 M) under a nitrogen atmosphere in a round bottom flask and the resulting solution was cooled to 0°C, followed by stirring for 10 minutes. Triflic anhydride (1.05 eq.) was added thereto at 0°C and then the resulting mixture was warmed to room temperature and stirred for 6 hours. The mixture was poured into ethyl acetate, the result was washed with a 5% aqueous hydrochloric acid solution, and the aqueous layer was extracted with ethyl acetate. The organic layer was dried over anhydrous sodium sulfate and concentrated under reduced pressure, and the compound was recrystallized using ethyl acetate to obtain **6** (77% yield).

### Reactant 6-1: 4-oxo-3-phenyl-3,4-dihydrophthalazin-1-yl-trifluoromethanesulfonate

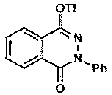

¹H NMR (600 MHz, CDCl₃) δ 8.55 (d, *J* = 7.7 Hz, 1H), 8.02 - 7.93 (m, 2H), 7.91 (d, *J* = 7.8 Hz, 1H), 7.69 (d, *J* = 7.9 Hz, 2H), 7.50 (t, *J* = 7.8 Hz, 2H), 7.40 (t, *J* = 7.5 Hz, 1H) .

### Preparation Example 1-3

A preparation process was performed in accordance with the following Reaction Scheme 1-3 using **4** prepared in Preparation Example 1-1 or **6** prepared in Preparation Example 1-2 as Reactant **7.**

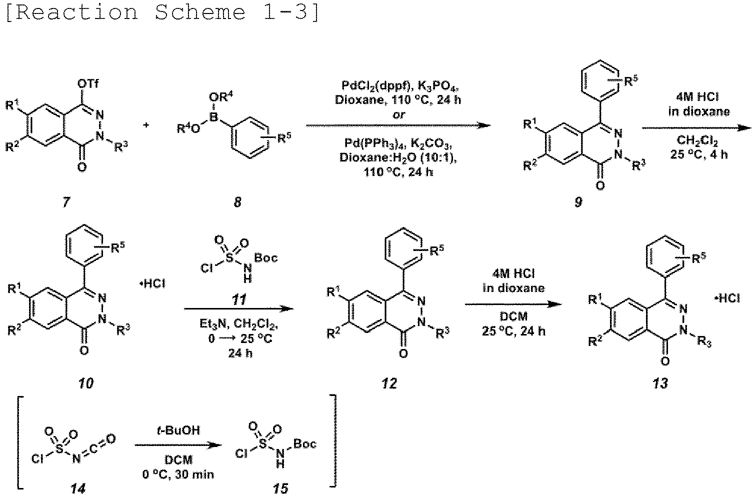

**Step 1 (Representative Synthesis Method A):** In Reaction Scheme 1-3, **7** (1.0 eq.) and [1,1'-bis(diphenylphosphino)ferrocene]palladium(II) dichloride (0.1 eq.), **8** (1.05 eq.) and potassium triphosphate (1.5 eq.) were dissolved in 1,4-dioxane (0.3 M) in a round bottom flask and refluxed under a nitrogen atmosphere for 4 hours. After the reaction was completed, the mixture was poured into water, followed by extraction with ethyl acetate. The organic layer was dried over anhydrous magnesium sulfate and concentrated under reduced pressure. The obtained residue was purified by column chromatography (EtOAc : Hexane = 1: 1) to obtain **9** (40-80% yield).

**Step 1 (Representative Synthesis Method B):** in Reaction Scheme 1-3, 7 (1.0 eq.), tetrakis (triphenylphosphine) palladium (0) (0.06 eq.), **8** (1.25 eq.) and potassium carbonate (3.75 eq.) were dissolved in 1,4-dioxane (0.09 M) and water (0.9 M) in a round bottom flask and refluxed under a nitrogen atmosphere for 4 hours. After the reaction was complete, the mixture was poured into water, followed by extraction with ethyl acetate. The organic layer was dried over anhydrous magnesium sulfate and concentrated under reduced pressure. The obtained residue was purified by column chromatography (EtOAc : Hexane = 1: 1) to obtain **9** (40-80% yield).

Examples of the present invention prepared according to Preparation Example 1 as described above are as follows.

### [Example 1]: 4-phenylphthalazin-1(2H)-one

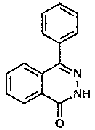

¹H NMR (400 MHz, DMSO): δ 12.87 (s, 1H), 8.39-8.32 (m, 1H), 7.91 (td, *J* = 6.0, 5.2, 3.2 Hz, 2H), 7.71-7.67 (m, 1H), 7.62-7.54 (m, 5H).

### [Example 2]: tert -butyl (3-(4-oxo-3,4-dihydrophthalazin-1-yl)phenyl)carbamate

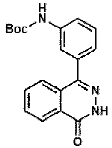

¹H NMR (400 MHz, DMSO) δ 12.83 (s, 1H), 9.55 (s, 1H), 8.99 - 8.16 (m, 1H), 7.94 - 7.86 (m, 2H), 7.71 (d, *J* = 9.2 Hz, 2H), 7.58 (d, *J* = 7.2 Hz, 1H), 7.42 (t, *J* = 7.9 Hz, 1H), 7.17 (d, *J* = 7.7 Hz, 1H), 1.47 (s, 9H) .

### [Example 3]: tert-butyl (3(3-methyl-4-oxo-3,4-dihydrophthalazin-1-yl)phenyl)carbamate

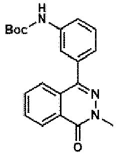

¹H NMR (400 MHz, DMSO) δ 9.55 (s, 1H), 8.61 - 8.20 (m, 1H), 8.00 - 7.82 (m, 2H), 7.73 (s, 1H), 7.69 (dd, *J* = 6.1, 3.0 Hz, 1H), 7.58 (d, *J* = 8.4 Hz, 1H), 7.43 (t, *J* = 7.9 Hz, 1H), 7.17 (dt, *J* = 7.6, 1.3 Hz, 1H), 3.78 (s, 3H), 1.47 (s, 9H).

### [Example 4]: tert-butyl (3(3-benzyl-4-oxo-3,4-dihydrophthalazin-1-yl)phenyl)carbamate

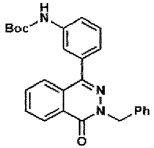

¹H NMR (400 MHz, DMSO) δ 9.55 (s, 1H), 8.45 - 8.26 (m, 1H), 7.99 - 7.88 (m, 2H), 7.79 - 7.67 (m, 2H), 7.60 (dd, 1H), 7.48 - 7.21 (m, 7H), 7.18 (d, *J* = 7.3 Hz, 1H), 5.39 (s, 2H), 1.47 (s, 9H).

### [Example 5]: tert-butyl(3-(4-oxo-3,4-dihydrophthalazin-1-yl)carbamate

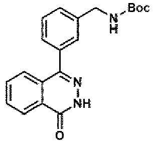

¹H NMR (400 MHz, DMSO) δ 12.85 (s, 1H), 8.38 - 8.31 (m, 1H), 7.93 - 7.85 (m, 2H), 7.72 - 7.67 (m, 1H), 7.53 - 7.43 (m, 4H), 7.40 (d, J = 7.5 Hz, 1H), 4.22 (d, J = 6.2 Hz, 2H), 1.38 (s, 9H).

### [Example 6]: tert-butyl 3-(3-methyl-4-oxo-3,4-dihydrophthalazin-1-yl)benzyl carbamate

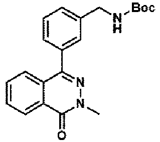

¹H NMR (400 MHz, MeOD) δ 8.50 - 8.44 (m, 1H), 7.94 - 7.84 (m, 2H), 7.81 (d, *J* = 7.0 Hz, 1H), 7.58 - 7.45 (m, 4H), 4.35 (s, 2H), 3.90 (s, 3H), 1.46 (s, 9H).

### [Example 7]: tert-butyl (3-(3-benzyl-4-oxo-3,4-dihydrophthalazin-1-yl)benzyl)carbamate

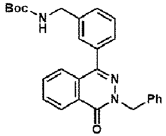

¹H NMR (400 MHz, CDCl3 δ 8.53 (d, *J* = 7.1 Hz, 1H), 7.90 - 7.61 (m, 3H), 7.58 - 7.37 (m, 7H), 7.37 - 7.28 (m, 3H), 5.47 (s, 2H), 4.42 (d, *J* = 5.6 Hz, 2H), 1.46 (s, 9H).

### [Example 8]: tert-butyl (4-(4-oxo-3,4-dihydrophthalazin-1-yl)benzyl)carbamate

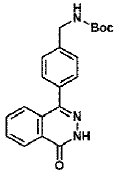

¹H NMR (400 MHz, MeOD) δ 8.44 (dd, *J* = 6.3, 2.9 Hz, 1H), 7.95 - 7.84 (m, 2H), 7.80 (d, *J* = 3.3 Hz, 1H), 7.70 (d, *J* = 7.3 Hz, 1H), 7.47 (d, *J* = 8.1 Hz, 2H), 7.26 (d, *J* = 7.7 Hz, 1H), 4.35 (s, 2H), 1.48 (s, 9H).

### [Example 9]: tert- butyl (4-3-methyl-4-oxo-3,4-dihydrophthalazin-1-yl)benzyl)carbamate

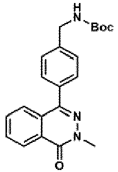

¹H NMR (400 MHz, DMSO) δ 8.40 - 8.33 (m, 1H), 7.94 - 7.86 (m, 2H), 7.69 (s, 1H), 7.55 (d, *J* = 7.9 Hz, 2H), 7.49 (s, 1H), 7.41 (d, *J* = 7.8 Hz, 2H), 4.23 (d, *J* = 6.2 Hz, 2H), 3.78 (s, 3H), 1.42 (s, 9H).

### [Example 10]: tert-butyl (4-(3-ethyl-4-oxo-3,4-dihydrophthalazin-1-yl)benzyl)carbamate

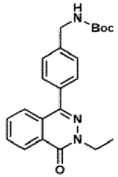

¹H NMR (400 MHz, MeOD) δ 8.50 - 8.40 (m, 1H), 7.91 - 7.85 (m, 2H), 7.82 - 7.77 (m, 1H), 7.58 (d, *J* = 7.9 Hz, 2H), 7.48 (d, *J* = 7.9 Hz, 2H), 4.37 - 4.32 (m, 4H), 1.48 (s, 9H), 1.44 (t, *J* = 7.2 Hz, 3H).

### [Example 11]: tert-butyl 4-(3-isopropyl-4-oxo-3,4-dihydrophthalazin-1-yl)benzyl carbamate

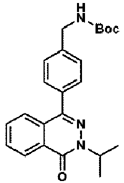

¹H NMR (400 MHz, CDCl₃) δ 10.53 - 10.43 (m, 1H), 9.79 - 9.65 (m, 3H), 9.55 (d, *J =* 8.2 Hz, 2H), 9.42 (d, *J* = 7.9 Hz, 2H), 7.44 (dt, *J* = 13.3, 6.8 Hz, 1H), 6.39 (d, *J* = 6.1 Hz, 2H), 3.45 (s, 9H), 3.40 (d, *J* = 6.6 Hz, 6H).

### [Example 12]: tert-butyl (4-(4-oxo-3-phenyl-3,4-dihydrophthalazin-1-yl)benzyl)carbamate

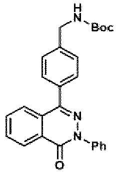

¹H NMR (400 MHz, MeOD) δ 8.53 (dd, *J* = 6.1, 3.3 Hz, 1H), 7.93 (dt, *J* = 7.2, 3.6 Hz, 2H), 7.85 (dd, *J* = 6.2, 3.4 Hz, 1H), 7.71 - 7.61 (m, 4H), 7.49 (ddd, *J* = 22.9, 15.2, 7.4 Hz, 5H), 4.34 (s, 2H), 1.47 (s, 9H).

### [Example 13]: tert-butyl (4-(3-benzyl-4-oxo-3,4-dihydrophthalazin-1-yl)benzyl)carbamate

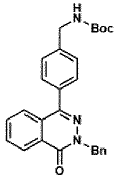

¹H NMR (400 MHz, MeOD) δ 8.49 - 8.42 (m, 1H), 7.91 - 7.84 (m, 2H), 7.81 (d, *J* = 6.0 Hz, 1H), 7.56 (d, *J* = 7.9 Hz, 2H), 7.49 - 7.40 (m, 4H), 7.36 - 7.24 (m, 3H), 5.46 (s, 2H), 4.34 (s, 2H), 1.48 (s, 9H).

### [Example 14]: tert-butyl (4-(3-(4-fluorobenzyl)-4-oxo-3,4-dihydrophthalazin-1-yl)benzyl)carbamate

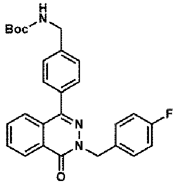

¹H NMR (400 MHz, DMSO) δ 8.38 (dd, *J* = 5.9, 3.2 Hz, 1H), 7.91 (dd, *J* = 6.0, 3.3 Hz, 2H), 7.75 - 7.67 (m, 1H), 7.55 (d, *J* = 8.1 Hz, 2H), 7.49 (s, 1H), 7.42 (t, *J* = 9.3 Hz, 5H), 7.16 (t, *J* = 8.9 Hz, 2H), 5.36 (s, 2H), 1.41 (s, 9H).

### [Example 15]: tert-butyl (4-(7-methoxy-4-oxo-3,4-dihydrophthalazin-1-yl)benzyl)carbamate

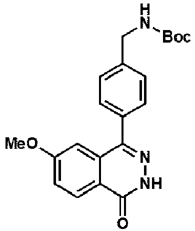

¹H NMR (400 MHz, DMSO) : δ 12.70 (s, 1H), 8.27 (d, *J* = 8.8 Hz, 1H), 7.58 (d, *J* = 8.0 Hz, 2H), 7.52-7.44 (m, 2H), 7.40 (d, *J =* 7.9 Hz, 2H), 7.03 (d, *J* = 2.5 Hz, 1H), 4.23 (d, *J* = 6.1 Hz, 2H), 3.82 (s, 3H), 1.41 (s, 9H).

### [Example 16]: tert-butyl 4-(7-methoxy-3-methyl-4-oxo-3,4-dihydrophthalazin-1-yl)benzyl carbamate

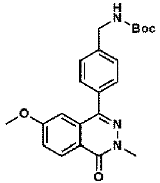

¹H NMR (400 MHz, MeOD) δ 8.36 (d, *J* = 8.9 Hz, 1H) , 7.58 (d, *J =* 8.1 Hz, 2H), 7.50 - 7.42 (m, 3H), 7.10 (d, *J* = 2.5 Hz, 1H), 4.35 (s, 2H), 3.86 (s, 3H), 3.84 (s, 3H), 1.48 (s, 9H) .

### [Example 17]: tert-butyl 4-(6-methoxy-4-oxo-3,4-dihydrophthalazin-1-yl)benzyl carbamate

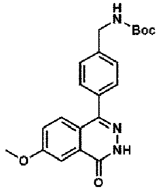

¹H NMR (400 MHz, MeOD) δ 7.84 (d, *J* = 2.8 Hz, 1H), 7.72 (d, *J =* 9.0 Hz, 1H), 7.59 - 7.50 (m, 2H), 7.50 - 7.36 (m, 3H), 4.34 (s, 2H), 3.99 (s, 3H), 1.48 (s, 9H).

### [Example 18]: tert-butyl (4-(6-methoxy-3-methyl-4-oxo-3,4-dihydrophthalazin-1-yl)benzyl)carbamate

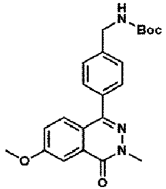

¹H NMR (400 MHz, DMSO) δ 7.74 (d, *J* = 2.8 Hz, 1H), 7.63 (d, *J =* 9.0 Hz, 1H), 7.55 - 7.43 (m, 4H), 7.40 (d, *J* = 8.2 Hz, 2H), 4.23 (d, *J* = 6.1 Hz, 2H), 3.96 (s, 3H), 3.77 (s, 3H), 1.41 (s, 9H).

### [Example 19]: tert-butyl (4-(6-methoxy-4-oxo-3,4-dihydrophthalazin-1-yl)benzyl)carbamate

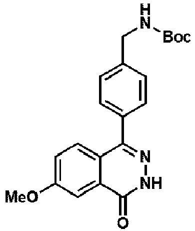

¹H NMR (400 MHz, CDCl₃): δ 9.92 (s, 1H), 7.88 (d, *J* = 2.8 Hz, 1H), 7.67 (d, *J* = 9.0 Hz, 1H), 7.54 (d, *J* = 8.1 Hz, 2H), 7.44 (d, *J* = 7.9 Hz, 2H), 7.33 (dd, *J =* 8.9, 2.8 Hz, 1H), 4.93 (s, 1H), 4.42 (d, *J* = 5.9 Hz, 2H), 3.99 (s, 3H), 1.49 (s, 9H).

### [Example 20]: tert-butyl (4-(6,7-dimethoxy-3-methyl-4-oxo-3,4-dihydrophthalazin-1-yl)benzyl)carbamate

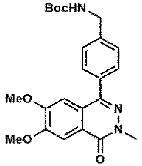

¹H NMR (400 MHz, MeOD) δ 7.82 (s, 1H), 7.59 (d, *J* = 7.9 Hz, 2H), 7.48 (d, *J* = 7.9 Hz, 2H), 7.11 (s, 1H), 4.35 (s, 2H), 4.03 (s, 3H), 3.88 (s, 3H), 3.83 (s, 3H), 1.48 (s, 9H).

### [Example 21]: tert-butyl (1-(4-(4-oxo-3,4-dihydrophthalazin-1-yl)phenyl)ethyl)carbamate

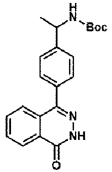

¹H NMR (400 MHz, DMSO) δ 12.83 (s, 1H), 8.33 (s, 1H), 8.08 - 7.79 (m, 2H), 7.69 (s, 1H), 7.54 (d, *J* = 8.2 Hz, 2H), 7.46 (d, *J* = 8.0 Hz, 2H), 1.39 (s, 9H), 1.23 (s, 3H), 0.85 (m, 1H).

### [Example 22]: tert-butyl (R)- (1-(4-(4-oxo-3,4-dihydrophthalazin-1-yl)phenyl)ethyl)carbamate

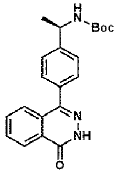

¹H NMR (400 MHz, MeOD) δ 8.44 (dd, *J* = 6.3, 3.1 Hz, 1H), 7.93 - 7.84 (m, 2H), 7.84 - 7.77 (m, 2H), 7.56 (d, *J* = 8.2 Hz, 2H), 7.50 (d, *J* = 8.1 Hz, 2H), 1.47 (d, *J* = 7.1 Hz, 3H), 1.45 (s, 9H).

### [Example 23]: tert-butyl (2-(4-(4-oxo-3,4-dihydrophthalazin-1-yl)phenyl)propan-2-yl)carbamate

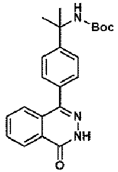

¹H NMR (400 MHz, MeOD) δ 8.44 (dd, *J* = 6.3, 3.2 Hz, 1H), 7.89 (dd, *J* = 5.9, 3.4 Hz, 2H), 7.86 - 7.80 (m, 1H), 7.57 (q, *J* = 8.2 Hz, 4H), 1.65 (s, 6H), 1.42 (s, 9H).

### [Example 24]: tert-butyl 4-(4-oxo-3,4-dihydrophthalazin-1-yl)-2-(trifluoromethyl)benzyl carbamate

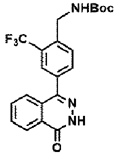

¹H NMR (DMSO, 600 MHz) δ 12.93 (s, 1H), 8.37-8.34 (m, 1H), 7.94-7.89 (m, 4H), 7.68-7.62 (m, 3H), 4.42 (d, *J* = 6 Hz, 2H), 1.43 (s, 9H).

### [Example 25]: tert-butyl methyl(4-(4-oxo-3,4-dihydrophthalazin-1-yl)benzyl)carbamate

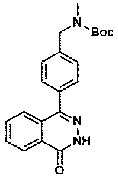

¹H NMR (400 MHz, CDCl₃) δ 10.27 (s, 1H), 8.56 - 8.50 (m, 1H), 7.85 - 7.74 (m, 3H), 7.56 (d, *J* = 7.8 Hz, 2H), 7.39 (d, *J* = 7.3 Hz, 2H), 4.53 (s, 2H), 2.88 (d, *J* = 14.3 Hz, 3H), 1.51 (s, 9H)

### [Example 26]: tert-butyl 6-(4-oxo-3,4-dihydrophthalazin-1-yl)-3,4-dihydroisoquinoline-2(1H)-carboxylate

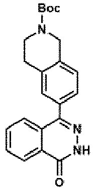

¹H NMR (400 MHz, CDCl₃) δ 9.94 (s, 1H), 8.59 - 8.46 (m, 1H), 7.90 - 7.69 (m, 3H), 7.44 - 7.34 (m, 2H), 7.28 (s, 1H), 4.67 (s, 2H), 3.71 (t, J = 5.8 Hz, 2H), 2.92 (t, J = 5.9 Hz, 2H), 1.52 (s, 9H).

### [Example 27]: tert-butyl-6-(3-methyl-4-oxo-3,4-dihydrophthalazin-1-yl)-3,4-dihydroisoquinoline-2(1H)-carboxylate

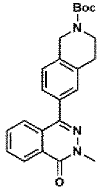

¹H NMR (400 MHz, DMSO) δ 8.42 - 8.33 (m, 1H), 7.90 (d, *J* = 9.2 Hz, 2H), 7.72 (s, 1H), 7.38 (d, *J* = 12.1 Hz, 3H), 4.60 (s, 2H), 3.78 (s, 3H), 3.61 (t, 2H), 2.87 (t, 2H), 1.45 (s, 9H).

### [Example 28]: tert-butyl 6-(6,7-dimethoxy-4-oxo-3,4-dihydrophthalazin-1-yl)-3,4-dihydroisoquinoline-2(1H)-carboxylate

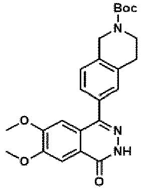

¹H NMR (400 MHz, DMSO) δ 12.69 (s, 1H), 7.70 (s, 1H), 7.48 - 7.40 (m, 2H), 7.34 (d, *J =* 7.9 Hz, 1H), 7.09 (s, 1H), 4.60 (s, 2H), 3.97 (s, 3H), 3.79 (s, 3H), 3.61 (t, *J* = 6.0 Hz, 2H), 2.88 (t, *J* = 5.9 Hz, 2H), 1.45 (s, 9H).

### [Example 29]: tert-butyl-6-(6,7-dimethoxy-3-methyl-4-oxo-3,4-dihydrophthalazin-1-yl)-3,4-dihydroisoquinoline-2(1H)-carboxylate

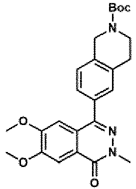

¹H NMR (400 MHz, DMSO) δ 7.71 (s, 1H), 7.45 (d, *J* = 9.5 Hz, 2H), 7.36 (s, 1H), 7.07 (s, 1H), 4.60 (s, 2H), 3.97 (s, 3H), 3.80 (s, 3H), 3.76 (s, 3H), 3.61 (t, 2H), 2.88 (t, 2H), 1.45 (s, 9H).

**Step 2:** In Reaction Scheme 1-3, **9** (1.0 eq.) was dissolved in dichloromethane (0.04 M) in a round bottom flask. Then, a hydrochloride solution (4.0 M in 1,4-dioxane, 0.2 M) was added thereto, followed by stirring at room temperature for 4 hours. After the reaction was complete, the reaction product was concentrated under reduced pressure, and the resulting residue was washed with diethyl ether, filtered and dried to obtain **10** (50-90% yield).

### [Example 30]: 4-(3-aminophenyl)phthalazin-1(2H)-one hydrochloride

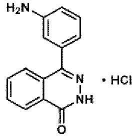

¹H NMR (400 MHz, DMSO) δ 12.80 (s, 1H), 8.33 (m, *J* = 7.2 Hz, 1H), 7.90 (m, *J* = 5.7 Hz, 2H), 7.73 (d, *J* = 7.2 Hz, 1H), 7.31 (m, 1H), 6.91 (m, 3H).

### [Example 31]: 4-(3-aminophenyl)-2-methylphthalazin-1(2H)-one hydrochloride

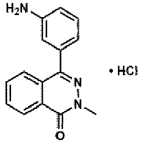

¹H NMR (400 MHz, DMSO) δ 8.41 - 8.31 (m, 1H), 7.98 - 7.80 (m, 2H), 7.79 - 7.68 (m, 1H), 7.37 (s, 1H), 7.06 (s, 3H), 3.78 (s, 3H).

### [Example 32]: 4-(3-aminophenyl)-2-benzylphthalazin-1(2H)-one hydrochloride

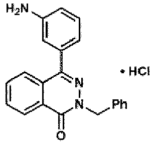

¹H NMR (400 MHz, DMSO) δ 8.42 - 8.35 (m, 1H), 8.00 - 7.88 (m, 2H), 7.84 - 7.72 (m, 1H), 7.40 - 7.18 (m, 6H), 6.92 (s, 3H), 5.38 (s, 2H).

### [Example 33]: 4-(3-(aminomethyl)phenyl)phthalazin-1(2H)-one hydrochloride

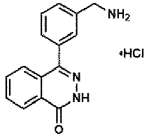

¹H NMR (400 MHz, DMSO) δ 12.92 (s, 1H), 8.45 - 8.31 (m, 4H), 7.96 - 7.86 (m, 2H), 7.79 - 7.69 (m, 2H), 7.69 - 7.58 (m, 3H), 4.14 (q, J = 5.8 Hz, 2H).

### [Example 34]: 4-(3-(aminomethyl)phenyl)-2-methylphthalazin-1-(2H)-one hydrochloride

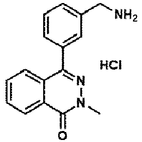

¹H NMR (400 MHz, MeOD) δ 8.52 - 8.46 (m, 1H), 7.97 - 7.86 (m, 2H), 7.80 (d, *J* = 7.1 Hz, 1H), 7.75 - 7.62 (m, 4H), 4.24 (s, 2H), 3.91 (s, 3H).

### [Example 35]: 4-(3-(aminomethyl)phenyl)-2-benzylphthalazin-1(2H)-one hydrochloride

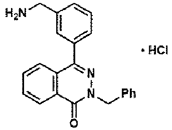

¹H NMR (400 MHz, MeOD) δ 8.51 - 8.38 (m, 1H), 7.94 - 7.84 (m, 2H), 7.83 - 7.74 (m, 1H), 7.73 - 7.59 (m, 4H), 7.42 (d, *J* = 6.8 Hz, 2H), 7.36 - 7.19 (m, 3H), 5.47 (s, 2H), 4.24 (s, 2H).

### [Example 36]: 4-(4-(aminomethyl)phenyl)phthalazin-1(2H)-one hydrochloride

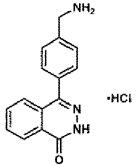

¹H NMR (400 MHz, DMSO) δ 12.91 (s, 1H), 8.42-8.20 (m, 4H), 7.98-7.90 (m, 2H), 7.71-7.62 (m, 5H), 4.20-4.12 (m, 2H).

### [Example 37]: 4-(4-aminomethyl)phenyl)-2-methylphthalazin-1(2H)-one hydrochloride

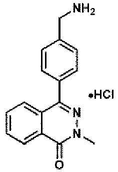

¹H NMR (400 MHz, DMSO) δ 8.39 (dd, *J* = 6.1, 3.3 Hz, 1H), 8.23 (s, 3H), 7.92 (dt, *J* = 7.2, 3.6 Hz, 2H), 7.66 (qd, *J* = 7.2, 6.1, 3.0 Hz, 5H), 4.15 (s, 2H), 3.78 (d, *J* = 3.1 Hz, 3H).

### [Example 38]: 4-(4-(aminomethyl)phenyl)-2-ethylphthalazin-1(2H)-one hydrochloride

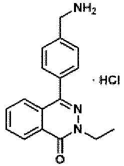

¹H NMR (400 MHz, MeOD) δ 8.50 - 8.45 (m, 1H), 7.94 - 7.85 (m, 2H), 7.78 - 7.70 (m, 3H), 7.65 (d, *J* = 7.9 Hz, 2H), 4.34 (q, *J =* 7.2 Hz, 2H), 4.24 (s, 2H), 1.44 (t, *J =* 7.2 Hz, 3H).

### [Example 39]: 4-(4-(aminomethyl)phenyl)-2-isopropylphthalazin-1-(2H)-one hydrochloride

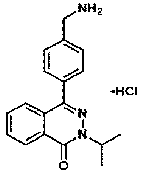

¹H NMR (400 MHz, MeOD) δ 8.40 - 8.34 (m, 1H), 7.83 - 7.74 (m, 2H), 7.71 - 7.67 (m, 1H), 7.64 (d, *J* = 8.2 Hz, 2H), 7.56 (d, *J* = 8.2 Hz, 2H), 5.34 (p, *J* = 6.6 Hz, 1H), 4.15 (s, 2H), 1.34 (d, *J* = 6.6 Hz, 6H).

### [Example 40]: 4-(4-aminomethyl)phenyl)-2-phenylphthalazin-1(2H)-one hydrochloride

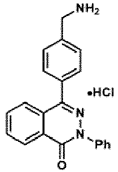

¹H NMR (400 MHz, MeOD) δ 8.59 - 8.51 (m, 1H), 7.94 (td, *J* = 7.1, 6.3, 3.4 Hz, 2H), 7.84 - 7.79 (m, 1H), 7.77 (d, *J* = 8.0 Hz, 2H), 7.70 - 7.63 (m, 4H), 7.53 (dd, *J* = 8.6, 6.9 Hz, 2H), 7.44 (t, *J* = 7.3 Hz, 1H), 4.23 (s, 2H).

### [Example 41]: 4-(4-aminomethyl)phenyl)-2-benzylphthalazin-1(2H)-one hydrochloride

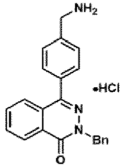

¹H NMR (400 MHz, MeOD) δ 8.52 - 8.44 (m, 1H), 7.93 - 7.85 (m, 2H), 7.78 - 7.73 (m, 1H), 7.70 (d, *J* = 8.3 Hz, 2H), 7.63 (d, *J* = 8.1 Hz, 2H), 7.44 - 7.39 (m, 2H), 7.35 - 7.25 (m, 3H), 5.47 (s, 2H), 4.22 (s, 2H).

### [Example 42]: 4-(4-(aminomethyl)phenyl)-2-(4-fluorobenzyl) phthalazin-1(2H)-one hydrochloride

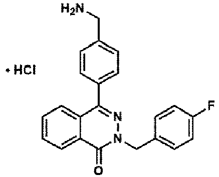

¹H NMR (400 MHz, DMSO) δ 8.43 - 8.37 (m, 1H), 8.25 - 8.00 (m, 2H), 7.94 (dd, *J* = 6.0, 3.2 Hz, 2H), 7.71 - 7.60 (m, 5H), 7.43 (dd, *J* = 8.6, 5.6 Hz, 2H), 7.17 (t, *J* = 8.9 Hz, 2H), 4.14 (s, 2H).

### [Example 43]: 4-(4-(aminomethyl)phenyl)-6-methoxyphthalazin-1(2H)-one hydrochloride

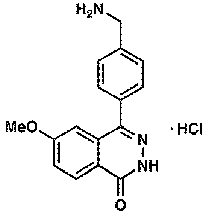

¹H NMR (400 MHz, DMSO) δ 12.74 (s, 1H), 8.29 (d, *J* = 8.8 Hz, 1H), 7.66 (d, *J* = 8.3 Hz, 2H), 7.60 (d, *J =* 8.0 Hz, 2H), 7.50 (dd, *J =* 8.9, 2.4 Hz, 1H), 7.00 (d, *J* = 2.5 Hz, 1H), 4.06 (s, 2H), 3.83 (s, 3H).

### [Example 44]: (4-(7-methoxy-3-methyl-4-oxo-3,4-dihydrophthalazin-1-yl)phenyl)methanaminium chloride

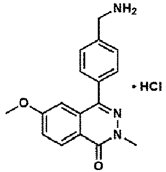

¹H NMR (400 MHz, MeOD) δ 8.39 (d, *J* = 8.9 Hz, 1H), 7.75 - 7.68 (m, 2H), 7.67 - 7.62 (m, 2H), 7.50 - 7.46 (m, 1H), 7.07 (d, *J* = 2.5 Hz, 1H), 4.21 (s, 2H), 3.86 (s, 3H), 3.85 (s, 3H).

### [Example 45]: (4-(6-methoxy-4-oxo-3,4-dihydrophthalazin-1-yl)phenyl)methanaminium chloride

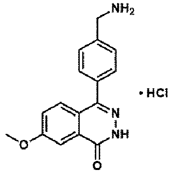

¹H NMR (400 MHz, MeOD) δ 7.87 (d, *J* = 2.8 Hz, 1H), 7.72 - 7.61 (m, 2H), 7.48 - 7.40 (m, 2H), 7.27 (d, *J* = 8.4 Hz, 1H), 6.83 (d, *J* = 8.5 Hz, 1H), 4.24 (s, 2H), 4.01 (s, 3H) .

### [Example 46]: 4-(4-(aminomethyl)phenyl)-7-methoxy-2-methylphthalazin-1(2H)-one hydrochloride

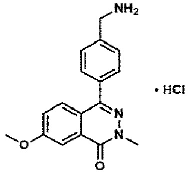

¹H NMR (400 MHz, DMSO) δ 8.19 (s, 2H), 7.76 (d, *J* = 2.7 Hz, 1H), 7.64 (s, 4H), 7.58 (d, *J* = 9.0 Hz, 1H), 7.49 (dd, *J* = 9.0, 2.7 Hz, 1H), 4.14 (s, 2H), 3.97 (s, 3H), 3.78 (s, 3H).

### [Example 47]: 4-(4-(aminomethyl)-phenyl)-7-methoxyphthalazin-1(2H)-one hydrochloride

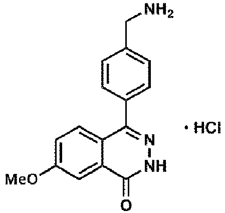

¹H NMR (400 MHz, DMSO) δ 12.82 (s, 1H), 8.34 (br s, 3H), 7.74 (d, *J* = 2.7 Hz, 1H), 7.68-7.62 (m, 4H), 7.57 (d, *J* = 9.0 Hz, 1H), 7.49 (dd, *J* = 9.0, 2.7 Hz, 1H), 4.14 (s, 2H), 3.96 (s, 3H).

### [Example 48]: 4-(4-(aminomethyl)phenyl)-6,7-dimethoxyphthalazin-1(2H)-one hydrochloride

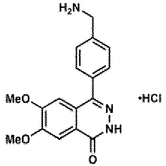

¹H NMR (400 MHz, DMSO) δ 12.76 (s, 1H), 8.27 (s, 3H), 7.77-7.68 (m, 3H), 7.63 (d, *J* = 8.1 Hz, 2H), 7.03 (s, 1H), 4.15 (s, 2H), 3.97 (s, 3H), 3.79 (s, 3H).

### [Example 49]: 4-(4-(aminomethyl)phenyl)-6,7-dimethoxy-2-methylphthalazin-1(2H)-one hydrochloride

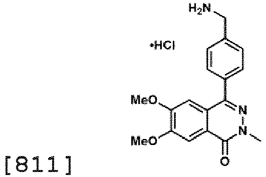

¹H NMR (400 MHz, MeOD) δ 7.86 (s, 1H), 7.79 - 7.71 (m, 2H), 7.67 (d, *J* = 8.2 Hz, 2H), 7.07 (s, 1H), 4.25 (s, 3H), 4.05 (s, 3H), 3.90 (s, 3H), 3.83 (s, 3H).

### [Example 50]: 4-(4-(1-aminoethyl)phenyl)phthalazin-1(2H)-one hydrochloride

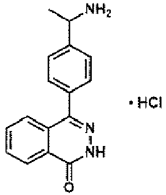

¹H NMR (400 MHz, MeOD) δ 8.52 - 8.39 (m, 1H), 7.94 - 7.84 (m, 2H), 7.78 - 7.69 (m, 3H), 7.69 - 7.60 (m, 2H), 4.60 (q, *J =* 6.9 Hz, 1H), 1.72 (d, *J =* 6.9 Hz, 3H).

### [Example 51]: (R)-4-(4-(1-aminoethyl)phenyl)phthalazin-1(2H)-one hydrochloride

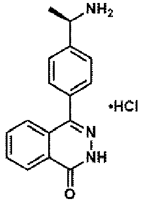

¹H NMR (400 MHz, MeOD) δ 8.49 - 8.42 (m, 1H), 7.94 - 7.85 (m, 2H), 7.77 - 7.73 (m, 1H), 7.72 (d, *J* = 8.2 Hz, 2H), 7.65 (d, *J* = 8.3 Hz, 2H), 4.59 (q, *J* = 6.8 Hz, 1H), 1.72 (d, *J* = 6.8 Hz, 3H).

### [Example 52]: 4-(4-(2-aminopropan-2-yl)phenyl)phthalazin-1(2H)-one hydrochloride

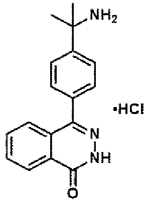

¹H NMR (400 MHz, MeOD) δ 8.48 - 8.44 (m, 1H), 7.93 - 7.88 (m, 2H), 7.77 - 7.68 (m, 5H), 1.83 (s, 6H).

### [Example 53]: 4-(4-(aminomethyl)-3-(trifluoromethyl)phenyl) phthalazin-1(2H)-one hydrochloride

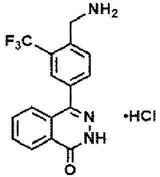

¹H NMR (DMSO, 600 MHz) δ 13.00 (s, 1H), 8.50 (br s, 3H), 8.38-8.36 (m, 1H), 8.08-8.06 (m, 1H), 8.02 (d, *J* = 2 Hz, 1H), 7.97-7.91 (m, 3H), 7.61-7.59 (m, 1H), 4.30 (s, 2H).

### [Example 54]: 4-(4-((methylamino)methyl)phenyl)phthalazin-1(2H)-one hydrochloride

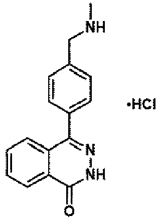

¹H NMR (400 MHz, MeOD) δ 8.49 - 8.41 (m, 1H), 7.91 (dt, *J* = 6.1, 3.5 Hz, 2H), 7.82 - 7.65 (m, 5H), 4.32 (s, 2H), 2.80 (s, 3H).

### [Example 55]: 4-(1,2,3,4-tetrahydroisoquinolin-6-yl)phthalazin-1(2H)-one hydrochloride

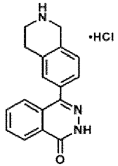

¹H NMR (400 MHz, DMSO) δ 12.88 (s, 1H), 9.57 (s, 2H), 8.38 - 8.31 (m, 1H), 7.96 - 7.84 (m, 2H), 7.70 - 7.62 (m, 1H), 7.47 (d, J = 7.0 Hz, 2H), 7.40 (d, J = 8.5 Hz, 1H), 3.42 (t, J = 6.2 Hz, 2H), 3.10 (t, J = 6.3 Hz, 2H).

### [Example 56]: 2-methyl-4-(1,2,3,4-tetrahydroisoquinolin-6-yl) phthalazin-1(2H)-one hydrochloride

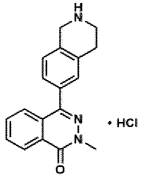

¹H NMR (400 MHz, CDCl₃) δ 10.37 (s, 1H), 8.54 (dd, *J* = 7.2, 1.9 Hz, 1H), 7.85 - 7.72 (m, 2H), 7.70 - 7.66 (m, 1H), 7.55 - 7.40 (m, 2H), 7.31 (d, *J* = 8.0 Hz, 1H), 4.49 (s, 2H), 3.92 (s, 3H), 3.57 (t, 2H), 3.32 (t, 2H).

### [Example 57]: 6,7-dimethoxy-4-(1,2,3,4-tetrahydroisoquinolin-6-yl)phthalazin-1(2H)-one hydrochloride

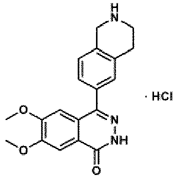

¹H NMR (400 MHz, DMSO) δ 12.75 (s, 1H), 9.14 (s, 2H), 7.71 (s, 1H), 7.58 - 7.48 (m, 2H), 7.40 (d, *J* = 7.9 Hz, 1H), 7.04 (s, 1H), 4.37 (s, 2H), 3.97 (s, 3H), 3.79 (s, 3H), 3.49 - 3.40 (t, *J* = 6.0 Hz, 2H), 3.11 (t, *J* = 6.1 Hz, 2H).

### [Example 58]: 6,7-dimethoxy-2-methyl-4-(1,2,3,4-tetrahydroisoquinolin-6-yl)phthalazin-1(2H)-one hydrochloride

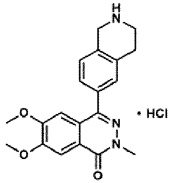

¹H NMR (400 MHz, DMSO) δ 9.05 (s, 1H), 7.74 (s, 1H), 7.61 - 7.47 (m, 2H), 7.41 (d, *J* = 7.3 Hz, 1H), 7.04 (s, 1H), 4.37 (s, 2H), 3.99 (s, 3H), 3.81 (s, 3H), 3.78 (s, 3H), 3.43 (t, 2H), 3.11 (t, 2H).

### [Example 59]: 6,7-dimethoxy-4-(1,2,3,4-tetrahydroisoquinolin-7-yl)phthalazin-1(2H)-one

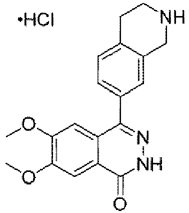

¹H NMR (400 MHz, DMSO) δ 12.77 (s, 1H), 9.32 (s, 2 H), 7.72 (s, 1 H), 7.65 (d, *J* = 1.24 Hz, 1H), 7.53 (s, 1 H), 7.41 (d, J = 8.0 Hz, 1H), 7.06 (s, 1 H), 4.38 (s, 2H), 3.98 (s, 3 H), 3.82 (s, 3 H), 3.44-3.45 (m, 2H), 3.09-3.13 (m, 2H)

**Step 3:** dichloromethane (0.2 M) was added to **10** (1.0 eq.) of Reaction Scheme 1-3 under a nitrogen atmosphere in a round bottom flask at 0°C. A solution of *tert*-butanol (1.0 eq.) in dichloromethane (0.2 M) was slowly added dropwise to the resulting solution, followed by stirring at room temperature for 30 minutes. Then, a solution of **11** (1.0 eq.) and trimethylamine (2.5 eq.) in dichloromethane (0.1 M) was slowly added dropwise at 0°C, followed by stirring at room temperature for one day. After the reaction was completed, the reaction product was concentrated under reduced pressure and dried and then the obtained residue was extracted with ethyl acetate and water. The obtained residue was purified by column chromatography (DCM : MeOH = 40: 1) to obtain **12** (20-40% yield).

### [Example 60]: tert-butyl (N-(3-(4-oxo-3,4-dihydrophthalazin-1-yl)phenyl)sulfamoyl)carbamate

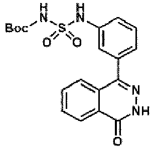

¹H NMR (400 MHz, CDCl3) δ 10.73 (s, 1H), 8.52 - 8.46 (m, 1H), 8.40 (s, 1H), 7.87 - 7.65 (m, 2H), 7.62 (s, 2H), 7.56 - 7.41 (m, 2H), 7.33 (d, *J* = 7.6 Hz, 1H), 1.44 (s, 9H).

### [Example 61]: tert-butyl (N -(3-(3-methyl-4-oxo-3,4-dihydrophthalazin-1-yl)phenyl)sulfamoyl)carbamate

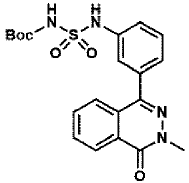

¹H NMR (400 MHz, DMSO) δ 8.37 (d, *J* = 7.4 Hz, 1H), 7.96 - 7.81 (m, 2H), 7.70 (d, *J* = 7.2 Hz, 1H), 7.51 - 7.36 (m, 1H), 7.36 (s, 1H), 7.30 (d, *J* = 9.2 Hz, 1H), 7.21 (d, *J* = 7.5 Hz, 1H), 6.69 (s, 2H), 1.29 (s, 9H).

### [Example 62]: tert-butyl (N -(3-(3-benzyl-4-oxo-3,4-dihydrophthalazin-1-yl)phenyl)sulfamoyl)carbamate

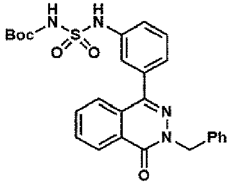

¹H NMR (400 MHz, DMSO) δ 8.43 - 8.32 (m, 1H), 8.13 (d, *J* = 6.9 Hz, 2H), 7.99 - 7.83 (m, 2H), 7.73 (d, *J* = 6.6 Hz, 1H), 7.44 (t, *J* = 7.8 Hz, 1H), 7.40 - 7.25 (m, 5H), 7.22 (d, *J* = 7.1 Hz, 1H), 6.71 (d, *J* = 6.8 Hz, 2H), 5.38 (s, 2H), 1.26 (s, 9H).

### [Example 63]: tert-butyl (N-(3-(4-oxo-3,4-dihydrophthalazin-1-yl)benzyl)sulfamoyl)carbamate

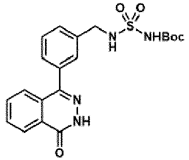

¹H NMR (400 MHz, MeOD) δ 8.49 - 8.43 (m, 1H), 7.95 - 7.87 (m, 2H), 7.87 - 7.83 (m, 1H), 7.63 (d, J = 1.6 Hz, 1H), 7.59 - 7.52 (m, 3H), 4.34 (s, 2H), 1.41 (s, 9H).

### [Example 64]: tert-butyl N-(3-(3-methyl-4-oxo-3,4-dihydrophthalazin-1-yl)benzyl)sulfamoyl carbamate

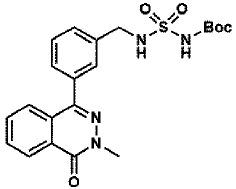

¹H NMR (400 MHz, MeOD) δ 8.46 (dd, *J* = 6.1, 3.2 Hz, 1H), 7.93 - 7.85 (m, 2H), 7.85 - 7.78 (m, 1H), 7.66 (s, 1H), 7.57 (s, 1H), 7.53 (d, *J* = 5.6 Hz, 2H), 4.26 (s, 2H), 3.90 (s, 3H), 1.33 (s, 9H).

### [Example 65]: tert-butyl (N-(3-(3-benzyl-4-oxo-3,4-dihydrophthalazin-1-yl)benzyl)sulfamoyl)carbamate

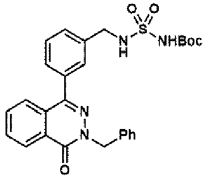

¹H NMR (400 MHz, MeOD) δ 8.44 (dd, *J =* 6.1, 3.1 Hz, 1H), 7.85 (ddq, *J =* 13.6, 6.4, 3.4 Hz, 3H), 7.62 (s, 1H), 7.59 - 7.47 (m, 3H), 7.43 (d, *J* = 7.2 Hz, 2H), 7.32 (t, *J* = 7.3 Hz, 2H), 7.25 (dd, *J* = 8.4, 6.1 Hz, 1H), 5.45 (s, 2H), 4.32 (s, 2H), 1.37 (s, 9H).

### [Example 66]: tert-butyl (N-(4-(3-methyl-4-oxo-3,4-dihydrophthalazin-1-yl)benzyl)sulfamoyl)carbamate

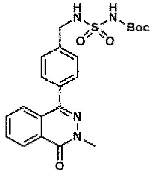

¹H NMR (400 MHz, MeOD) δ 8.51 - 8.41 (m, 1H), 7.91 - 7.82 (m, 2H), 7.82 - 7.77 (m, 1H), 7.57 (d, *J* = 0.8 Hz, 4H), 4.31 (s, 2H), 3.89 (s, 3H), 1.45 (d, *J* = 1.6 Hz, 9H).

### [Example 67]: tert-butyl (N-(4-(3-ethyl-4-oxo-3,4-dihydrophthalazin-1-yl)benzyl)sulfamoyl)carbamate

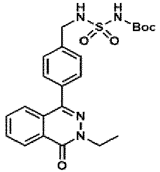

¹H NMR (400 MHz, MeOD) δ 8.49 - 8.43 (m, 1H), 7.92 - 7.84 (m, 2H), 7.83 - 7.78 (m, 1H), 7.58 (d, *J* = 2.0 Hz, 4H), 4.38 - 4.31 (m, 4H), 1.47 - 1.41 (m, 12H).

### [Example 68]: tert-butyl N-(4-(3-isopropyl-4-oxo-3,4-dihydrophthalazin-1-yl)benzyl)sulfamoyl carbamate

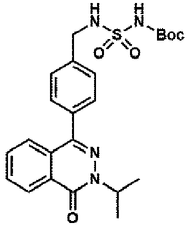

¹H NMR (400 MHz, MeOD) δ 8.52 - 8.43 (m, 1H), 7.94 - 7.85 (m, 2H), 7.86 - 7.81 (m, 1H), 7.62 (s, 4H), 5.51 - 5.39 (m, 1H), 4.35 (s, 2H), 1.46 (d, *J =* 6.6 Hz, 6H), 1.29 (s, 9H).

### [Example 69]: tert-butyl (N-(4-(4-oxo-3-phenyl-3,4-dihydrophthalazin-1-yl)benzyl)sulfamoyl)carbamate

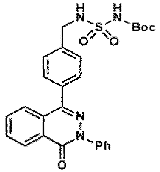

¹H NMR (400 MHz, MeOD) δ 8.57 - 8.50 (m, 1H), 7.97 - 7.90 (m, 2H), 7.89 - 7.83 (m, 1H), 7.66 (dd, *J* = 13.5, 7.7 Hz, 4H), 7.54 (dd, *J* = 23. 6, 7.9 Hz, 4H), 7.43 (t, *J* = 7.5 Hz, 1H), 4.31 (s, 2H), 1.41 (s, 9H).

### [Example 70]: tert-butyl (N-(4-3-benzyl-4-oxo-3,4-dihydrophthalazin-1-yl)benzyl)sulfamoyl)carbamate

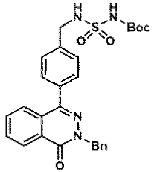

¹H NMR (400 MHz, MeOD) δ 8.55 - 8.38 (m, 1H), 7.93 - 7.84 (m, 2H), 7.84 - 7.78 (m, 1H), 7.56 (s, 4H), 7.42 (d, *J* = 6.9 Hz, 2H), 7.36 - 7.24 (m, 3H), 5.46 (s, 2H), 4.30 (s, 2H), 1.42 (s, 9H).

### [Example 71]: tert-butyl (N-(4-(3-(4-fluorobenzyl)-4-oxo-3,4-dihydrophthalazin-1-yl)benzyl)sulfamoyl)carbamate

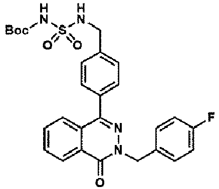

¹H NMR (400 MHz, DMSO) δ 10.88 (s, 1H), 8.39 (dd, J = 6.1, 3.1 Hz, 1H), 7.94 - 7.89 (m, 2H), 7.73 - 7.68 (m, 1H), 7.58 - 7.48 (m, 4H), 7.43 (dd, *J =* 8.6, 5.6 Hz, 2H), 7.16 (t, *J =* 8.9 Hz, 2H), 5.36 (s, 2H), 4.20 (s, 2H), 1.38 (s, 9H).

### [Example 72]: tert-butyl (4-(7-methoxy-4-oxo-3,4-dihydrophthalazin-1-yl)benzyl)sulfamoyl)carbamate

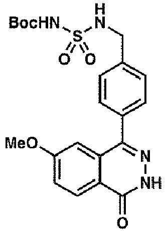

¹H NMR (400 MHz, MeOD) δ 8.36 (d, *J* = 8.9 Hz, 1H), 7.63 - 7.51 (m, 4H), 7.46 (dd, J = 8.9, 2.5 Hz, 1H), 7.13 (d, J = 2.5 Hz, 1H), 4.33 (s, 2H), 3.85 (s, 3H), 1.46 (s, 9H) .

### [Example 73]: tert-butyl N-(4-(7-methoxy-3-methyl-4-oxo-3,4-dihydrophthalazin-1-yl)benzyl)sulfamoyl carbamate

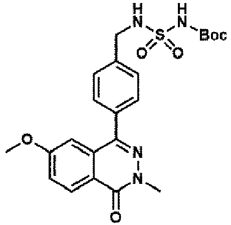

¹H NMR (400 MHz, MeOD) δ 8.37 (d, *J* = 8.9 Hz, 1H), 7.62 - 7.55 (m, 4H), 7.46 (dd, J = 9.0, 2.5 Hz, 1H), 7.13 (d, *J* = 2.5 Hz, 1H), 4.29 (s, 2H), 3.86 (s, 3H), 3.85 (s, 3H), 1.46 (s, 9H).

### [Example 74]: tert-butyl N-(4-(6-methoxy-4-oxo-3,4-dihydrophthalazin-1-yl)benzyl)sulfamoyl carbamate

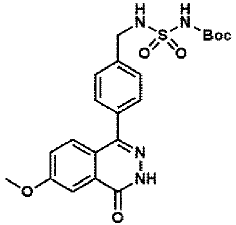

¹H NMR (400 MHz, MeOD) δ 7.86 (d, *J* = 2.7 Hz, 1H), 7.74 (d, *J* = 9.0 Hz, 1H), 7.61 - 7.53 (m, 4H), 7.45 (dd, *J* = 9.0, 2.8 Hz, 1H), 4.31 (s, 2H), 4.00 (s, 3H), 1.46 (s, 9H).

### [Example 75]: tert-butyl (N-(4-(6-methoxy-3-methyl-4-oxo-3,4-dihydrophthalazin-1-yl)benzyl)sulfamoyl)carbamate

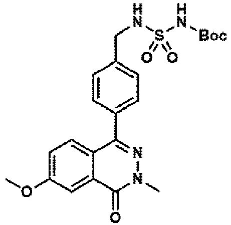

¹H NMR (400 MHz, MeOD) δ 7.86 (d, *J* = 2.7 Hz, 1H), 7.73 (d, *J* = 9.0 Hz, 1H), 7.56 (s, 4H), 7.42 (dd, *J* = 9.0, 2.7 Hz, 1H), 4.32 (s, 2H), 4.00 (s, 3H), 3.88 (s, 3H), 1.46 (s, 9H) .

### [Example 76]: tert-butyl (N-(4-(6,7-dimethoxy-4-oxo-3,4-dihydrophthalazin-1-yl)benzyl)sulfamoyl)carbamate

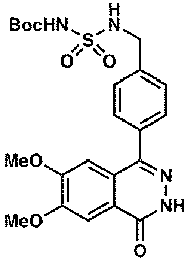

¹H NMR (400 MHz, DMSO) δ 12.70 (s, 1H), 10.89 (s, 1H), 8.25 (br m, 1H), 7.70 (s, 1H), 7.60 (d, *J* = 7.9 Hz, 2H), 7.50 (d, *J =* 7.9 Hz, 2H), 7.07 (s, 1H), 4.23 (d, *J* = 6.2 Hz, 2H), 3.97 (s, 3H), 3.79 (s, 3H), 1.40 (s, 9H).

### [Example 77]: tert-butyl(N-(4-(6,7-dimethoxy-3-methyl-4-oxo-3,4-dihydrophthalazin-1-yl)benzyl)sulfamoyl)carbamate

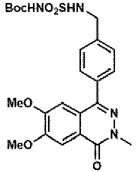

¹H NMR (400 MHz, MeOD) δ 7.79 (s, 1H), 7.64 - 7.54 (m, 4H), 7.09 (d, *J* = 3.1 Hz, 1H), 4.34 (s, 2H), 4.02 (s, 3H), 3.87 (s, 3H), 3.83 (s, 3H), 1.48 (s, 9H).

### [Example 78]: tert-butyl (N-(1-(4-(4-oxo-3,4-dihydrophthalazin-1-yl)phenyl)ethyl)sulfamoyl)carbamate

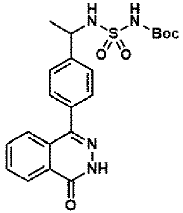

¹H NMR (400 MHz, MeOD) δ 8.54 - 8.33 (m, 1H), 7.94 - 7.86 (m, 2H), 7.85 - 7.73 (m, 1H), 7.56 (d, *J* = 8.4 Hz, 2H), 7.50 (d, *J* = 8.2 Hz, 2H), 1.47 (d, *J* = 7.1 Hz, 3H), 1.45 (s, 9H), 1.29 (m, 1H).

### [Example 79]: tert-butyl (R)-(N-(1-(4-(4-oxo-3,4-dihydrophthalazin-1-yl)phenyl)ethyl)sulfamoyl)carbamate

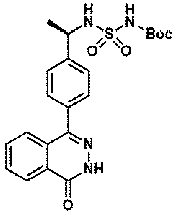

¹H NMR (400 MHz, MeOD) δ 8.49 - 8.40 (m, 1H), 7.93 - 7.85 (m, 2H), 7.84 - 7.79 (m, 1H), 7.56 (s, 4H), 4.64 (q, *J* = 6.9 Hz, 1H), 1.55 (d, *J* = 7.0 Hz, 3H), 1.40 (s, 9H).

### [Example 80]: tert-butyl (N-(2-(4-(4-oxo-3,4-dihydrophthalazin-1-yl)phenyl)propan-2-yl)sulfamoyl)carbamate

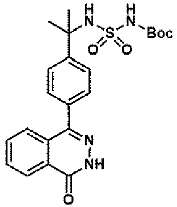

¹H NMR (400 MHz, MeOD) δ 8.53 (d, *J* = 7.7 Hz, 2H), 7.94 - 7.83 (m, 2H), 7.76 (d, *J =* 8.2 Hz, 1H), 7.58 (d, *J* = 8.1 Hz, 1H), 7.00 (d, *J =* 7.7 Hz, 2H), 1.49 (s, 6H), 1.36 (s, 9H) .

### [Example 81]: tert-butyl N-(4-(4-oxo-3,4-dihydrophthalazin-1-yl)-2-(trifluoromethyl)benzyl)sulfamoyl carbamate

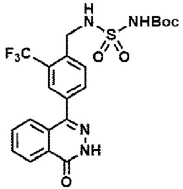

¹H NMR (DMSO, 600 MHz) δ 12.95 (s, 1H), 11.07 (s, 1H), 8.47 (s, 1H), 8.37-8.34 (m, 1H), 7.95-7.90 (m, 5H), 7.65-7.63 (m, 1H), 4.42 (d, *J* = 6 Hz, 2H), 1.43 (s, 9H).

### [Example 82]: tert-butyl (N-methyl-N-(4-(4-oxo-3,4-dihydrophthalazin-1-yl)benzyl)sulfamoyl)carbamate

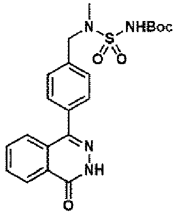

¹H NMR (400 MHz, MeOD) δ 8.51 - 8.45 (m, 1H), 7.96 - 7.90 (m, 2H), 7.87 - 7.80 (m, 1H), 7.64 (d, *J* = 8.1 Hz, 2H), 7.59 (d, *J* = 8.0 Hz, 2H), 4.57 (s, 2H), 2.90 (s, 3H), 1.55 (s, 9H).

### [Example 83]: tert-butyl ((6-(4-oxo-3,4-dihydrophthalazin-1-yl)-3,4-dihydroisoquinolin-2(1H)-yl)sulfonyl)carbamate

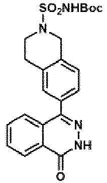

¹H NMR (400 MHz, DMSO) δ 12.83 (s, 1H), 11.15 (s, 1H), 8.40 - 8.29 (m, 1H), 7.98 - 7.82 (m, 2H), 7.72 - 7.63 (m, 1H), 7.38 (q, J = 8.0 Hz, 3H), 4.56 (s, 2H), 3.57 (t, J = 5.8 Hz, 2H), 2.96 (t, J = 5.9 Hz, 2H), 1.35 (s, 9H).

### [Example 84]: tert-butyl ((6-(3-methyl-4-oxo-3,4-dihydrophthalazin-1-yl)-3,4-dihydroisoquinolin-2(1H)-yl)sulfonyl)carbamate

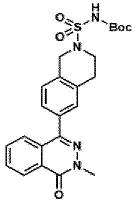

¹H NMR (400 MHz, DMSO) δ 11.17 (s, 1H), 8.63 - 8.27 (m, 1H), 8.18 - 7.81 (m, 2H), 7.81 - 7.52 (m, 1H), 7.41 (q, *J* = 8.1 Hz, 3H), 4.57 (s, 2H), 3.79 (s, 3H), 3.58 (t, *J* = 5.2 Hz, 2H), 2.98 (t, *J* = 5.2 Hz, 2H), 1.37 (s, 9H).

### [Example 85]: tert-butyl ((6-(6,7-dimethoxy-4-oxo-3,4-dihydrophthalazin-1-yl)-3,4-dihydroisoquinolin-2(1H)-yl)sulfonyl)carbamate

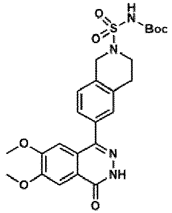

¹H NMR (400 MHz, DMSO) δ 12.68 (s, 1H), 7.69 (s, 1H), 7.50 - 7.38 (m, 2H), 7.34 (d, *J =* 7.5 Hz, 1H), 7.07 (s, 1H), 4.49 (s, 2H), 3.97 (s, 3H), 3.79 (s, 3H), 3.51 (t, *J* = 6.0 Hz, 2H), 2.95 (t, *J* = 6.1 Hz, 2H), 1.33 (s, 9H).

### [Example 86]: tert-butyl ((6-(6,7-dimethoxy-3-methyl-4-oxo-3,4-dihydrophthalazin-1-yl)-3,4-dihydroisoquinolin-2(1H)-yl)sulfonyl)carbamate

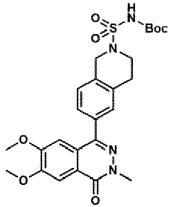

¹H NMR (400 MHz, MeOD) δ 7.75 (d, *J* = 1.6 Hz, 1H), 7.42 (d, *J =* 9.5 Hz, 2H), 7.31 (d, *J =* 7.6 Hz, 1H), 7.07 (s, 1H), 4.65 (s, 1H), 4.45 (s, 1H), 4.00 (s, 3H), 3.85 (s, 3H), 3.81 (s, 3H), 3.69 (t, *J =* 5.9 Hz, 1H), 3.53 (t, *J* = 5.9 Hz, 1H), 3.04 (t, *J =* 5.8 Hz, 2H), 1.43 (s, 9H) .

**Step 4:** In Reaction Scheme 1-3, **12** (1.0 eq.) was dissolved in dichloromethane (0.04 M) in a round bottom flask. Then, a hydrochloride solution (4.0 M in 1,4-dioxane, 0.1 M) was added thereto, followed by stirring at room temperature for 24 hours. After the reaction was complete, the reaction product was concentrated under reduced pressure and the resulting residue was washed with diethyl ether and filtered and dried to obtain **13** (20-40% yield).

### [Example 87]: N -(3-(4-oxo-3,4-dihydrophthalazin-1-yl)phenyl)sulfamide hydrochloride

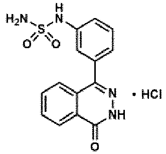

¹H NMR (400 MHz, DMSO) δ 12.85 (s, 1H), 9.71 (s, 1H), 8.50 - 8.23 (m, 1H), 7.94 - 7.83 (m, 2H), 7.71 (d, *J =* 9.1 Hz, 1H), 7.45 (t, *J* = 7.7 Hz, 1H), 7.38 (s, 1H), 7.31 (d, *J =* 8.1 Hz, 1H), 7.18 (d, *J* = 8.7 Hz, 3H).

### [Example 88]: N-(3-(3-methyl-4-oxo-3,4-dihydrophthalazin-1-yl)phenyl)sulfamide hydrochloride

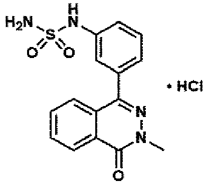

¹H NMR (400 MHz, MeOD) δ 8.48 - 8.41 (m, 1H), 7.92 - 7.78 (m, 3H), 7.52 - 7.46 (m, 2H), 7.39 (ddd, *J* = 8.3, 2.1, 1.0 Hz, 1H), 7.30 (dt, *J* = 7.9, 1.4 Hz, 1H), 3.90 (s, 3H).

### [Example 89]: N -(3-(3-benzyl-4-oxo-3,4-dihydrophthalazin-1-yl)phenyl)sulfamide hydrochloride

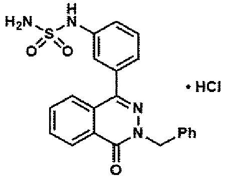

¹H NMR (400 MHz, DMSO) δ 9.72 (s, 1H), 8.43 - 8.35 (m, 1H), 7.92 (dd, *J* = 6.0, 3.2 Hz, 2H), 7.78 - 7.68 (m, 1H), 7.46 (t, *J* = 8.0 Hz, 1H), 7.41 - 7.24 (m, 7H), 7.23 - 7.11 (m, 3H), 5.39 (s, 2H).

### [Example 90]: N-(3-(3-benzyl-4-oxo-3,4-dihydrophthalazin-1-yl)benzyl)sulfamide hydrochloride

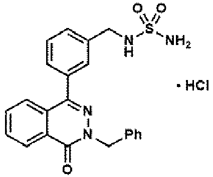

¹H NMR (400 MHz, MeOD) δ 8.49 (d, *J* = 9.1 Hz, 1H), 7.91 (dd, *J* = 7.1, 2.4 Hz, 2H), 7.86 (s, 1H), 7.69 - 7.53 (m, 4H), 7.46 (d, *J* = 6.9 Hz, 2H), 7.39 - 7.25 (m, 3H), 5.50 (s, 2H), 4.34 (s, 2H).

### [Example 91]: N -(4-(4-oxo-3,4-dihydrophthalazin-1-yl)phenyl)sulfamide

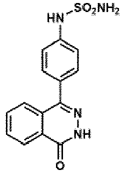

¹H NMR (400 MHz, DMSO) δ 12.64 (s, 1H), 8.31 (dd, *J* = 7.8, 1.7 Hz, 1H), 7.93 - 7.74 (m, 3H), 7.29 - 7.16 (m, 2H), 6.72 - 6.62 (m, 2H), 5.42 (s, 2H), 4.09 (t, *J* = 5.3 Hz, 1H).

### [Example 92]: N -(4-(3-methyl-4-oxo-3,4-dihydrophthalazin-1-yl)phenyl)sulfamide

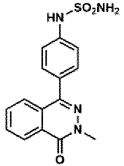

¹H NMR (400 MHz, DMSO) δ 8.41 - 8.32 (m, 1H), 7.95 - 7.85 (m, 2H), 7.74 (s, 1H), 7.50 (d, *J* = 8.3 Hz, 2H), 7.30 (d, *J* = 8.2 Hz, 2H), 7.17 (s, 2H), 3.77 (d, *J* = 6.1 Hz, 3H).

### [Example 93]: N -(4-(3-benzyl-4-oxo-3,4-dihydrophthalazin-1-yl)phenyl)sulfamide

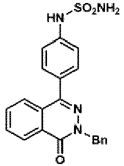

¹H NMR (400 MHz, MeOD) δ 8.46 - 8.39 (m, 1H), 7.92 - 7.83 (m, 3H), 7.57 (t, *J* = 7.3 Hz, 1H), 7.42 (t, *J* = 6.1 Hz, 3H), 7.35 - 7.18 (m, 7H), 6.85 - 6.81 (m, 2H).

### [Example 94]: N-(3-(3-methyl-4-oxo-3,4-dihydrophthalazin-1-yl)benzyl)sulfamide trifluoroacetic acid

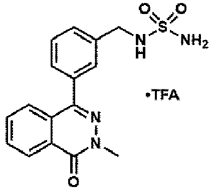

¹H NMR (400 MHz, MeOD) δ 8.49 - 8.43 (m, 1H), 7.93 - 7.84 (m, 2H), 7.84 - 7.79 (m, 1H), 7.67 (s, 1H), 7.63 - 7.57 (m, 1H), 7.57 - 7.50 (m, 2H), 4.34 (s, 2H), 3.91 (s, 3H).

### [Example 95]: N-(3-(3-oxo-3,4-dihydrophthalazin-1-yl)benzyl)sulfamide hydrochloride

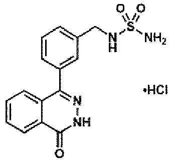

¹H NMR (400 MHz, (CD₃)₂CO) δ 8.46 - 8.37 (m, 1H), 7.94 - 7.84 (m, 2H), 7.84 - 7.76 (m, 1H), 7.73 - 7.63 (m, 1H), 7.63 - 7.46 (m, 3H), 4.38 (d, J = 2.6 Hz, 2H).

### [Example 96]: N-(4-(4-oxo-3,4-dihydrophthalazin-1-yl)benzyl) sulfamide hydrochloride

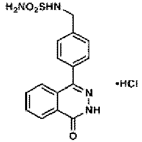

¹H NMR (400 MHz, DMSO) δ 12.84 (s, 1H), 8.43-8.29 (m, 1H), 8.01-7.84 (m, 2H), 7.71-7.66 (m, 1H), 7.59-7.50 (m, 4H), 7.18 (t, *J =* 6.8 Hz, 1H), 6.69 (s, 2H), 4.19 (d, *J =* 6.3 Hz, 2H).

### [Example 97]: N-(4-(3-methyl-4-oxo-3,4-dihydrophthalazin-1-yl)benzyl)sulfamide hydrochloride

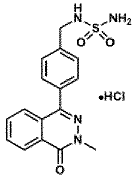

¹H NMR (400 MHz, MeOD) δ 8.51 - 8.38 (m, 1H), 7.88 (td, *J* = 7.2, 6.4, 3.5 Hz, 2H), 7.83 - 7.76 (m, 1H), 7.59 (s, 4H), 4.33 (s, 2H), 3.89 (s, 3H).

### [Example 98]: N-(4-(3-ethyl-4-oxo-3,4-dihydrophthalazin-1-yl)benzyl)sulfamide hydrochloride

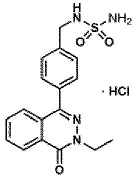

¹H NMR (400 MHz, MeOD) δ 8.49 - 8.42 (m, 1H), 7.92 - 7.84 (m, 2H), 7.84 - 7.76 (m, 1H), 7.60 (s, 4H), 4.38 - 4.30 (m, 4H), 1.44 (t, *J* = 7.1 Hz, 3H).

### [Example 99]: N-(4-(3-isopropyl-4-oxo-3,4-dihydrophthalazin-1-yl)benzyl)sulfamide trifluoroacetic acid

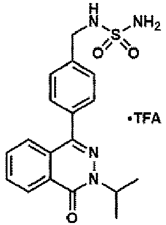

¹H NMR (400 MHz, MeOD) δ 8.48 - 8.43 (m, 1H), 7.90 - 7.85 (m, 2H), 7.85 - 7.80 (m, 1H), 7.60 (s, 4H), 5.44 (p, *J* = 6.6 Hz, 1H), 4.33 (s, 2H), 1.45 (d, *J* = 6.7 Hz, 6H).

### [Example 100]: N-(4-(4-oxo-3-phenyl-3,4-dihydrophthalazin-1-yl)benzyl)sulfamide hydrochloride

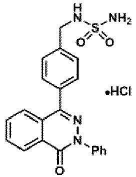

¹H NMR (400 MHz, MeOD) δ 8.57 - 8.49 (m, 1H), 7.94 (t, *J* = 4.9 Hz, 2H), 7.86 (s, 1H), 7. 66 (dd, *J* = 11.8, 7.9 Hz, 4H), 7.60 (d, *J* = 7.5 Hz, 2H), 7.53 (t, *J* = 7.6 Hz, 2H), 7.43 (t, *J* = 7.2 Hz, 1H), 4.33 (s, 2H).

### [Example 101]: N-(4-(3-benzyl-4-oxo-3,4-dihydrophthalazin-1-yl)benzyl)sulfamide hydrochloride

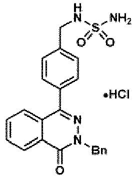

¹H NMR (400 MHz, MeOD) δ 8.54 - 8.32 (m, 1H), 7.92 - 7.84 (m, 2H), 7.83 - 7.78 (m, 1H), 7.58 (s, 4H), 7.43 (d, *J* = 7.5 Hz, 2H), 7.35 - 7.24 (m, 3H), 5.46 (s, 2H), 4.32 (s, 2H).

### [Example 102]: N-(4-(3-(4-fluorobenzyl)-4-oxo-3,4-dihydrophthalazin-1-yl)benzyl)sulfamide hydrochloride

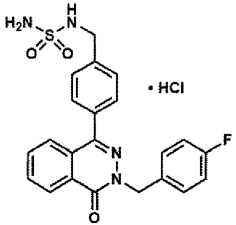

¹H NMR (400 MHz, MeOD) δ 8.48 - 8.43 (m, 1H), 7.90 - 7.86 (m, 2H), 7.83 - 7.77 (m, 1H), 7.58 (s, 4H), 7.48 (dd, *J* = 8.7, 5.5 Hz, 2H), 7.05 (t, *J* = 8.8 Hz, 2H), 5.44 (s, 2H), 4.32 (s, 2H).

### [Example 103] N-(4-(7-methoxy-4-oxo-3,4-dihydrophthalazin-1-yl)benzyl)sulfamide hydrochloride

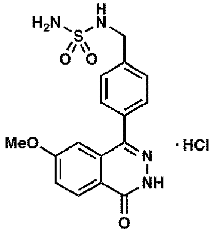

¹H NMR (400 MHz, MeOD) δ 11.70 (s, 1H), 8.34 (d, *J* = 8.8 Hz, 1H), 7.68 - 7.53 (m, 5H), 7.44 (dd, *J* = 8.8, 2.4 Hz, 1H), 7.14 (d, *J* = 2.4 Hz, 1H), 6.27 (s, 1H), 6.04 (s, 1H), 4.38 (d, *J* = 6.3 Hz, 2H), 3.88 (s, 3H).

### [Example 104]: N-(4-(6,7-dimethoxy-4-oxo-3,4-dihydrophthalazin-1-yl)benzyl)sulfamide hydrochloride

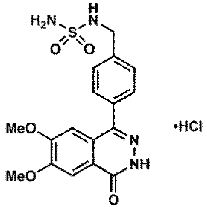

¹H NMR (400 MHz, DMSO) δ 12.84 (s, 1H), 8.43-8.29 (m, 1H), 8.01-7.84 (m, 2H), 7.71-7.66 (m, 1H), 7.59-7.50 (m, 4H), 7.18 (t, *J* = 6.8 Hz, 1H), 6.69 (s, 2H), 4.19 (d, *J* = 6.3 Hz, 2H).

### [Example 105]: N-(4-(7-methoxy-3-methyl-4-oxo-3,4-dihydrophthalazin-1-yl)benzyl)sulfamide hydrochloride

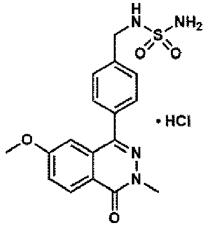

¹H NMR (400 MHz, MeOD) δ 8.37 (d, *J* = 8.9 Hz, 1H), 7.60 (s, 4H), 7.46 (dd, *J* = 8.9, 2.5 Hz, 1H), 7.11 (d, *J* = 2.5 Hz, 1H), 4.33 (s, 2H), 3.86 (s, 3H), 3.84 (s, 3H).

### [Example 106]: N-(4-(6-methoxy-4-oxo-3,4-dihydrophthalazin-1-yl)benzyl)sulfamide hydrochloride

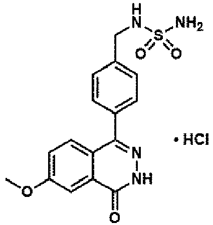

¹H NMR (400 MHz, MeOD) δ 7.86 (d, *J* = 2.8 Hz, 1H), 7.73 (d, *J* = 8.9 Hz, 1H), 7.63 - 7.50 (m, 4H), 7.45 (dd, *J* = 9.0, 2.8 Hz, 1H), 4.32 (s, 2H), 4.00 (s, 3H).

### [Example 107]: N-(4-(6-methoxy-3-methyl-4-oxo-3,4-dihydrophthalazin-1-yl)benzyl)sulfamide hydrochloride

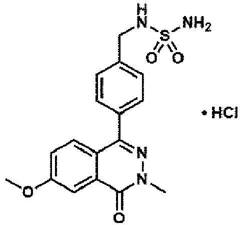

¹H NMR (400 MHz, DMSO) δ 7.74 (d, *J* = 2.7 Hz, 1H), 7.62 (d, *J* = 8.9 Hz, 1H), 7.53 (s, 4H), 7.47 (dd, *J* = 8.9, 2.8 Hz, 1H), 7.16 (t, *J* = 6.5 Hz, 1H), 6.67 (s, 2H), 4.18 (d, *J* = 6.4 Hz, 2H), 3.95 (s, 3H), 3.77 (s, 3H).

### [Example 108]: N-(4-(6,7-dimethoxy-3-methyl-4-oxo-3,4-dihydrophthalazin-1-yl)benzyl)sulfamide hydrochloride

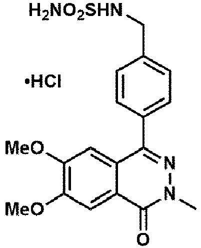

¹H NMR (400 MHz, MeOD) δ 7.82 (s, 1H), 7.60 (s, 4H), 7.10 (s, 1H), 4.32 (s, 2H), 4.02 (s, 3H), 3.87 (s, 3H), 3.82 (s, 3H).

### [Example 109]: N-(3-(6,7-dimethoxy-4-oxo-3,4-dihydrophthalazin-1-yl)-benzyl) sulfamide

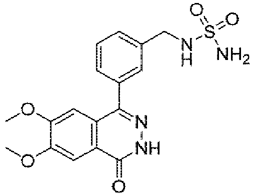

¹H NMR (400 MHz, DMSO) δ 12.74 (s, 1H), 7.71 (s, 1 H), 7.66 (s, 1H), 7.49-7.53 (m, 3 H), 7.22 (brs, 1 H), 7.10 (s, 1H), 6.68 (s, 2H), 4.19 (d, *J* = 6.08 Hz, 2 H), 3.98 (s, 3 H), 3.83 (s, 3 H)

### [Example 110]: N-(3-(6,7-dimethoxy-3-methyl-4-oxo-3,4-dihydrophthalazin-1-yl)-benzyl) sulfamide

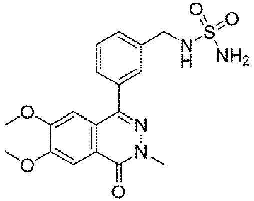

¹H NMR (400 MHz, DMSO) δ 7.73 (s, 1 H), 7.66 (s, 1H), 7.50-7.53 (m, 3 H), 7.22 (t, *J* = 6.32 Hz, 1 H), 7.07 (s, 1H), 6.67 (s, 2H), 4.20 (d, *J* = 6.32 Hz, 2 H), 3.98 (s, 3 H), 3.81 (s, 3 H), 3.78 (s, 3H)

### [Example 111]: N-(4-(6,7-dimethoxy-4-oxo-3,4-dihydrophthalazin-1-yl)-2-fluorobenzyl)sulfamide hydrochloride

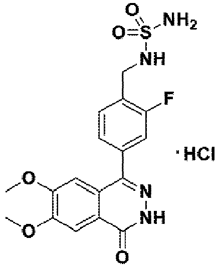

LC/MS [M+H]⁺ 409.07

### [Example 112]: N-(4-(6,7-dimethoxy-4-oxo-3,4-dihydrophthalazin-1-yl)-3-fluorobenzyl)sulfamide hydrochloride

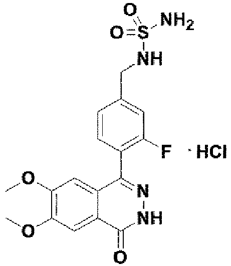

LCMS [M+H]⁺ 409.16

### [Example 113]: N-(4-(6,7-dimethoxy-4-oxo-3,4-dihydrophthalazin-1-yl)-2-fluorobenzyl)-N-methyl sulfamide hydrochloride

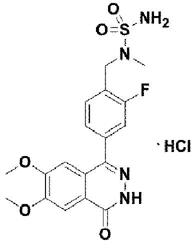

LCMS [M+H]⁺ 423.10

### [Example 114]: N-(4-(6,7-dimethoxy-4-oxo-3,4-dihydrophthalazin-1-yl)-3-fluorobenzyl)-N-methyl sulfamide hydrochloride

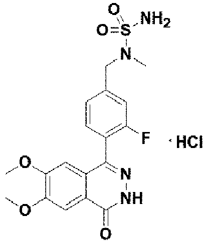

LCMS [M+H]⁺ 423.29

### [Example 115]: N-(4-(6,7-dimethoxy-4-oxo-3,4-dihydrophthalazin-1-yl)-2,3-difluorobenzyl)sulfamide hydrochloride

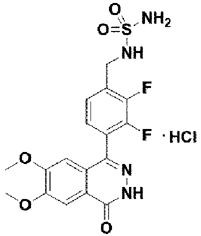

LCMS [M+H]⁺427.17

### [Example 116]: N-(4-(6,7-dimethoxy-4-oxo-3,4-dihydrophthalazin-1-yl)-3-(trifluorobenzyl methyl)benzyl) sulfamide hydrochloride

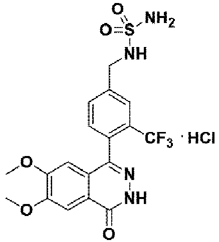

LCMS [M+H]⁺ 459.20.

### [Example 117]: N-(4-(6,7-dimethoxy-4-oxo-3,4-dihydrophthalazin-1-yl)-2,3-difluorobenzyl)-N-methyl sulfamide hydrochloride

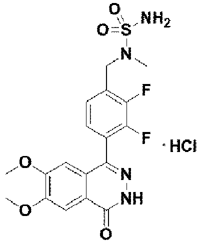

LCMS [M+H]⁺ 441.29

### [Example 118]: N-(4-(6,7-dimethoxy-4-oxo-3,4-dihydrophthalazin-1-yl)-3-(trifluorobenzyl methyl)benzyl)-N-methyl sulfamide hydrochloride

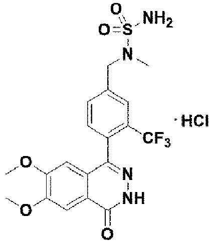

LCMS [M+H]⁺ 458.33

### [Example 119]: N-(4-(6,7-dimethoxy-4-oxo-3,4-dihydrophthalazin-1-yl)-2-(trifluorobenzyl methyl)benzyl)sulfamide hydrochloride

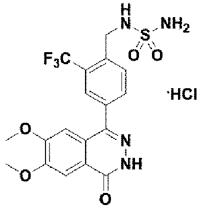

LCMS [M+H]⁺ 459.1

### [Example 120]: N-(4-(6,7-dimethoxy-4-oxo-3,4-dihydrophthalazin-1-yl)-2-(trifluorobenzyl methyl)benzyl)-N-methyl sulfamide hydrochloride

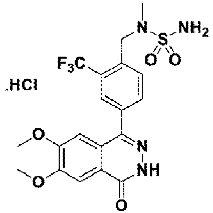

LCMS [M+H]⁺473.10

### [Example 121]: 7-(4-oxo-3,4-dihydrophthalazin-1-yl)-3,4-dihydroisoquinoline-2(1H) sulfamide hydrochloride

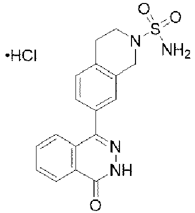

### [Example 122]: 7-(6,7-dimethoxy-4-oxo-3,4-dihydrophthalazin-1-yl)-3,4-dihydroisoquinoline-2(1H)-sulfamide

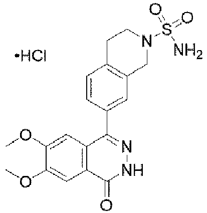

¹H NMR (400 MHz, DMSO) δ 12.71(s, 1H), 7.70 (s, 1 H), 7.45 (d, *J* = 8.60 Hz, 1H), 7.34 (d, *J* = 7.92 Hz, 1H), 7.20 (s, 1 H), 7.07 (s, 3H), 6.97 (s, 1 H), 4.31 (s, 2H), 3.98 (s, 3 H), 3.81 (s, 3 H), 3.07-3.11 (m, 2H), 3.01-3.05 (m, 2H)

### [Example 123]: N-(1-(4-(4-oxo-3,4-dihydrophthalazin-1-yl)phenyl)ethyl)sulfamide hydrochloride

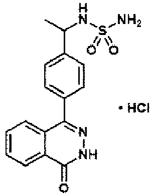

¹H NMR (400 MHz, MeOD) δ 8.47 - 8.42 (m, 1H), 7.89 (dd, *J* = 6.0, 3.4 Hz, 2H), 7.84 - 7.78 (m, 1H), 7.58 (d, *J* = 3.5 Hz, 4H), 4.66 (q, *J* = 6.8 Hz, 1H), 1.57 (d, *J* = 6.9 Hz, 3H) .

### [Example 124] : (R)-N-(1-(4-(4-oxo-3,4-dihydrophthalazin-1-yl)phenyl)ethyl)sulfamide hydrochloride

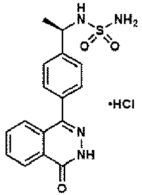

¹H NMR (400 MHz, MeOD) δ 8.44 (dq, *J* = 7.4, 3.8 Hz, 1H), 7.89 (dt, *J* = 7.2, 3.7 Hz, 2H), 7.81 (dt, *J* = 5.9, 3.6 Hz, 1H), 7.73 - 7.48 (m, 4H), 4.66 (q, *J* = 6.9 Hz, 1H), 1.57 (d, *J* = 7.0 Hz, 3H).

### [Example 125]: N-(2-(4-(4-oxo-3,4-dihydrophthalazin-1-yl)phenyl)propan-2-yl)sulfamide hydrochloride

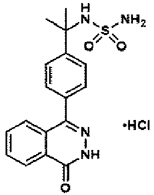

¹H NMR (400 MHz, MeOD) δ 8.47 - 8.43 (m, 1H), 7.92 - 7.87 (m, 2H), 7.82 (s, 1H), 7.61 (d, *J* = 1.3 Hz, 4H), 1.60 (s, 6H).

### [Example 126]: N-(4-(4-oxo-3,4-dihydrophthalazin-1-yl)-2-(trifluoromethyl)benzyl)sulfamide hydrochloride

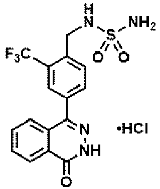

¹H NMR (DMSO, 600 MHz) δ 12.94 (s, 1H), 8.36-8.35 (m, 1H), 7.99-7.89 (m, 5H), 7.65 (d, *J* = 8 Hz, 1H), 7.34 (t, *J* = 6 Hz, 1H), 6.80 (s, 2H), 4.37 (d, *J* = 7 Hz, 2H).

### [Example 127]: N-methyl-N-(4-(4-oxo-3,4-dihydrophthalazin-1-yl)benzyl)sulfamide

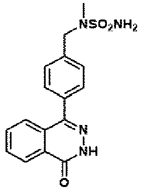

¹H NMR (400 MHz, MeOD) δ 8.50 - 8.38 (m, 1H), 7.95 - 7.85 (m, 2H), 7.84 - 7.75 (m, 1H), 7.59 (q, *J* = 7.9 Hz, 4H), 4.31 (s, 2H), 2.71 (s, 3H).

### [Example 128]: 6-(4-oxo-3,4-dihydrophthalazin-1-yl-3,4-dihydroisoquinoline-2(1H)-sulfonamide hydrochloride

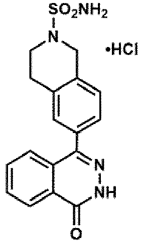

¹H NMR (400 MHz, DMSO) δ 12.83 (s, 1H), 8.37 - 8.30 (m, 1H), 7.90 (m, *J* = 6.4, 5.4, 3.5 Hz, 2H), 7.76 - 7.65 (m, 1H), 7.37 (d, *J* = 12.0 Hz, 3H), 6.96 (s, 2H), 4.30 (s, 2H), 3.00 (t, *J* = 5.8 Hz, 2H).

### [Example 129]: 6-(3-methyl-4-oxo-3,4-dihydrophthalazin-1-yl)-3,4-dihydroisoquinoline-2(1H)-sulfonamide hydrochloride

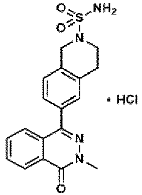

¹H NMR (400 MHz, MeOD) δ 8.49 - 8.42 (m, 1H), 7.93 - 7.84 (m, 2H), 7.83 - 7.76 (m, 1H), 7.43 (d, *J* = 5.7 Hz, 2H), 7.33 (d, *J* = 8.5 Hz, 1H), 4.42 (s, 2H), 3.89 (s, 3H), 3.57 - 3.38 (t, 2H), 3.08 (t, *J* = 5.9 Hz, 2H).

### [Example 130]: 6-(6,7-dimethoxy-4-oxo-3,4-dihydrophthalazin-1-yl)-3,4-dihydroisoquinoline-2(1H)-sulfonamide hydrochloride

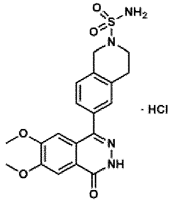

¹H NMR (400 MHz, MeOD) δ 7.83 (s, 1H), 7.44 (d, *J* = 7.1 Hz, 2H), 7.35 - 7.23 (d, *J* = 7.9 Hz, 1H), 7.14 (s, 1H), 4.41 (s, 2H), 4.03 (s, 3H), 3.84 (s, 3H), 3.50 - 3.45 (t, 2H), 3.08 (t, *J* = 6.0 Hz, 2H).

### [Example 131]: 6-(6,7-dimethoxy-3-methyl-4-oxo-3,4-dihydrophthalazin-1-yl)-3,4-dihydroisoquinoline-2(1H)-sulfonamide hydrochloride

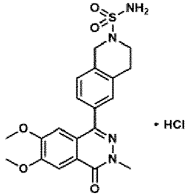

¹H NMR (400 MHz, MeOD) δ 7.83 (s, 1H), 7.44 (s, 2H), 7.33 (d, *J* = 9.2 Hz, 1H), 7.12 (d, *J* = 3.7 Hz, 1H), 4.46 (s, 1H), 4.42 (s, 1H), 4.03 (s, 3H), 3.88 (s, 3H), 3.84 (s, 3H), 3.54 (t, 1H), 3.49 (t, 1H), 3.09 (t, 2H).

### [Preparation Example 2]

A phthalazine derivative was prepared through the following Reaction Scheme 2 using, as a reactant, Product 2 obtained in Preparation Example 1.

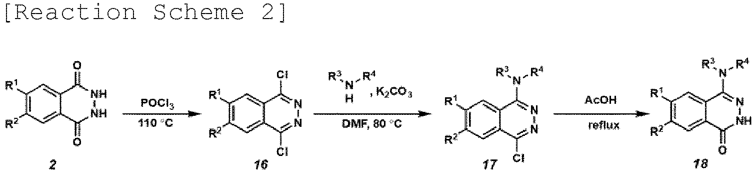

**Step 1: 2** was stirred in phosphorus oxychloride (0.67 M) in a round bottom flask at 110°C for 1 hour. After the reaction was complete, the reaction mixture was cooled to room temperature, slowly added to ice water, and stirred for 10 minutes. The reaction product was filtered, and the solid was washed with distilled water and dried under reduced pressure to obtain **16.** Examples of **16** are as follows.

### Reactant 16-1: 1,4-dichlorophthalazine

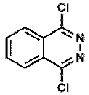

¹H NMR (400 MHz, DMSO) δ 8.43-8.35 (m, 2H), 8.32-8.25 (m, 2H).

### Reactant 16-2: 1,4-dichloro-6,7-dimethoxyphthalazine

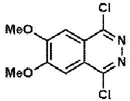

¹H NMR (DMSO, 600 MHz) δ 7.53 (s, 2H), 4.08 (s, 6H).

**Step 2: 16** (1.0 eq.) was dissolved in *N,N-*dimethylformamide (0.25 M) in a round bottom flask, amine (1.5 eq. mmol) and potassium carbonate (4.0 eq.) were added thereto, followed by stirring at 80°C under a nitrogen atmosphere for 14 hours. After the reaction was complete, the mixture was poured into water, followed by extraction with ethyl acetate. The organic layer was dried with anhydrous sodium sulfate and concentrated under reduced pressure. The obtained residue was separated by column chromatography (5% MeOH/CH₂Cl₂) to obtain **17.** Examples of 17 are as follows.

### Reactant 17-1: N-(2-(1-(4-chlorophthalazin-1-yl)piperidin-4-yl)ethyl)sulfamide

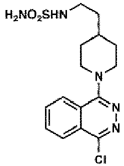

¹H NMR (400 MHz, DMSO) δ 8.24-8.16 (m, 1H), 8.14-8.02 (m, 3H), 6.52-6.44 (m, 3H), 3.87-3.78 (m, 2H), 3.05-2.92 (m, 4H), 1.88-1.78 (m, 2H), 1.73-1.60 (m, 1H), 1.58-1.39 (m, 4H).

### Reactant 17-2: tert-butyl ((1-(4-chlorophthalazin-1-yl)piperidin-4-yl)methyl)carbamate

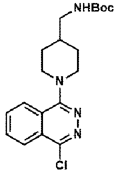

¹H NMR (DMSO, 600 MHz) δ 8.20-8.18 (m, 1H), 8.11-8.09 (m, 1H), 8.08-8.05 (m, 2H), 6.95 (t, *J* = 6 Hz, 1H), 3.82 (d, *J* = 12 Hz, 2H), 2.97 (t, *J* = 12 Hz, 2H), 2.93 (t, *J* = 6 Hz, 2H), 1.79 (d, *J* = 12 Hz, 2H), 1.67-1.63 (m, 1H), 1.47-1.42 (m, 2H), 1.39 (s, 9H).

### Reactant 17-3: tert-butyl ((1-(4-chloro-6,7-dimethoxyphthalazin-1-yl)piperidin-4-yl)methyl)carbamate

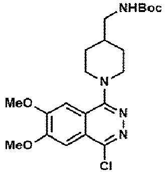

¹H NMR (DMSO, 600 MHz) δ 7.39 (s, 1H), 7.25 (s, 1H), 6.92 (t, *J* = 6 Hz, 1H), 4.01 (s, 3H), 4.01 (s, 3H), 3.75 (d, *J* = 12 Hz, 2H), 2.94-2.90 (m, 4H), 1.79 (d, *J* = 13 Hz, 2H), 1.66-1.63 (m, 1H), 1.47-1.43 (m, 2H), 1.39 (s, 9H).

**Step 3: 17** *N*-(2-(1-(4-chlorophthalazin-1-yl)piperidin-4-yl)ethyl)sulfamide was dissolved in acetic acid (0.1 M) in a round bottom flask, followed by refluxing at 120°C for 10 hours. The reaction product was concentrated, diluted with ethyl acetate and neutralized with sodium bicarbonate. An aqueous solution layer was extracted with ethyl acetate and the organic layer was dried with sodium sulfate and concentrated under reduced pressure. The obtained residue was solidified with diethyl ether to obtain **18.**

### [Example 132]: tert-butyl ((1-(4-oxo-3,4-dihydrophthalazin-1-yl)piperidin-4-yl)methyl)carbamate

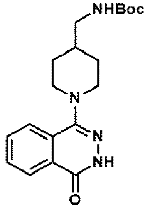

¹H NMR (DMSO, 600 MHz) δ 12.08 (s, 1H), 8.23-8.21 (m, 1H), 7.93-7.87 (m, 2H), 7.84-7.81 (m, 1H), 6.92 (t, *J* = 6 Hz, 1H), 3.41 (d, *J* = 12 Hz, 2H), 2.91 (t, *J* = 6 Hz, 2H), 2.67 (t, *J* = 12 Hz, 2H), 1.75 (d, *J* = 12 Hz, 2H), 1.57-1.51 (m, 1H), 1.41-1.35 (m, 11H).

### [Example 133]: tert-butyl ((1-(6,7-dimethoxy-4-oxo-3,4-dihydrophthalazin-1-yl)piperidin-4-yl)methyl)carbamate

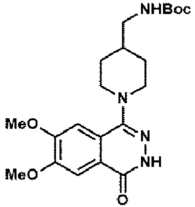

¹H NMR (DMSO, 600 MHz) δ 11.96 (s, 1H), 7.57 (s, 1H), 7.16 (s, 1H), 6.92 (t, *J* = 6 Hz, 1H), 3.93 (s, 3H), 3.92 (s, 3H), 3.41 (d, *J* = 12 Hz, 2H), 2.91 (t, *J* = 6 Hz, 2H), 2.67-2.63 (m, 2H), 1.75 (d, *J* = 12 Hz, 2H), 1.58-1.51 (m, 1H), 1.38 (s, 9H), 1.37--1.35 (m, 2H).

### [Example 134]: 4-(4-(aminomethyl)piperidin-1-yl)phthalazin-1(2H)-one hydrochloride

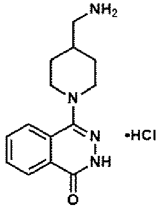

¹H NMR (DMSO, MHz) δ 12.13 (s, 1H), 8.24-8.22 (m, 1H), 7.97-7.81 (m, 6H), 3.46 (d, *J* = 12 Hz, 2H), 2.82-2.78 (m, 2H), 2.73-2.69 (m, 2H), 1.86 (d, *J* = 13 Hz, 2H), 1.78-1.75 (m, 1H), 1.50-1.44 (m, 2H).

### [Example 135]: 4-(4-(aminomethyl)piperidin-1-yl)-6,7-dimethoxyphthalazin-1(2H)-one hydrochloride

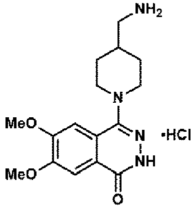

¹H NMR (DMSO, 600 MHz) δ 12.00 (s, 1H), 7.79 (br s, 3H), 7.58 (s, 1H), 7.16 (s, 1H), 3.94 (s, 3H), 3.93 (s, 3H), 3.46 (d, *J* = 12 Hz, 2H), 2.82-2.80 (m, 2H), 2.69 (t, *J* = 12 Hz, 2H), 1.85 (d, *J* = 13 Hz, 2H), 1.79-1.72 (m, 1H), 1.51-1.45 (m, 2H).

### [Example 136]: tert-butyl N-((1-(4-oxo-3,4-dihydrophthalazin-1-yl)piperidin-4-yl)methyl)sulfamoyl carbamate

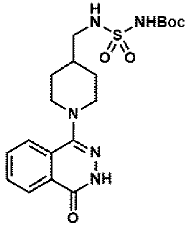

¹H NMR (DMSO, 600 MHz) δ 12.08 (s, 1H), 10.82 (s, 1H), 8.23-8.21 (m, 1H), 7.93-7.86 (m, 2H), 7.84-7.81 (m, 1H), 7.70 (br s, 1H), 3.41 (d, *J* = 13 Hz, 2H), 2.86 (s, 2H), 2.67 (t, *J* = 12 Hz, 2H), 1.83 (d, *J* = 13 Hz, 2H), 1.65-1.63 (m, 1H), 1.43-1.35 (m, 11H).

### [Example 137]: tert-butyl N-((1-(6,7-dimethoxy-4-oxo-3,4-dihydrophthalazin-1-yl)piperidin-4-yl)methyl)sulfamoyl carbamate

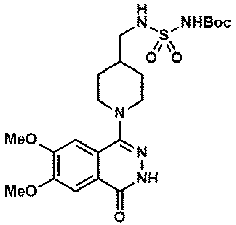

¹H NMR (DMSO, 600 MHz) δ 11.96 (s, 1H), 10.81 (s, 1H), 7.70 (br s, 1H), 7.57 (s, 1H), 7.16 (s, 1H), 3.94 (s, 3H), 3.92 (s, 3H), 3.41 (d, *J* = 12 Hz, 2H), 2.86 (br s, 2H), 2.65 (t, *J* = 12 Hz, 2H), 1.83 (d, *J* = 13 Hz, 2H), 1.68-1.60 (m, 1H), 1.42 (s, 9H), 1.40-1.36 (m, 2H).

### [Example 138]: N-((1-(4-oxo-3,4-dihydrophthalazin-1-yl)piperidin-4-yl)methyl)sulfamide hydrochloride

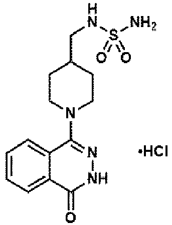

¹H NMR (DMSO, 600 MHz) δ 12.07 (s, 1H), 8.23-8.21 (m, 1H), 7.93-7.88 (m, 2H), 7.84-7.81 (m, 1H), 6.59 (t, *J* = 6 Hz, 1H), 6.49 (s, 2H), 3.42 (d, *J* = 12 Hz, 2H), 2.85 (t, *J* = 6 Hz, 2H), 2.71-2.67 (m, 2H), 1.85 (d, *J* = 12 Hz, 2H), 1.68-1.64 (m, 1H), 1.44-1.37 (m, 2H).

### [Example 139]: N-((1-(6,7-dimethoxy-4-oxo-3,4-dihydrophthalazin-1-yl)piperidin-4-yl)methyl)sulfamide hydrochloride

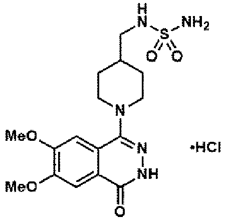

¹H NMR (DMSO, 600 MHz) δ 11.96 (s, 1H), 7.57 (s, 1H), 7.17 (s, 1H), 6.58 (br s, 1H), 6.49 (br s, 2H), 3.94 (s, 3H), 3.93 (s, 3H), 3.42 (d, *J* = 13 Hz, 2H), 2.85 (d, *J* = 7 Hz, 2H), 2.69-2.65 (m, 2H), 1.85 (d, *J* = 13 Hz, 2H), 1.67-1.64 (m, 1H), 1.43-1.37 (m, 2H).

### [Example 140]: N-(4-(6,7-dimethoxy-3-methyl-4-oxo-3,4-dihydrophthalazin-1-yl)-amino)phenyl)sulfamide hydrochloride

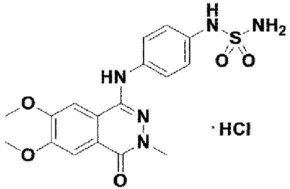

LCMS [M+H]⁺ 406.13

### [Example 141]: N-(3-(6,7-dimethoxy-3-methyl-4-oxo-3,4-dihydrophthalazin-1-yl)-amino)phenyl)sulfamide hydrochloride

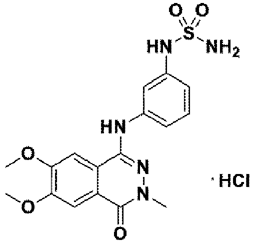

LCMS [M+H]⁺ 406.12

### [Example 142] : N-(2-(1-(4-oxo-3,4-dihydrophthalazin-1-yl)piperidin-4-yl)ethyl)sulfamide trifluoroacetic acid

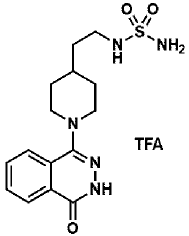

¹H NMR (600 MHz, DMSO) δ 12.08 (s, 1H), 8.23-8.20 (m, 1H), 7.94-7.87 (m, 2H), 7.85-7.80 (m, 2H), 7.17 (s, 1H), 7.09 (s, 1H), 7.00 (s, 1H), 3.41 (d, *J* = 12.3 Hz, 2H), 3.10 (q, *J* = 6.7 Hz, 2H), 2.72-2.65 (m, 2H), 1.81-1.76 (m, 2H), 1.52-1.36 (m, 5H).

### [Preparation Example 3]

**Preparation Example 3** includes a process of preparing Product **23**.** Then, a phthalazine derivative was prepared through Preparation Example 3-2 using Product **22**** or **23**** as Reactant **24**.**

### Preparation Example 3-1: Reactant 23**

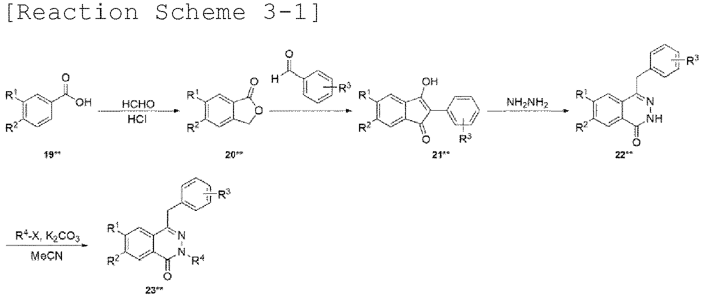

**Step 1: 19**** (1.0 eq.) was dissolved in a 37% aqueous hydrochloric acid solution (0.37 M) in a round bottom flask, and 30% formaldehyde (1.25 eq.) was added thereto, followed by stirring at 90°C for 10 hours. The reaction product was cooled to room temperature and was poured into water, followed by extraction with ethyl acetate. The organic layer was washed with a 2.5 M sodium hydroxide aqueous solution and a brine aqueous solution, dried with anhydrous sodium sulfate and concentrated under reduced pressure. The obtained residue was purified by column chromatography (EtOAc:hexanes = 4:6) to obtain **20**** (59-70% yield).

### Reactant 20**-1: 5,6-dimethoxyisobenzofuran-1(3H)-one

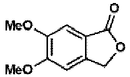

¹H NMR (400 MHz, CDCl₃): δ 7.32 (s, 1H), 6.90 (s, 1H), 5.23 (s, 2H), 3.98 (s, 3H), 3.95 (s, 3H).

**Step 2: 20**** (1.0 eq.) and aryl aldehyde (1.0 eq.) were dissolved in ethyl acetate in a round bottom flask and a sodium methoxide solution (25% in methanol) (4.0 eq.) was slowly added thereto at 0°C. The reaction product was stirred at room temperature for 1 hour, and at 80°C for 1 hour, and then concentrated under reduced pressure. Ice water was added to the residue and then the pH of the mixture was adjusted to 2-3 with acetic acid. The reaction product was filtered to obtain Solid **21**** (21-60%).

### Reactant 21**-1: 3-hydroxy-5,6-dimethoxy-2-phenyl-1H-inden-1-one

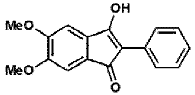

¹H NMR (400 MHz, CDCl₃): δ 7.42 (s, 2H), 7.38-7.28 (m, 3H), 7.21-7.15 (m, 2H), 4.21 (s, 1H), 4.05 (s, 6H).

### Reactant 21**-2: 2-(4-bromophenyl)-3-hydroxy-5,6-dimethoxy-1H-inden-1-one

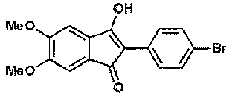

¹H NMR (400 MHz, CDCl₃): δ 7.48 (d, *J* = 8.5 Hz, 2H), 7.41 (s, 2H), 7.07 (d, *J* = 8.4 Hz, 2H), 4.17 (s, 1H), 4.05 (s, 6H).

### Reactant 21**-3: 2-(3-bromophenyl)-3-hydroxy-5,6-dimethoxy-1H-inden-1-one

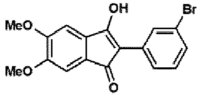

¹H NMR (400 MHz, CDCl ₃): δ 7.44 (ddd, *J* = 7.9, 1.9, 1.1 Hz, 1H), 7.41 (s, 2H), 7.32 (t, *J* = 1.9 Hz, 1H), 7.23 (t, *J* = 7.9 Hz, 1H), 7.17-7.13 (m, 1H), 4.18 (s, 1H), 4.06 (s, 6H).

**Step 3-1: 21**** (1.0 eq.) was added to water in a round bottom flask and 4.7M hydrazine monohydrate (24.5 eq.) was added thereto. The reaction product was stirred at 100°C for 5 hours and cooled to room temperature. The solid was filtered and washed with ethanol to obtain **22**** (40-62% yield).

**Step 3-2: 23**** was prepared from Reactant **22**** through Step 2-2 of Preparation Example 1-1.

### [Example 143]: 4-benzylphthalazin-1(2H)-one

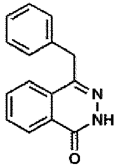

¹H NMR (400 MHz, DMSO) δ 12.60 (s, 1H), 8.25 (dd, *J* = 7.8, 1.0 Hz, 1H), 7.93 (d, *J* = 7.6 Hz, 1H), 7.88-7.83 (m, 1H), 7.80 (t, *J* = 7.5 Hz, 1H), 7.33-7.25 (m, 4H), 7.18 (t, *J* = 6.9 Hz, 1H), 4.29 (s, 2H).

### [Example 144]: 4-benzyl-6,7-dimethoxyphthalazin-1(2H)-one

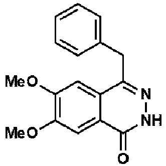

¹H NMR (400 MHz, DMSO): δ 12.41 (s, 1H), 7.59 (s, 1H), 7.37-7.25 (m, 5H), 7.19 (t, *J* = 7.2 Hz, 1H), 4.28 (s, 2H), 3.90 (s, 3H), 3.83 (s, 3H).

### [Example 145]: tert-butyl (3-((6,7-dimethoxy-4-oxo-3,4-dihydrophthalazin-1-yl)methyl)phenyl)carbamate

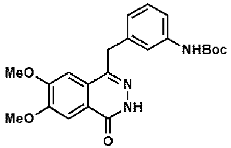

¹H NMR (600 MHz, CDCl₃) δ 10.10 (s, 1H), 7.76 (s, 1H), 7.51 (s, 1H), 7.20 (t, *J* = 7.8 Hz, 1H), 7.13-7.09 (m, 2H), 6.95 (d, *J* = 7.5 Hz, 1H), 6.46 (s, 1H), 4.23 (s, 2H), 4.01 (s, 3H), 3.88 (s, 3H), 1.49 (s, 9H).

### [Example 146]: tert-butyl (3-((6,7-dimethoxy-3-methyl-4-oxo-3,4-dihydrophthalazin-1-yl)methyl)phenyl)carbamate

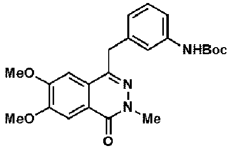

¹H NMR (600 MHz, DMSO) δ 9.27 (s, 1H), 7.59 (s, 1H), 7.47 (s, 1H), 7.28-7.24 (m, 2H), 7.16 (t, *J* = 7.9 Hz, 1H), 6.97 (d, *J* = 7.6 Hz, 1H), 4.22 (s, 2H), 3.90 (s, 3H), 3.84 (s, 3H), 3.72 (s, 3H), 1.43 (s, 9H).

### [Example 147]: tert-butyl (4-((4-oxo-3,4-dihydrophthalazin-1-yl)methyl)phenyl)carbamate

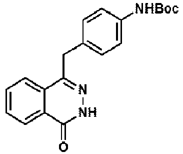

¹H NMR (600 MHz, DMSO)δ 12.51 (s, 1H), 9.19 (s, 1H), 8.20 (dd, *J* = 7.8, 1.5 Hz, 1H), 7.88-7.85 (m, 1H), 7.84-7.79 (m, 1H), 7.78-7.74 (m, 1H), 7.30 (d, *J* = 8.2 Hz, 2H), 7.14 (d, *J* = 8.6 Hz, 2H), 4.17 (s, 2H), 1.41 (s, 9H).

### [Example 148]: tert-butyl (4-((3-methyl-4-oxo-3,4-dihydrophthalazin-1-yl)methyl)phenyl)carbamate

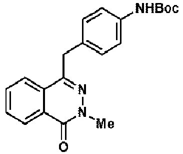

¹H NMR (600 MHz, DMSO) δ 9.24 (s, 1H), 8.32-8.20 (m, 1H), 7.91-7.88 (m, 1H), 7.85-7.79 (m, 2H), 7.34 (d, *J* = 8.2 Hz, 2H), 7.20 (d, *J* = 8.6 Hz, 2H), 4.22 (s, 2H), 3.74 (s, 3H), 1.44 (s, 9H).

### [Example 149]: tert-butyl (4-((6,7-dimethoxy-4-oxo-3,4-dihydrophthalazin-1-yl)methyl)phenyl)carbamate

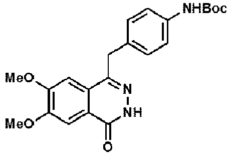

¹H NMR (600 MHz, CDCl₃) δ 9.75 (s, 1H), 7.76 (s, 1H), 7.30 (d, *J* = 8.0 Hz, 2H), 7.20 (d, *J* = 8.5 Hz, 2H), 7.01 (s, 1H), 6.42 (s, 1H), 4.20 (s, 2H), 4.01 (s, 3H), 3.85 (s, 3H), 1.50 (s, 9H).

### [Example 150]: tert-butyl (4-((6,7-dimethoxy-3-methyl-4-oxo-3,4-dihydrophthalazin-1-yl)methyl)phenyl)carbamate

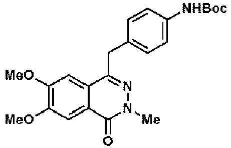

¹H NMR (600 MHz, CDCl ₃) δ 7.77 (s, 1H), 7.29 (d, *J* = 8.2 Hz, 2H), 7.19 (d, *J* = 8.5 Hz, 2H), 6.95 (s, 1H), 6.42 (s, 1H), 4.19 (s, 2H), 4.00 (s, 3H), 3.87 (s, 3H), 3.81 (s, 3H), 1.49 (s, 9H).

### [Example 151]: tert-butyl (4-((4-oxo-3,4-dihydrophthalazin-1-yl)methyl)phenyl)(methyl)carbamate

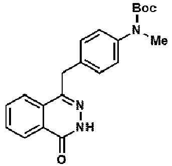

¹H NMR (600 MHz, DMSO) δ 12.59 (s, 1H), 8.25 (dd, *J* = 7.9, 1.4 Hz, 1H), 7.96 (d, *J* = 8.0 Hz, 1H), 7.87 (td, *J* = 8.2, 7.7, 1.5 Hz, 1H), 7.81 (t, *J* = 7.6 Hz, 1H), 7.27 (d, *J* = 8.5 Hz, 2H), 7.18 (d, *J* = 8.4 Hz, 2H), 4.28 (s, 2H), 3.12 (s, 3H), 1.36 (s, 9H).

### [Example 152]: tert-butyl (4-((6,7-dimethoxy-4-oxo-3,4-dihydrophthalazin-1-yl)methyl)phenyl)(methyl)carbamate

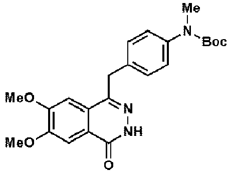

¹H NMR (600 MHz, DMSO) δ 12.41 (s, 1H), 7.59 (s, 1H), 7.30 (d, *J* = 8.5 Hz, 2H), 7.27 (s, 1H), 7.18 (d, *J* = 8.5 Hz, 2H), 4.26 (s, 2H), 3.90 (s, 3H), 3.84 (s, 3H), 3.12 (s, 3H), 1.35 (s, 9H).

### [Example 153]: tert-butyl (4-((6,7-dimethoxy-3-methyl-4-oxo-3,4-dihydrophthalazin-1-yl)methyl)phenyl) (methyl)carbamate

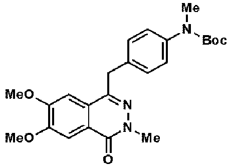

¹H NMR (600 MHz, DMSO) δ 7.60 (s, 1H), 7.32 (d, *J* = 8.3 Hz, 2H), 7.25 (s, 1H), 7.18 (d, *J* = 8.4 Hz, 2H), 4.27 (s, 2H), 3.90 (s, 3H), 3.82 (s, 3H), 3.72 (s, 3H), 3.11 (s, 3H), 1.34 (s, 9H).

### [Example 154]: tert-butyl (5-((6,7-dimethoxy-4-oxo-3,4-dihydrophthalazin-1-yl)methyl)indoline-1-carboxylate

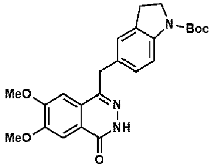

¹H NMR (600 MHz, DMSO) δ 12.40 (s, 1H), 7.70-7.47 (m, 2H), 7.28 (s, 1H), 7.14 (apparent s, 2H), 4.19 (s, 2H), 3.90 (s, 3H), 3.87-3.81 (m, 5H), 2.99 (t, *J* = 8.6 Hz, 2H), 1.47 (s, 9H).

### [Example 155]: tert-butyl (5-((6,7-dimethoxy-3-methyl-4-oxo-3,4-dihydrophthalazin-1-yl)methyl)indoline-1-carboxylate

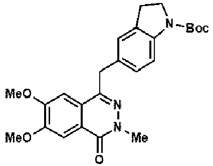

¹H NMR (600 MHz, DMSO) δ 7.69-7.49 (m, 2H), 7.26 (s, 1H), 7.16 (apparent s, 2H), 4.20 (s, 2H), 3.90 (s, 3H), 3.87 (m, 5H), 3.72 (s, 3H), 2.99 (t, *J* = 8.7 Hz, 2H), 1.47 (s, 9H).

### Preparation Example 3-2

A preparation process was performed using the following Reaction Scheme 3-2 using **22**** or **23**** prepared in Preparation Example 3-1 as Reactant **24**.**

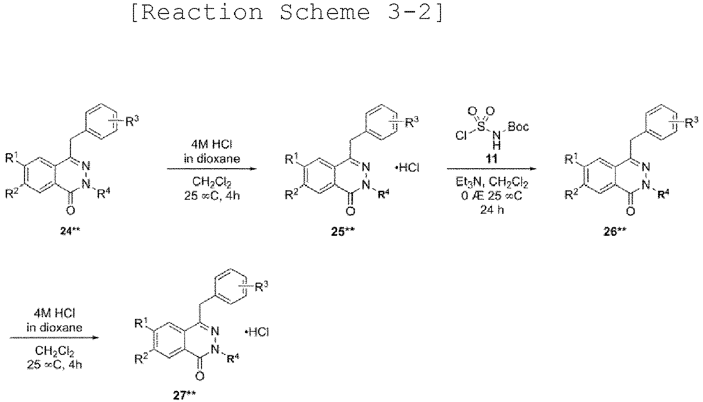

**Step 1: 25**** was prepared from Reactant **24**** through Step 2 of Preparation Example 1-3.

### [Example 156]: 4-(3-aminobenzyl)-6,7-dimethoxyphthalazin-1(2H)-one hydrochloride

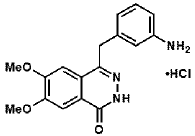

¹H NMR (600 MHz, DMSO) δ 12.48 (s, 1H), 7.60 (s, 1H), 7.45-7.34 (m, 2H), 7.26 (s, 1H), 7.17-7.04 (m, 2H), 4.32 (s, 2H), 3.91 (s, 3H), 3.86 (s, 3H).

### [Example 157]: 4-(3-aminobenzyl)-6,7-dimethoxy-2-methylphthalazin-1(2H)-one hydrochloride

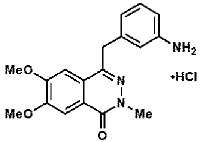

¹H NMR (600 MHz, DMSO) δ 7.61 (s, 1H), 7.37-7.29 (m, 3H), 7.25 (s, 1H), 7.06 (d, *J* = 29.7 Hz, 2H), 4.32 (s, 2H), 3.91 (s, 3H), 3.84 (s, 3H), 3.72 (s, 3H).

### [Example 158]: 4-(4-aminobenzyl)-phthalazin-1(2H)-one hydrochloride

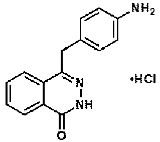

¹H NMR (600 MHz, DMSO) δ 12.59 (s, 1H), 9.59 (br s, 2H), 8.25 (dd, *J* = 7.8, 1.4 Hz, 1H), 7.92 (d, *J* = 8.0 Hz, 1H), 7.87 (td, *J* = 8.1, 7.6, 1.4 Hz, 1H), 7.82 (td, *J* = 7.5, 1.2 Hz, 1H), 7.37 (apparent br s, 2H), 7.18 (apparent br s, 2H), 4.31 (s, 2H).

### [Example 159]: 4-(4-aminobenzyl)-2-methylphthalazin-1(2H)-one hydrochloride

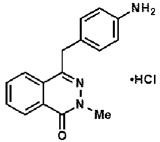

¹H NMR (600 MHz, DMSO) δ 9.49 (s, 2H), 8.29-8.27 (m, 1H), 7.92-7.89 (m, 1H), 7.86-7.80 (m, 2H), 7.38 (d, *J* = 7.8 Hz, 2H), 7.16 (apparent br s, 2H), 4.32 (s, 2H), 3.74 (s, 3H).

### [Example 160]: 4-(4-aminobenzyl)-6,7-dimethoxyphthalazin-1(2H)-one hydrochloride

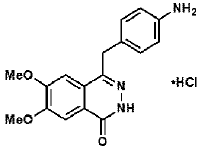

¹H NMR (600 MHz, DMSO) δ 12.44 (s, 1H), 10.02 (br s, 3H), 7.59 (s, 1H), 7.44 (d, *J* = 8.4 Hz, 2H), 7.31-7.25 (m, 3H), 4.32 (s, 2H), 3.91 (s, 3H), 3.86 (s, 3H).

### [Example 161]: 4-(4-aminobenzyl)-6,7-dimethoxy-2-methylphthalazin-1(2H)-one hydrochloride

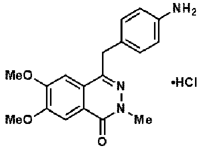

¹H NMR (600 MHz, DMSO) δ 7.60 (s, 1H), 7.38 (apparent br s, 2H), 7.24 (s, 1H), 7.13 (apparent br s, 2H), 4.29 (s, 2H), 3.91 (s, 3H), 3.83 (s, 3H), 3.71 (s, 3H).

### [Example 162]: 4-(4-(methylamino)benzyl)phthalazin-1(2H)-one hydrochloride

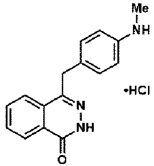

¹H NMR (DMSO, 600 MHz) δ 12.57 (s, 1H), 8.25 (d, *J* = 8 Hz, 1H), 7.94 (d, *J* = 9 Hz, 1H), 7.86 (t, *J* = 7 Hz, 1H), 7.81 (t, *J* = 7 Hz, 1H), 7.26 (apparent br s, 2H), 6.98 (apparent br s, 2H), 4.24 (s, 2H), 2.76 (s, 3H).

### [Example 163]: 6,7-dimethoxy-4-(4-(methylamino)benzyl)phthalazin-1(2H)-one hydrochloride

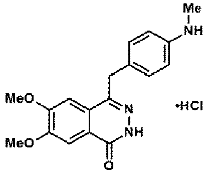

¹H NMR (DMSO, 600 MHz) δ 12.41 (s, 1H), 7.59 (s, 1H), 7.36 (apparent br s, 2H), 7.28 (s, 1H), 7.12 (apparent br s, 2H), 4.26 (s, 2H), 3.91 (s, 3H), 3.86 (s, 3H), 2.80 (s, 3H).

### [Example 164]: 6,7-dimethoxy-2-methyl-4-(4-(methylamino)benzyl)phthalazin-1(2H)-one hydrochloride

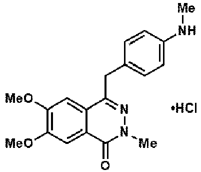

¹H NMR (600 MHz, DMSO) δ 7.59 (s, 1H), 7.30 (apparent br s, 2H), 7.26 (s, 1H), 6.94 (apparent br s, 2H), 4.23 (s, 2H), 3.90 (s, 3H), 3.84 (s, 3H), 3.71 (s, 3H), 2.75 (s, 3H).

### [Example 165]: 4-(indolin-5-yl-methyl)-6,7-dimethoxyphthalazin-1(2H)-one hydrochloride

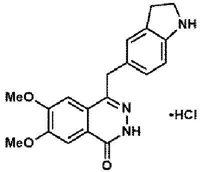

¹H NMR (DMSO, 600 MHz) δ 12.42 (s, 1H), 7.59 (s, 1H), 7.35 (br s, 1H), 7.33-7.29 (m, 2H), 7.23 (br s, 1H), 4.30 (s, 2H), 3.91 (s, 3H), 3.87 (s, 3H), 3.64 (t, *J* = 8 Hz, 2H), 3.10 (t, *J* = 8 Hz, 2H).

### [Example 166]: 4-(indolin-5-yl-methyl)-6,7-dimethoxy-2-methylphthalazin-1(2H)-one hydrochloride

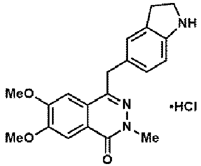

¹H NMR (DMSO, 600 MHz) δ 7.60 (s, 1H), 7.38-7.30 (m, 2H), 7.27 (s, 1H), 7.19 (br s, 1H), 4.30 (s, 2H), 3.91 (s, 3H), 3.84 (s, 3H), 3.72 (s, 3H), 3.62 (t, *J* = 8 Hz, 2H), 3.08 (t, *J* = 8 Hz, 2H).

**Step 2: 26**** was prepared from Reactant **25**** through Step 3 of Preparation Example 1-3.

### [Example 167]: tert-butyl (N-(3-((6,7-dimethoxy-4-oxo-3,4-dihydrophthalazin-1-yl)methyl)phenyl)sulfamoyl)carbamate

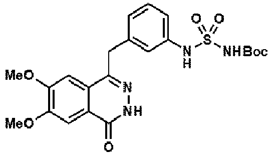

¹H NMR (600 MHz, DMSO) δ 12.42 (s, 1H), 11.16 (s, 1H), 10.20 (s, 1H), 7.58 (s, 1H), 7.25 (t, *J* = 7.8 Hz, 1H), 7.23 (s, 1H), 7.12 (d, *J* = 7.4 Hz, 1H), 7.09 (s, 1H), 7.04 (d, *J* = 8.1 Hz, 1H), 4.22 (s, 2H), 3.90 (s, 3H), 3.84 (s, 3H), 1.24 (s, 9H).

### [Example 168]: tert-butyl (N-(3-((6,7-dimethoxy-3-methyl-4-oxo-3,4-dihydrophthalazin-1-yl)methyl)phenyl)sulfamoyl)carbamate

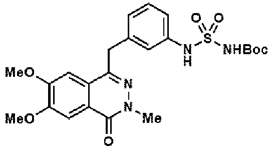

¹H NMR (600 MHz, DMSO) δ 11.16 (s, 1H), 10.19 (s, 1H), 7.59 (s, 1H), 7.25 (t, *J* = 7.8 Hz, 1H), 7.22 (s, 1H), 7.15 (d, *J* = 7.7 Hz, 1H), 7.10 (s, 1H), 7.04 (d, *J* = 8.1 Hz, 1H), 4.23 (s, 2H), 3.90 (s, 3H), 3.83 (s, 3H), 3.72 (s, 3H), 1.23 (s, 9H).

### [Example 169]: tert-butyl (N-(4-((4-oxo-3,4-dihydrophthalazin-1-yl)methyl)phenyl)sulfamoyl)carbamate

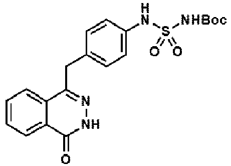

¹H NMR (600 MHz, DMSO) δ 12.57 (s, 1H), 11.15 (s, 1H), 10.14 (s, 1H), 8.24 (dd, *J* = 7.8, 1.5 Hz, 1H), 7.96-7.89 (m, 1H), 7.86-7.83 (m, 1H), 7.80 (td, *J* = 7.5, 1.2 Hz, 1H), 7.28-7.17 (m, 2H), 7.06 (d, *J* = 8.1 Hz, 2H), 4.23 (s, 2H), 1.24 (s, 9H).

### [Example 170]: tert-butyl (N-(4-((3-methyl-4-oxo-3,4-dihydrophthalazin-1-yl)methyl)phenyl)sulfamoyl)carbamate

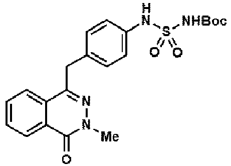

¹H NMR (600 MHz, DMSO) δ 11.14 (s, 1H), 10.16 (s, 1H), 8.27 (dd, *J* = 7.6, 1.6 Hz, 1H), 7.93-7.88 (m, 1H), 7.85-7.79 (m, 2H), 7.27 (d, *J* = 8.0 Hz, 2H), 7.06 (d, *J* = 8.2 Hz, 2H), 4.25 (s, 2H), 3.74 (s, 3H), 1.23 (s, 9H).

### [Example 171]: tert-butyl (N-(4-((6,7-dimethoxy-4-oxo-3,4-dihydrophthalazin-1-yl)methyl)phenyl)sulfamoyl)carbamate

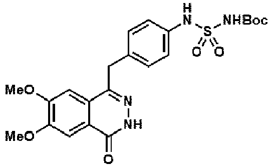

¹H NMR (600 MHz, DMSO) δ 12.40 (s, 1H), 11.11 (s, 1H), 10.15 (s, 1H), 7.57 (s, 1H), 7.28 (d, *J* = 8.3 Hz, 2H), 7.23 (s, 1H), 7.08 (d, *J* = 8.5 Hz, 2H), 4.22 (s, 2H), 3.89 (s, 3H), 3.83 (s, 3H), 1.21 (s, 9H).

### [Example 172]: tert-butyl (N-(4-((6,7-dimethoxy-3-methyl-4-oxo-3,4-dihydrophthalazin-1-yl)methyl)phenyl)sulfamoyl)carbamate

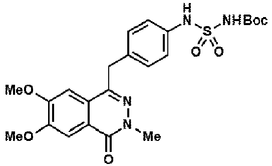

¹H NMR (600 MHz, DMSO) δ 11.12 (s, 1H), 10.18 (s, 1H), 7.58 (s, 1H), 7.30 (d, *J* = 8.1 Hz, 2H), 7.21 (s, 1H), 7.08 (d, *J* = 8.4 Hz, 2H), 4.23 (s, 2H), 3.89 (s, 3H), 3.81 (s, 3H), 3.72 (s, 3H), 1.19 (s, 9H).

### [Example 173]: tert-butyl N-methyl-N-(4-((4-oxo-3,4-dihydrophthalazin-1-yl)methyl)phenyl)sulfamoyl carbamate

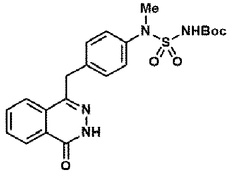

¹H NMR (DMSO, 600 MHz) δ 12.61 (s, 1H), 11.09 (s, 1H), 8.26-8.25 (m, 1H), 7.99-7.97 (m, 1H), 7.90-7.87 (m, 1H), 7.83-7.81 (m, 1H), 7.36 (d, *J* = 8 Hz, 2H), 7.26 (d, *J* = 8 Hz, 2H), 4.30 (s, 2H), 3.27 (s, 3H), 1.33 (s, 9H).

### [Example 174]: tert-butyl N-(4-((6,7-dimethoxy-4-oxo-3,4-dihydrophthalazin-1-yl)methyl)phenyl)-N-methylsulfamoyl carbamate

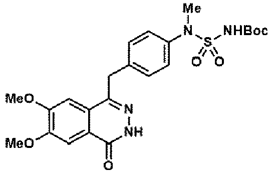

¹H NMR (DMSO, 600 MHz) δ 12.43 (s, 1H), 11.08 (s, 1H), 7.59 (s, 1H), 7.37 (br s, 2H), 7.28 (s, 2H), 7.26 (s, 1H), 4.27 (s, 2H), 3.90 (s, 3H), 3.85 (s, 3H), 3.28 (s, 3H), 1.31 (s, 9H).

### [Example 175]: tert-butyl N-(4-((6,7-dimethoxy-3-methyl-4-oxo-3,4-dihydrophthalazin-1-yl)methyl)phenyl)-N-methylsulfamoyl carbamate

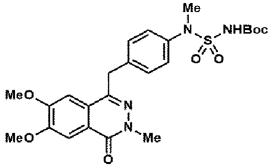

¹H NMR (DMSO, 600 MHz) δ 11.07 (s, 1H), 7.60 (s, 1H), 7.40 (d, *J* = 8 Hz, 2H), 7.27 (d, *J* = 8 Hz, 2H), 7.25 (s, 1H), 4.29 (s, 2H), 3.90 (s, 3H), 3.83 (s, 3H), 3.73 (s, 3H), 3.28 (s, 3H), 1.30 (s, 9H).

### [Example 176]: tert-butyl (5-((6,7-dimethoxy-4-oxo-3,4-dihydrophthalazin-1-yl)methyl)indolin-1-yl)sulfonyl carbamate

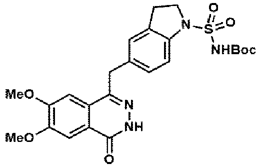

¹H NMR (DMSO, 600 MHz) δ 12.41 (s, 1H), 11.48 (s, 1H), 7.57 (s, 1H), 7.27 (s, 1H), 7.23 (d, *J* = 8 Hz, 1H), 7.18 (s, 1H), 7.09 (d, *J* = 8 Hz, 1H), 4.21 (s, 2H), 4.10 (t, *J* = 8 Hz, 2H), 3.89 (s, 3H), 3.85 (s, 3H), 3.02 (t, *J* = 8 Hz, 2H), 1.11 (s, 9H).

### [Example 177]: tert-butyl (5-((6,7-dimethoxy-3-methyl-4-oxo-3,4-dihydrophthalazin-1-yl)methyl)indolin-1-yl)sulfonyl carbamate

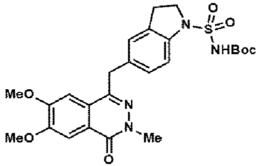

¹H NMR (DMSO, 600 MHz) δ 11.48 (s, 1H), 7.58 (s, 1H), 7.27-7.23 (m, 2H), 7.20-7.17 (m 1H), 7.09 (d, *J* = 8 Hz, 1H), 4.22 (s, 2H), 4.13-4.05 (m, 2H), 3.90 (s, 3H), 3.83 (s, 3H), 3.72 (s, 3H), 3.01 (t, *J* = 8 Hz, 2H), 1.10 (s, 9H).

**Step 3: 27**** was prepared from Reactant **26**** through Step 4 of Preparation Example 1-3.

### [Example 178]: (N-(3-((6,7-dimethoxy-4-oxo-3,4-dihydrophthalazin-1-yl)methyl)phenyl)sulfamide hydrochloride

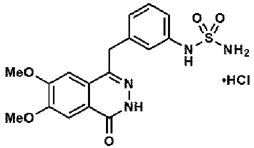

¹H NMR (600 MHz, DMSO) δ 12.42 (s, 1H), 9.45 (s, 1H), 7.58 (s, 1H), 7.25 (s, 1H), 7.17 (t, *J* = 7.9 Hz, 1H), 7.13 (t, *J* = 2.0 Hz, 1H), 7.06 (s, 2H), 7.01 (ddd, *J* = 8.1, 2.3, 1.0 Hz, 1H), 6.93 (d, *J* = 7.7 Hz, 1H), 4.21 (s, 2H), 3.90 (s, 3H), 3.84 (s, 3H).

### [Example 179]: N-(3-((6,7-dimethoxy-3-methyl-4-oxo-3,4-dihydrophthalazin-1-yl)methyl)phenyl)sulfamide hydrochloride

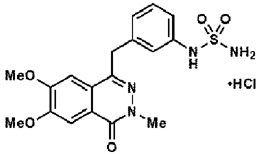

¹H NMR (600 MHz, DMSO) δ 9.44 (s, 1H), 7.60 (s, 1H), 7.23 (s, 1H), 7.19-7.14 (m, 2H), 7.06 (s, 2H), 7.02-6.99 (m, 1H), 6.94 (d, *J* = 7.5 Hz, 1H), 4.22 (s, 2H), 3.91 (s, 3H), 3.82 (s, 3H), 3.73 (s, 3H).

### [Example 180]: N-(4-((4-oxo-3,4-dihydrophthalazin-1-yl)methyl)phenyl)sulfamide hydrochloride

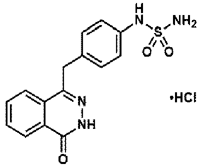

¹H NMR (DMSO, 600 MHz) δ 12.56 (s, 1H), 9.39 (s, 1H), 8.25-8.23 (m, 1H), 7.94-7.93 (m, 1H), 7.88-7.85 (m, 1H), 7.82-7.79 (m, 1H), 7.21-7.20 (m, 2H), 7.08-7.05 (m, 2H), 7.01 (s, 2H), 4.22 (s, 2H).

### [Example 181]: N-(4-((3-methyl-4-oxo-3,4-dihydrophthalazin-1-yl)methyl)phenyl)sulfamide hydrochloride

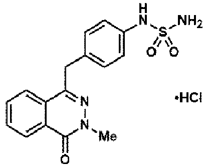

¹H NMR (DMSO, 600 MHz) δ 9.39 (s, 1H), 8.28-8.26 (m, 1H), 7.93-7.92 (m, 1H), 7.86-7.83 (m, 1H), 7.82-7.79 (m, 1H), 7.24-7.22 (m, 2H), 7.07-7.05 (m, 2H), 7.01 (s, 2H), 4.23 (s, 2H), 3.75 (s, 3H).

### [Example 182]: N-(4-((6,7-dimethoxy-4-oxo-3,4-dihydrophthalazin-1-yl)methyl)phenyl)sulfamide hydrochloride

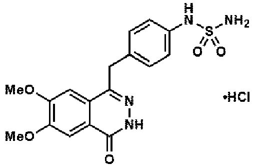

¹H NMR (600 MHz, DMSO) δ 12.38 (s, 1H), 9.37 (s, 1H), 7.58 (s, 1H), 7.28 (s, 1H), 7.23 (d, *J* = 8.5 Hz, 2H), 7.08 (d, *J* = 8.5 Hz, 2H), 7.00 (s, 2H), 4.20 (s, 2H), 3.90 (s, 3H), 3.85 (s, 3H).

### [Example 183]: N-(4-((6,7-dimethoxy-3-methyl-4-oxo-3,4-dihydrophthalazin-1-yl)methyl)phenyl)sulfamide hydrochloride

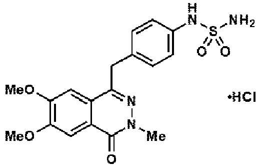

¹H NMR (600 MHz, DMSO) δ 9.38 (s, 1H), 7.59 (s, 1H), 7.27-7.24 (m, 3H), 7.07 (d, *J* = 8.6 Hz, 2H), 7.00 (s, 2H), 4.21 (s, 2H), 3.90 (s, 3H), 3.83 (s, 3H), 3.72 (s, 3H).

### [Example 184]: N-methyl-N-(4-((4-oxo-3,4-dihydrophthalazin-1-yl)methyl)phenyl)sulfamide hydrochloride

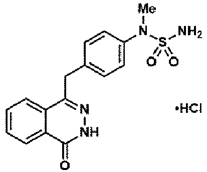

¹H NMR (DMSO, 600 MHz) δ 12.60 (s, 1H), 8.26-8.25 (m, 1H), 8.00-7.99 (m, 1H), 7.90-7.88 (m, 1H), 7.83-7.81 (m, 1H), 7.32 (d, *J* = 9 Hz, 2H), 7.25 (d, *J* = 8 Hz, 2H), 6.96 (s, 2H), 4.28 (s, 2H), 3.05 (s, 3H).

### [Example 185]: N-(4-((6,7-dimethoxy-4-oxo-3,4-dihydrophthalazin-1-yl)methyl)phenyl)-N-methylsulfamide hydrochloride

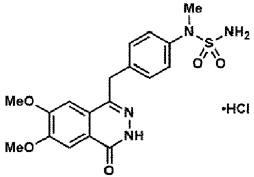

¹H NMR (DMSO, 600 MHz) δ 12.43 (s, 1H), 7.59 (s, 1H), 7.34 (d, *J* = 8 Hz, 2H), 7.31 (s, 1H), 7.26 (d, *J* = 8 Hz, 2H), 4.26 (s, 2H), 3.90 (s, 3H), 3.87 (s, 3H), 3.05 (s, 3H).

### [Example 186]: N-(4-((6,7-dimethoxy-3-methyl-4-oxo-3,4-dihydrophthalazin-1-yl)methyl)phenyl)-N-methylsulfamide hydrochloride

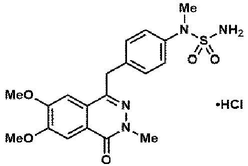

¹H NMR (DMSO, 600 MHz) δ 7.60 (s, 1H), 7.36 (d, *J* = 8 Hz, 2H), 7.29 (s, 1H), 7.26 (d, *J* = 9 Hz, 2H), 6.95 (s, 2H), 4.28 (s, 2H), 3.91 (s, 3H), 3.85 (s, 3H), 3.73 (s, 3H), 3.04 (s, 3H).

### [Example 187]: 5-((6,7-dimethoxy-4-oxo-3,4-dihydrophthalazin-1-yl)methyl)indoline-1-sulfonamide hydrochloride

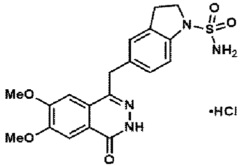

¹H NMR (DMSO, 600 MHz) δ 12.40 (s, 1H), 7.58 (s, 1H), 7.30 (s, 1H), 7.19 (br s, 2H), 7.17-7.16 (m, 3H), 4.20 (s, 2H), 3.90 (s, 3H), 3.87 (s, 3H), 3.74 (t, *J* = 8 Hz, 2H), 2.98 (t, *J* = 8 Hz, 2H).

### [Example 188]: 5-((6,7-dimethoxy-3-methyl-4-oxo-3,4-dihydrophthalazin-1-yl)methyl)indoline-1-sulfonamide hydrochloride

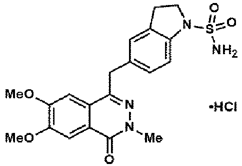

¹H NMR (DMSO, 600 MHz) δ 7.59 (s, 1H), 7.28 (s, 1H), 7.20-7.17 (m, 5H), 4.21 (s, 2H), 3.90 (s, 3H), 3.85 (s, 3H), 3.75 (d, *J* = 8 Hz, 2H), 3.72 (s, 3H), 2.97 (t, *J* = 8 Hz, 2H).

### [Example 189]: N-(3-fluoro-4-((4-oxo-3,4-dihydrophthalazin-1-yl)methyl)phenyl)sulfamide hydrochloride

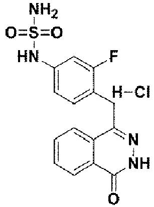

LCMS [M+H]⁺ 349.33

### [Example 190]: N-(2-fluoro-4-((4-oxo-3,4-dihydrophthalazin-1-yl)methyl)phenyl)sulfamide hydrochloride

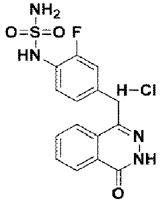

LCMS [M+H]⁺ 349.21

### [Example 191]: N-(3-(trifluorobenzyl methyl)-4-((4-oxo-3,4-dihydrophthalazin-1-yl)methyl)phenyl)sulfamide hydrochloride

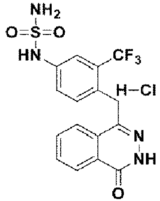

LCMS [M+H]⁺ 399.23

### [Example 192]: N-(2-(trifluorobenzyl methyl)-4-((4-oxo-3,4-dihydrophthalazin-1-yl)methyl)phenyl)sulfamide hydrochloride

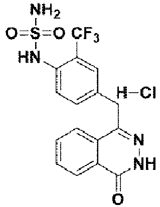

LCMS [M+H]⁺ 399.34

### [Example 193]: 4-(3-bromobenzyl)-6,7-dimethoxyphthalazin-1(2H)-one

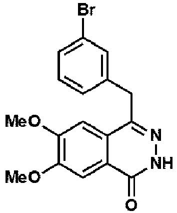

¹H NMR (400 MHz, DMSO) δ 12.44 (s, 1H), 7.61 (s, 2H), 7.42 (d, *J* = 7.9 Hz, 1H), 7.38-7.31 (m, 2H), 7.27 (t, *J* = 7.7 Hz, 1H), 4.32 (s, 2H), 3.93 (s, 3H), 3.90 (s, 3H) .

### [Example 194]: 4-(4-bromobenzyl)-6,7-dimethoxyphthalazin-1(2H)-one

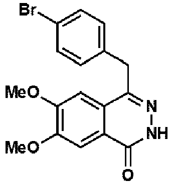

¹H NMR (400 MHz, DMSO) δ 12.42 (s, 1H), 7.59 (s, 1H), 7.49 (d, *J* = 8.3 Hz, 2H), 7.30 (d, *J* = 8.3 Hz, 2H), 7.26 (s, 1H), 4.27 (s, 2H), 3.91 (s, 3H), 3.86 (s, 3H).

### [Preparation Example 4-1]

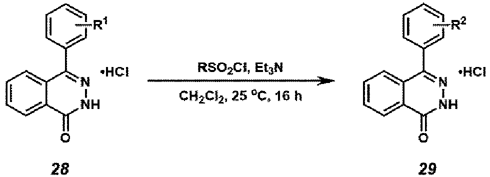

28 (1.0 eq.) and RSO₂Cl (1.1 eq.) were dissolved in dichloromethane (0.08 M) in a round bottom flask and triethylamine (4.5 eq.) was added thereto, followed by stirring at room temperature for 16 hours. After the reaction was complete, water was poured into the mixture, followed by extraction with dichloromethane. The organic layer was dried over anhydrous magnesium sulfate and concentrated under reduced pressure. The obtained residue was purified by column chromatography (MeOH:CH₂Cl₂ = 1:20) to obtain ***29*** (20-40% yield).

### [Example 195]: N -(4-(4-oxo-3,4-dihydrophthalazin-1-yl)benzyl)methanesulfonamide

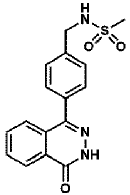

¹H NMR (400 MHz, MeOD) δ 8.47 (s, 1H), 7.96 - 7.89 (m, 2H), 7.82 (s, 1H), 7.67 - 7.58 (m, 4H), 4.40 (s, 2H), 2.96 (s, 3H).

### [Example 196]: N -(4-(4-oxo-3,4-dihydrophthalazin-1-yl)benzyl)cyclopropanesulfonamide

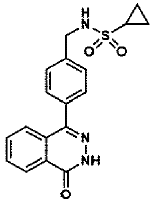

¹H NMR (400 MHz, MeOD) δ 8.52 - 8.44 (m, 1H), 8.00 - 7.88 (m, 2H), 7.85 - 7.78 (m, 1H), 7.62 (s, 4H), 4.44 (s, 2H), 2.57 - 2.48 (m, 1H), 1.13 - 1.05 (m, 2H), 1.05 - 0.94 (m, 2H).

### [Example 197]: 4-methyl-N-(4-(4-oxo-3,4-dihydrophthalazin-1-yl)benzyl)benzenesulfonamide

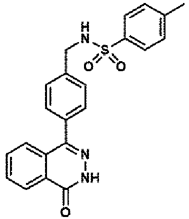

¹H NMR (400 MHz, MeOD) δ 8.51 - 8.41 (m, 1H), 7.96 - 7.89 (m, 2H), 7.80 - 7.70 (m, 3H), 7.51 (d, *J* = 7.9 Hz, 2H), 7.43 (d, *J* = 8.5 Hz, 2H), 7.39 (d, *J* = 7.8 Hz, 2H), 4.20 (s, 2H), 2.44 (s, 3H).

### [Example 198]: N-(4-(4-oxo-3,4-dihydrophthalazin-1-yl)benzyl)thiophene-2-sulfonamide

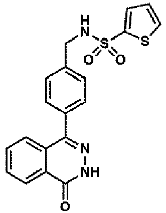

¹H NMR (400 MHz, MeOD) δ 8.47 - 8.41 (m, 1H), 7.93 - 7.85 (m, 2H), 7.80 - 7.71 (m, 2H), 7.61 (dd, *J* = 3.7, 1.3 Hz, 1H), 7.52 (d, *J* = 8.2 Hz, 2H), 7.45 (d, *J* = 8.3 Hz, 2H), 7.16 - 7.11 (m, 1H), 4.26 (s, 2H).

### [Preparation Example 4-2]

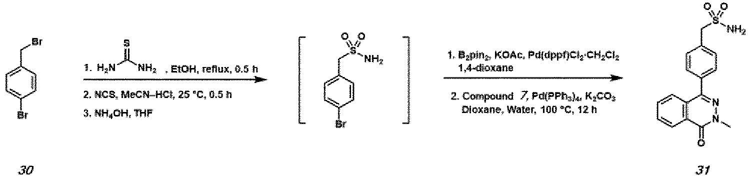

**Step 1-1**: 4-bromobenzyl bromide ***30*** (1.0 eq.) and thiourea (1.0 eq.) were refluxed in ethanol (1.0 M). The ethanol was removed by low pressure to obtain a solid or viscous oil and then the following reaction was performed without further purification.

**Step 1-2**: The product obtained in Step 1-1 was slowly added to a solution of *N*-chlorosuccinimide (5.0 eq.), 2M HCl (3.1 M), and acetonitrile (0.63 M). The internal temperature of the solution was maintained at 10°C to 20°C in a water bath. After the reaction was complete, diethyl ether was added thereto and the layers of the solution were separated by water addition. The organic layer was separated, dried with anhydrous sodium sulfate, concentrated under reduced pressure and separated by column chromatography to obtain a product.

**Step 1-3**: (4-bromophenyl)methane sulfonylchloride (1.0 eq.) was dissolved in tetrahydrofuran (0.1 M) and then a 25% ammonium hydroxide solution was added thereto at 0°C. The reaction mixture was stirred at 5°C for 1 hour. The reaction mixture was concentrated and the precipitate was washed with water and filtered to obtain a product (64° yield) .

**Step 2-1:** the product (1.0 eq.) of Step 1-3, B₂pin₂ (1.2 eq.) and potassium acetate (3.0 eq.) were dissolved in anhydrous dioxane (0.15 M). The mixture was stirred with nitrogen bubbling for 20 minutes. Then, [1,1'-bis(diphenylphosphino)ferrocene]palladium (II) dichloride (0.1 eq.) was added thereto and a reflux condenser was attached. The reaction mixture was stirred under a nitrogen atmosphere at 65°C for 18 hours. Then, the solution was warmed to 23°C, concentrated and separated by column chromatography to obtain a product (74% yield).

**Step 2-2:** the product obtained in Step 2-1, **7** (1.0 eq.), tetrakis (triphenylphosphine) palladium (0) (0.06 eq.), **8** (1.25 eq.) and potassium carbonate (3.75 eq.) were dissolved in 1,4-dioxane (0.09 M) and water (0.9 M) in a round bottom flask, followed by stirring under a nitrogen atmosphere for 4 hours. After the reaction was complete, the mixture was poured into water, followed by extraction with ethyl acetate. The organic layer was dried over anhydrous magnesium sulfate and concentrated under reduced pressure. The obtained residue was separated by column chromatography (EtOAc : Hexane = 1: 1) to obtain ***31*** (70% yield).

### [Example 199]: (4-(3-methyl-4-oxo-3,4-dihydrophthalazin-1-yl)phenyl)sulfamide

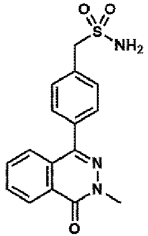

¹H NMR (400 MHz, CDCl3) δ 8.55 (d, *J* = 7.6 Hz, 1H), 7.85 - 7.67 (m, 3H), 7.64 (s, 4H), 4.53 (s, 2H), 4.44 (s, 2H), 3.93 (s, 3H).

### [Preparation Example 4-3]

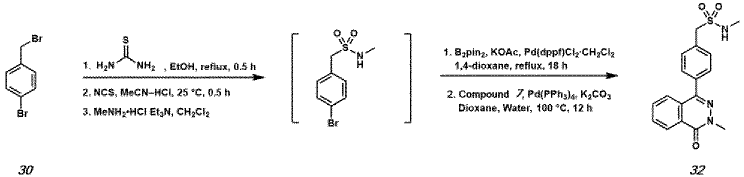

**Step 1-1**: a product was obtained in the same manner as in Step 1-1 of **[Preparation Example 4-2]**.

**Step 1-2**: a product was obtained in the same manner as in Step 1-2 of **[Preparation Example 4-2]**.

**Step 1-3**: the product of Step 1-2 (1.0 eq.), tetrahydrofuran (0.19M), and tert-butanol (0.37M) were stirred in a 40° aqueous methylamine solution (6.8 eq.) at room temperature for 2 hours. A saturated aqueous sodium hydrogen carbonate solution was added to the reaction mixture and then the organic layer was separated with ethyl acetate. The separated organic layer was washed with brine and water, dried with anhydrous sodium sulfate and concentrated under reduced pressure to obtain a product (89% yield) without further purification.

**Step 2-1:** a product (89% yield) was obtained in the same manner as in Step 2-1 of **[Preparation Example 4-2].**

**Step 2-2:** a product ***32*** (15% yield) was obtained in the same manner as in Step 2-2 of **[Preparation Example 4-2].**

### [Example 200]: N -methyl-1-(4-(3-methyl-4-oxo-3,4-dihydrophthalazin-1-yl)phenyl)methanesulfonamide

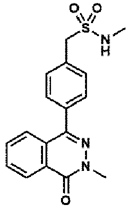

¹H NMR (400 MHz, CDCl3) δ 8.55 (d, *J* = 7.7 Hz, 1H), 7.83 - 7.68 (m, 3H), 7.67 - 7.55 (m, 5H), 4.36 (s, 2H), 3.93 (s, 3H), 2.82 (d, *J* = 5.2 Hz, 3H).

### [Preparation Example 4-4]

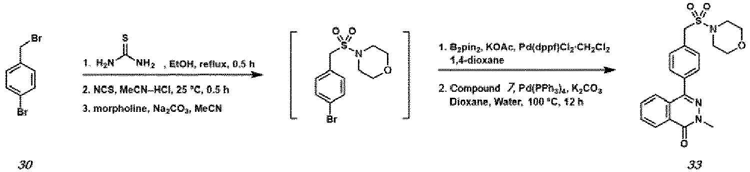

**Step 1-1:** a product was obtained in the same manner as in Step 1-1 of **[Preparation Example 4-2].**

**Step 1-2:** a product was obtained in the same manner as in Step 1-2 of **[Preparation Example 4-2].**

**Step 1-3:** sodium carbonate (2.0 eq.) was dissolved in acetonitrile (0.21M) and then morpholine (2.0 eq.) was added to the reaction mixture. Then, the solid (4-bromophenyl)-methanesulfonyl chloride (1.0 eq.) was added thereto and a flask was stirred under a nitrogen atmosphere for 2 hours. After the reaction was complete, the reaction mixture was diluted with distilled water and the aqueous solution was extracted with ethyl acetate. The organic layer was dried over sodium sulfate, filtered and concentrated to obtain a white solid without further purification (96% yield).

**Step 2-1:** a product (89% yield) was obtained in the same manner as in Step 2-1 of **[Preparation Example 4-2].**

**Step 2-2:** a product ***33*** (65% yield) was obtained in the same manner as in Step 2-2 of **[Preparation Example 4-2].**

### [Example 201]: 2-methyl-4-(4-((morpholinosulfonyl)methyl)phenyl)phthalazin-1(2H)-one

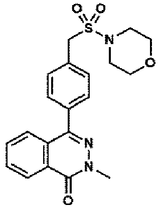

¹H NMR (400 MHz, DMSO) δ 8.41 - 8.34 (m, 1H), 7.95 - 7.85 (m, 2H), 7.70 - 7.58 (m, 5H), 4.57 (s, 2H), 3.79 (s, 3H), 3.66 - 3.59 (m, 4H), 3.21 - 3.14 (m, 4H).

### [Preparation Example 4-5]

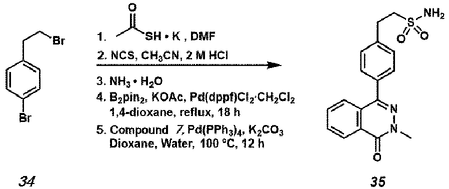

**Step 1:** potassium thioacetate (1.1 eq.) was dissolved at 0°C in dimethylformamide (0.3 M) and then bromide ***34*** (1.0 eq.) was slowly added thereto. The mixture was stirred at 25°C for 2 hours. After the reaction was complete, the mixture was poured into ethyl acetate and washed with brine. Then, the organic layer was dried with anhydrous sodium sulfate and concentrated under reduced pressure to obtain a product without further purification, which was used for the following reaction.

**Step 2:** N-chlorosuccinimide (4.0 eq.) was added to a solution of acetonitrile (0.2 M)/2M HCl (5/1 v/v) and the mixture was warmed to 0°C. Then, thioacetate (1.0 eq.) was slowly added thereto. The mixture was stirred for 1 hour. After the reaction was complete, the resulting mixture was extracted with diethyl ether and washed with brine, the organic layer was dried with anhydrous sodium sulfate and then concentrated under reduced pressure to obtain a product (73% yield).

**Step 3:** (4-bromophenyl)methane sulfonylchloride (1.0 eq.) was dissolved in tetrahydrofuran (0.1 M) and then a 25% ammonium hydroxide solution was added thereto at 0°C. The reaction mixture was stirred at 5°C for 1 hour. The reaction mixture was concentrated and then the precipitate was washed with water and filtered to obtain a product (82% yield).

**Step 4:** a product (74% yield) was obtained in the same manner as in Step 2-1 of **[Preparation Example 4-2].**

**Step 5:** a product ***35*** (70° yield) was obtained in the same manner as in Step 2-2 of **[Preparation Example 4-2].**

### [Example 202]: 2-(4-(3-methyl-4-oxo-3,4-dihydrophthalazin-1-yl)phenyl)ethane-1-sulfonamide

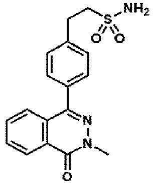

¹H NMR (400 MHz, CDCl3) δ 8.57 - 8.49 (m, 1H), 7.84 - 7.64 (m, 3H), 7.60 - 7.55 (m, 2H), 7.41 (d, *J* = 8.1 Hz, 2H), 4.57 (s, 2H), 3.92 (s, 3H), 3.50 - 3.42 (m, 2H), 3.32 - 3.24 (m, 2H).

### [Example 203]: 4-(4-(aminomethyl)-3-(trifluoromethyl)phenyl-6,7-dimethoxy phthalazin-1(2H)-one hydrochloride

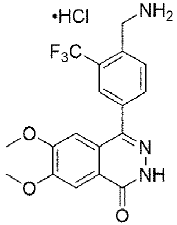

LCMS [M+H]⁺ 380.2

### [Experimental example]

Examples 1 to 203 prepared as described above were subjected to the following experiment.

### Experimental example 1: ENPP1 enzyme assay with cGAMP substrate

ENPP1 hydrolyzes nucleotides or nucleotide derivatives to produce nucleoside -5'-monophosphate and pyrophosphate. In addition, ENPP1 hydrolyzes 2',3'-cGAMP to produce 5'-adenosine monophosphate (AMP) and 5'-guanosine monophosphate (GMP). AMP produced from the reaction is measured using the AMP-Glo^{®} kit (Promega). The AMP-Glo^{®} kit contains two reagents. The first reagent terminates the AMP-producing enzymatic reaction, removes ATP and converts the produced AMP into ADP. The second reagent converts ADP to ATP which is used to generate luminescence in the luciferase reaction. The amount of luminescence measured is proportional to the amount of AMP produced by ENPP1.

The final reaction mixture contains 50 mM Tris (pH 8.5) buffer, 250 mM NaCl, 0.5 mM CaCl₂, 1 µM ZnCl₂, 5% glycerol and 1% DMSO. Serially diluted ENPP1 inhibitors (typically in the range of 10 µM to 0.5 nM) were pre-stored with human recombinant ENPP1 enzymes (R & D systems) at 3 ng/reaction and room temperature (RT) for 5 to 10 minutes. The reaction was initiated by addition of cGAMP (at a final concentration of 5 µM) and reacted at 37°C for 90 minutes. At the end of the reaction, 10 µl of the AMP-Glo first reagent was added to stop the reaction and the result was stored at room temperature for 1 hour. After storage, 20 µl of an AMP detection solution (a mixture of AMP-Glo II reagent and Kinase-Glo at a ratio of 1:100) was added and stored at room temperature for 1 hour. Luminescence signals were measured using a Victor^{®} plate reader (Perkin Elmer). The maximal activity control (containing enzymes and substrates only in the presence of 1% DMSO; MAX) and the minimal activity control (containing substrates and 1% DMSO; MIN) were evaluated simultaneously. In each experiment, serially diluted reference ENPP1 inhibitors were tested together. IC₅₀, indicating % residual activity versus compound concentration was determined by fitting inhibition curves using the 3-parameter analysis of GraphPad Prism^{®} software. Serially diluted samples of one compound were tested two or more times and an average IC₅₀ of each compound was calculated.

The experimental results are shown in Tables 1 and 2 below.

**[Table 1]**

| Example | Enzymatic activity |
|---|---|
| 1 | B |
| 2 | NA |
| 3 | NA |
| 4 | NA |
| 5 | C |
| 6 | NA |
| 9 | NA |
| 10 | NA |
| 12 | NA |
| 13 | C |
| 14 | C |
| 15 | NA |
| 18 | C |
| 20 | NA |
| 24 | NA |
| 26 | NA |
| 27 | NA |
| 28 | NA |
| 29 | C |
| 132 | NA |
| 133 | NA |
| 145 | NA |
| 146 | NA |
| 147 | NA |
| 148 | NA |
| 149 | NA |
| 150 | NA |
| 151 | NA |
| 152 | NA |
| 155 | NA |
| 30 | C |
| 31 | C |
| 32 | C |
| 33 | C |
| 35 | C |
| 37 | A |
| 38 | A |
| 39 | C |
| 40 | B |
| 41 | B |
| 42 | B |
| 43 | A |
| 44 | B |
| 46 | C |
| 47 | A |
| 48 | A |
| 49 | A |
| 51 | A |
| 52 | B |
| 53 | A |
| 55 | A |
| 56 | B |
| 57 | B |
| 58 | B |
| 59 | NA |
| 134 | B |
| 135 | C |
| 156 | NA |
| 157 | NA |
| 158 | NA |
| 159 | NA |
| 160 | NA |
| 161 | C |
| 162 | C |
| 163 | C |
| 164 | C |
| 165 | C |
| 166 | C |
| 60 | C |
| 61 | C |
| 62 | NA |
| 64 | C |
| 66 | C |
| 67 | C |
| 69 | NA |
| 70 | NA |
| 71 | C |
| 72 | C |
| 73 | NA |
| 76 | B |
| 77 | B |
| 81 | NA |
| 83 | NA |
| 84 | C |
| 85 | C |
| 136 | NA |
| 137 | NA |
| 167 | NA |
| 168 | NA |
| 169 | B |
| 170 | C |
| 171 | C |
| 172 | B |
| 173 | NA |
| 174 | NA |
| 176 | C |
| 177 | C |
| 87 | A |
| 88 | A |
| 89 | A |
| 91 | B |
| 92 | A |
| 93 | C |
| 95 | A |
| 90 | C |
| 94 | A |
| 96 | A |
| 97 | A |
| 98 | B |
| 99 | NA |
| 100 | A |
| 101 | B |
| 102 | B |
| 103 | A |
| 104 | A |
| 105 | B |
| 106 | B |
| 107 | C |
| 108 | B |
| 109 | B |
| 110 | B |
| 111 | A |
| 112 | B |
| 113 | B |
| 114 | B |
| 115 | B |
| 116 | B |
| 117 | C |
| 118 | C |
| 119 | B |
| 120 | C |
| 121 | A |
| 122 | A |
| 123 | A |
| 124 | A |
| 125 | B |
| 126 | A |
| 127 | A |
| 128 | B |
| 129 | B |
| 130 | B |
| 131 | B |
| 138 | A |
| 139 | A |
| 140 | C |
| 141 | B |
| 142 | C |
| 143 | C |
| 144 | C |
| 178 | C |
| 179 | C |
| 180 | A |
| 181 | A |
| 182 | A |
| 183 | A |
| 184 | B |
| 185 | A |
| 186 | A |
| 187 | A |
| 188 | A |
| 189 | A |
| 190 | A |
| 191 | A |
| 192 | A |
| 193 | NA |
| 194 | C |
| 195 | C |
| 196 | C |
| 197 | C |
| 198 | NA |
| 199 | NA |
| 200 | NA |
| 201 | NA |
| 202 | B |
| 203 | C |

**[Table 2]**

| **Enzymatic activity** | **A** | **B** | **C** | **NA** |
|---|---|---|---|---|
| IC50 (µM) | activity < 1 µM | 1 µM < activity <10 µM | activity > 10 µM | No activity |

### [Formulation Example]

Meanwhile, the novel compound represented by Formula 1 according to the present invention may be formulated in various forms according to purpose. Examples of some formulation methods for incorporating the compound represented by Formula 1 according to the present invention as an active ingredient are as follows, but the present invention is not limited thereto.

### Formulation Example 1: Tablet (direct pressurization)

5.0 mg of the active ingredient was sieved, 14.1 mg of lactose, 0.8 mg of crospovidone USNF, and 0.1 mg of magnesium stearate were mixed therewith, and the mixture was compressed into tablets.

### Formulation Example 2: Tablet (wet granulation)

5.0 mg of the active ingredient was sieved and mixed with 16.0 mg of lactose and 4.0 mg of starch. 0.3 mg of Polysorbate 80 was dissolved in pure water, and an appropriate amount of the resulting solution was added to the resulting mixture, followed by granulation. The granules were dried, sieved, and mixed with 2.7 mg of colloidal silicon dioxide and 2.0 mg of magnesium stearate. The granules were compressed into tablets.

### Formulation Example 3: Powders and capsules

5.0 mg of the active ingredient was sieved and then mixed with 14.8 mg of lactose, 10.0 mg of polyvinylpyrrolidone, and 0.2 mg of magnesium stearate. Hard No. 5 gelatin capsules were filled with the resulting mixture using an appropriate device.

### Formulation Example 4: Injection

Injections were prepared by incorporating 100 mg of the active ingredient as well as 180 mg of mannitol, 26 mg of Na₂HPO₄ · 12H₂O, and 2,974 mg of distilled water.

Although embodiments of the present invention have been described above, it will be obvious to those skilled in the art that the present invention can be implemented in other specific embodiments without changing the technical concepts or essential features of the present invention. Therefore, it should be construed that the aforementioned embodiments are illustrative and not restrictive in all respects.

## Claims

1. A compound selected from a phthalazine derivative compound represented by the following Formula 1, a pharmaceutically acceptable salt thereof, a hydrate thereof, a solvate thereof and a stereoisomer thereof: 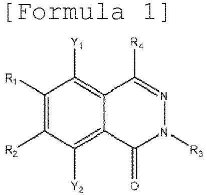 wherein
Y₁ and Y₂ are each independently hydrogen, a hydroxyl group, a halogen group, a C₁-C₁₃ alkyl group, a C₃-C₁₀ cyclic group, a C₁-C₆ alkoxy group, a C₃-C₁₀ cyclic group, or a C₃-C₁₀ heterocyclic group;
R₁ and R₂ are each independently hydrogen, a hydroxyl group, a halogen group, a C₁-C₁₃ alkyl group, a C₁-C₆ alkoxy group, a C₆-C₁₀ aryl group, a C₃-C₁₀ cyclic group, a C₃-C₁₀ heteroaryl group, a C₃-C₁₀ heterocyclic group, or -C(O)-(C₁-C₁₃ alkyl), or R₁, together with a nitrogen atom bonded to R₂, forms a 4- to 7-membered saturated, unsaturated or aromatic ring, which includes at least one of N, O, S, NH, C=N, C=O, -NHC(O)-, -NHC(O)NH-, -NHS(O)₂-, or SO₂, and is substituted with at least one of a C₁-C₁₃ alkyl group, a C₆-C₁₀ aryl group, a C₃-C₁₀ heteroaryl group, a hydroxyl group, a halide group or a cyano group;
R₃ is hydrogen, a hydroxyl group, a halogen group, a C₁-C₁₃ alkyl group, a C₁-C₆ alkoxy group, a C₆-C₁₀ aryl group, a C₃-C₁₀ cyclic group, a C₃-C₁₀ heteroaryl group, a C₃-C₁₀ heterocyclic group, or -C(O)-(C₁-C₁₃ alkyl);
R₄ is hydrogen, a hydroxyl group, a halogen group, a C,_-C₁₃ alkyl group, a C₁-C₆ alkoxy group, a C₁-C₆ alkenyl group, a C₆-C₁₀ aryl group, a C₃-C₁₀ cyclic group, a C₃-C₁₀ heteroaryl group, a C₃-C₁₀ heterocyclic group, an amino group, a nitro group, an amide group, a carboxylic group, a nitrile group, a urea group, a sulfonamide group, a sulfide group, a sulfonic group, or a phosphoryl group,
wherein the C₁-C ₆ alkyl group, the C₁-C₁₃ alkyl group or the C₃-C₁₀ cyclic group includes at least one substituent selected from the group consisting of hydrogen, a hydroxyl group, a halogen group, a C₁-C₁₃ alkyl group, a C₁-C₆ alkoxy group, an amino group (-NR₅R₆), a nitro group (-N(O)₂), an amide group (-(C=O)NR₅R₆), a carboxylic group (-C(O)OH), a nitrile group (-CN), a urea group (-NR₅(C=O)NR₆-), a sulfonamide group (-NHS(O)₂-), a sulfide group (-S-), a sulfonyl group (-S(O)₂-), a phosphoryl group (-P(O)R₅R₆), a C₆-C₁₀ aryl group, a C₃-C₁₀ heteroaryl group, and a C₃-C₁₀ heterocyclic group, and
the C₆-C₁₀ aryl group, the C₃-C₁₀ heteroaryl group or the C₃-C₁₀ heterocyclic group includes at least one substituent selected from the group consisting of hydrogen, a hydroxyl group, a halogen group, a carbonyl group (-(C=O)R₅R₆), a C₁-C₃ alkyl group substituted or unsubstituted with halogen or a C₃-C₁₀ heterocyclic group, a C₁-C₃ alkoxy group substituted or unsubstituted with halogen or a C₃-C₁₀ heterocyclic group, C₆-C₁₀ phenoxy, an amino group (-NR₅R₆), a nitro group (-N(O)₂), an amide group (-(C=O)NR₅R₆), a carboxylic group (-C(O)OH), a nitrile group (-CN), a urea group (-NR₅(C=O)NR₆-), a sulfonamide group (-NHS(O)₂-), a sulfide group (-S-), a sulfonyl group (-S(O)₂-), a phosphoryl group (-P(O)R₅R₆), a C₆-C₁₀ aryl group, a C₃-C₁₀ heteroaryl group, and a C₃-C₁₀ heterocyclic group; and
R₅ and R₆ are each independently hydrogen, a C₁-C₆ alkyl group, a C₁-C₆ alkenyl group, a C₁-C₆ alkynyl group, a C₆-C₁₀ aryl group, a C₃-C₁₀ heteroaryl group, a C₃-C₁₀ heterocyclic group, or R₅, together with a nitrogen or carbon atom bonded to R₆, forms a 3- to 7-membered saturated ring, which includes at least one of N, O, S, NH, C=N, C=O, -NHC(O)-, -NHC(O)NH-, -NHS(O)₂-, or SO₂, and is substituted with at least one of hydrogen, a C₁-C₁₃ alkyl group, a C₆-C₁₀ aryl group, a C₃-C₁₀ heteroaryl group, a hydroxyl group, a halide group or a cyano group, wherein the C₃-C₁₀ heteroaryl group and the C₃-C₁₀ heterocyclic group include at least one heteroatom selected from the group consisting of N, O, and S .

2. The compound according to claim 1, wherein Y₁ and Y₂ are each independently hydrogen or a C₁-C₁₃ alkyl group,
R₁ and R₂ are each independently hydrogen, a hydroxyl group, a C₁-C₁₃ alkyl group, or a C₁-C₆ alkoxy group,
R₃ is hydrogen, a C₁-C₁₃ alkyl group, or a C₆-C₁₀ aryl group, and
R₄ is a C₁-C₁₃ alkyl group, a C₆-C₁₀ aryl group, a C₃-C₁₀ cyclic group, a C₃-C₁₀ heteroaryl group, a C₃-C₁₀ heterocyclic group, an amino group, a nitro group, an amide group, a carboxylic group, a nitrile group, a urea group, a sulfonamide group, a sulfide group, a sulfonic group, or a phosphoryl group.

3. The compound according to claim 1, wherein Y₁ and Y₂ are each hydrogen,
R₁ and R₂ are each independently hydrogen, a C₁-C₄ alkyl group, or a C₁-C₄ alkoxy group,
R₃ is hydrogen, a C₁-C₄ alkyl group, or a C₆-C₁₀ aryl group, and
R₄ is a substituted or unsubstituted C₁-C₆ alkyl group, substituted or unsubstituted benzene, substituted or unsubstituted hexane, substituted or unsubstituted furan, substituted or unsubstituted thiophene, substituted or unsubstituted pyridine, substituted or unsubstituted benzofuran, substituted or unsubstituted naphthalene, substituted or unsubstituted anthracene, substituted or unsubstituted phenanthrene, substituted or unsubstituted pyridazine, substituted or unsubstituted piperidine, substituted or unsubstituted morpholine, substituted or unsubstituted pyrrolidine, substituted or unsubstituted pyrazine, substituted or unsubstituted imidazole, substituted or unsubstituted pyrazole, substituted or unsubstituted quinoline, substituted or unsubstituted pyrimidine, substituted or unsubstituted pyrrole, substituted or unsubstituted indole, substituted or unsubstituted purine, substituted or unsubstituted cyclopropane, or substituted or unsubstituted cyclobutane.

4. The compound according to claim 1, wherein Y₁ and Y₂ are each hydrogen,
R₁ and R₂ are each independently hydrogen or a methoxy group,
R₃ is hydrogen, a C₁-C₄ alkyl group, a phenyl group, or a benzyl group, and
R₄ is a substituted or unsubstituted phenyl group, a substituted or unsubstituted benzyl group,

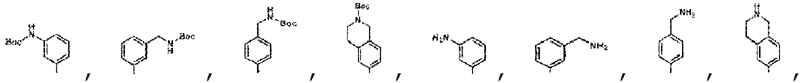

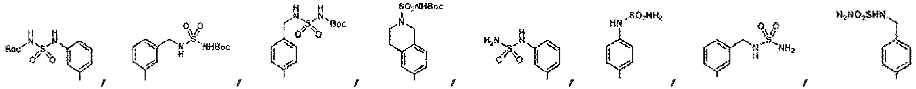

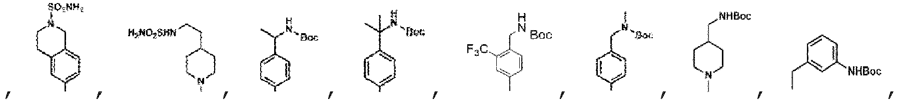

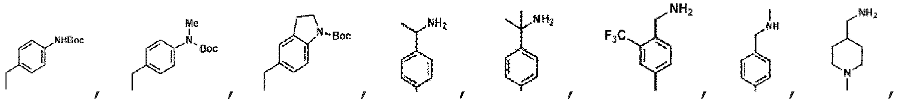

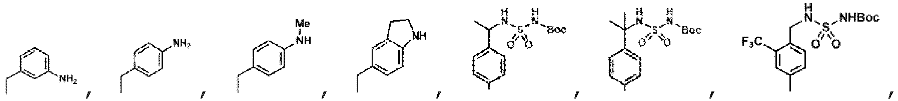

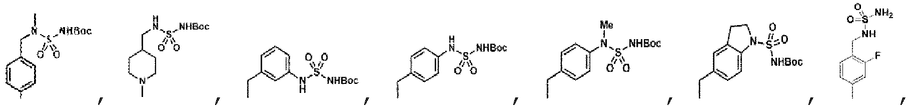

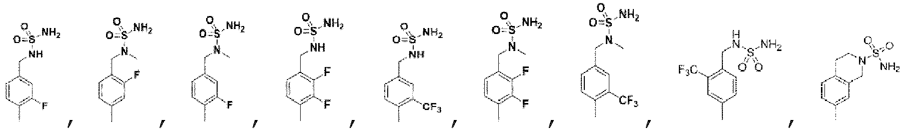

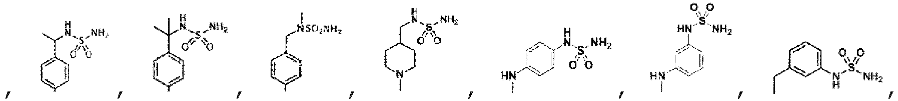

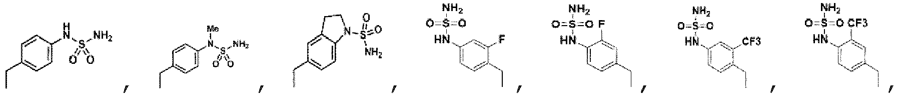

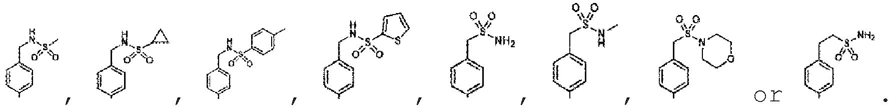

5. The compound according to claim 1, wherein the compound represented by Formula 1 is selected from the group consisting of the following Compounds Nos. 1 to 203:
Compound No. 1: 4-phenylphthalazin-1(2H)-one;
Compound No. 2: tert-butyl (3-(4-oxo-3,4-dihydrophthalazin-1-yl)phenyl)carbamate;
Compound No. 3: tert-butyl (3(3-methyl-4-oxo-3,4-dihydrophthalazin-1-yl)phenyl)carbamate;
Compound No. 4: tert-butyl (3(3-benzyl-4-oxo-3,4-dihydrophthalazin-1-yl)phenyl)carbamate;
Compound No. 5: tert-butyl(3-(4-oxo-3,4-dihydrophthalazin-1-yl)carbamate;
Compound No. 6: tert-butyl 3-(3-methyl-4-oxo-3,4-dihydrophthalazin-1-yl)benzyl carbamate;
Compound No. 7: tert-butyl (3-(3-benzyl-4-oxo-3,4-dihydrophthalazin-1-yl)benzyl)carbamate;
Compound No. 8: tert-butyl (4-(4-oxo-3,4-dihydrophthalazin-1-yl)benzyl)carbamate;
Compound No. 9: tert-butyl (4-3-methyl-4-oxo-3,4-dihydrophthalazin-1-yl)benzyl)carbamate;
Compound No. 10: tert-butyl (4-(3-ethyl-4-oxo-3,4-dihydrophthalazin-1-yl)benzyl)carbamate;
Compound No. 11: tert-butyl 4-(3-isopropyl-4-oxo-3,4-dihydrophthalazin-1-yl)benzyl carbamate;
Compound No. 12: tert-butyl (4-(4-oxo-3-phenyl-3,4-dihydrophthalazin-1-yl)benzyl)carbamate;
Compound No. 13: tert-butyl (4-3-benzyl-4-oxo-3,4-dihydrophthalazin-1-yl)benzyl)carbamate;
Compound No. 14: tert-butyl (4-(3-(4-fluorobenzyl)-4-oxo-3,4-dihydrophthalazin-1-yl)benzyl)carbamate;
Compound No. 15: tert-butyl (4-(7-methoxy-4-oxo-3,4-dihydrophthalazin-1-yl)benzyl)carbamate;
Compound No. 16: tert-butyl 4-(7-methoxy-3-methyl-4-oxo-3,4-dihydrophthalazin-1-yl)benzyl carbamate;
Compound No. 17: tert-butyl 4-(6-methoxy-4-oxo-3,4-dihydrophthalazin-1-yl)benzyl carbamate;
Compound No. 18: tert-butyl (4-(6-methoxy-3-methyl-4-oxo-3,4-dihydrophthalazin-1-yl)benzyl)carbamate;
Compound No. 19: tert-butyl (4-(6-methoxy-4-oxo-3,4-dihydrophthalazin-1-yl)benzyl)carbamate;
Compound No. 20: tert-butyl (4-(6,7-dimethoxy-3-methyl-4-oxo-3,4-dihydrophthalazin-1-yl)benzyl)carbamate;
Compound No. 21: tert-butyl (1-(4-(4-oxo-3,4-dihydrophthalazin-1-yl)phenyl)ethyl)carbamate;
Compound No. 22: tert-butyl (R)-(1-(4-(4-oxo-3,4-dihydrophthalazin-1-yl)phenyl)ethyl)carbamate;
Compound No. 23: tert-butyl (2-(4-(4-oxo-3,4-dihydrophthalazin-1-yl)phenyl)propan-2-yl)carbamate;
Compound No. 24: tert-butyl 4-(4-oxo-3,4-dihydrophthalazin-1-yl)-2-(trifluoromethyl)benzyl carbamate;
Compound No. 25: tert-butyl methyl(4-(4-oxo-3,4-dihydrophthalazin-1-yl)benzyl)carbamate;
Compound No. 26: tert-butyl 6-(4-oxo-3,4-dihydrophthalazin-1-yl)-3,4-dihydroisoquinoline-2(1H)-carboxylate;
Compound No. 27: tert-butyl-6-(3-methyl-4-oxo-3,4-dihydrophthalazin-1-yl)-3,4-dihydroisoquinoline-2(1H)-carboxylate;
Compound No. 28: tert-butyl 6-(6,7-dimethoxy-4-oxo-3,4-dihydrophthalazin-1-yl)-3,4-dihydroisoquinoline-2(1H)-carboxylate;
Compound No. 29: tert-butyl-6-(6,7-dimethoxy-3-methyl-4-oxo-3,4-dihydrophthalazin-1-yl)-3,4-dihydroisoquinoline-2(1H)-carboxylate;
Compound No. 30: 4-(3-aminophenyl)phthalazin-1(2H)-one hydrochloride;
Compound No. 31: 4-(3-aminophenyl)-2-methylphthalazin-1(2H)-one hydrochloride;
Compound No. 32: 4-(3-aminophenyl)-2-benzylphthalazin-1(2H)-one hydrochloride;
Compound No. 33: 4-(3-(aminomethyl)phenyl)phthalazin-1(2H)-one hydrochloride;
Compound No. 34: 4-(3-(aminomethyl)phenyl)-2-methylphthalazin-1-(2H)-one hydrochloride;
Compound No. 35: 4-(3-(aminomethyl)phenyl)-2-benzylphthalazin-1(2H)-one hydrochloride;
Compound No. 36: 4-(4-(aminomethyl)phenyl)phthalazin-1(2H)-one hydrochloride;
Compound No. 37: 4-(4-aminomethyl)phenyl)-2-methylphthalazin-1(2H)-one hydrochloride;
Compound No. 38: 4-(4-(aminomethyl)phenyl)-2-ethylphthalazin-1(2H)-one hydrochloride;
Compound No. 39: 4-(4-(aminomethyl)phenyl)-2-isopropylphthalazin-1-(2H)-one hydrochloride;
Compound No. 40: 4-(4-aminomethyl)phenyl)-2-phenylphthalazin-1(2H)-one hydrochloride;
Compound No. 41: 4-(4-aminomethyl)phenyl)-2-benzylphthalazin-1(2H)-one hydrochloride;
Compound No. 42: 4-(4-(aminomethyl)phenyl)-2-(4-fluorobenzyl)phthalazin-1(2H)-one hydrochloride;
Compound No. 43: 4-(4-(aminomethyl)phenyl)-6-methoxyphthalazin-1(2H)-one hydrochloride;
Compound No. 44: (4-(7-methoxy-3-methyl-4-oxo-3,4-dihydrophthalazin-1-yl)phenyl)methanaminium chloride;
Compound No. 45: (4-(6-methoxy-4-oxo-3,4-dihydrophthalazin-1-yl)phenyl)methanaminium chloride;
Compound No. 46: 4-(4-(aminomethyl)phenyl)-7-methoxy-2-methylphthalazin-1(2H)-one hydrochloride;
Compound No. 47: 4-(4-(aminomethyl)-phenyl)-7-methoxyphthalazin-1(2H)-one hydrochloride;
Compound No. 48: 4-(4-(aminomethyl)phenyl)-6,7-dimethoxyphthalazin-1(2H)-one hydrochloride;
Compound No. 49: 4-(4-(aminomethyl)phenyl)-6,7-dimethoxy-2-methylphthalazin-1(2H)-one hydrochloride;
Compound No. 50: 4-(4-(1-aminoethyl)phenyl)phthalazin-1(2H)-one hydrochloride;
Compound No. 51: (R)-4-(4-(1-aminoethyl)phenyl)phthalazin-1(2H)-one hydrochloride;
Compound No. 52: 4-(4-(2-aminopropan-2-yl)phenyl)phthalazin-1(2H)-one hydrochloride;
Compound No. 53: 4-(4-(aminomethyl)-3-(trifluoromethyl)phenyl)phthalazin-1(2H)-one hydrochloride;
Compound No. 54: 4-(4-((methylamino)methyl)phenyl)phthalazin-1(2H)-one hydrochloride;
Compound No. 55: 4-(1,2,3,4-tetrahydroisoquinolin-6-yl)phthalazin-1(2H)-one hydrochloride;
Compound No. 56: 2-methyl-4-(1,2,3,4-tetrahydroisoquinolin-6-yl)phthalazin-1(2H)-one hydrochloride;
Compound No. 57: 6,7-dimethoxy-4-(1,2,3,4-tetrahydroisoquinolin-6-yl)phthalazin-1(2H)-one hydrochloride;
Compound No. 58: 6,7-dimethoxy-2-methyl-4-(1,2,3,4-tetrahydroisoquinolin-6-yl)phthalazin-1(2H)-one hydrochloride;
Compound No. 59: 6,7-dimethoxy-4-(1,2,3,4-tetrahydroisoquinolin-7-yl) phthalazin-1(2H)-one;
Compound No. 60: tert-butyl (N-(3-(4-oxo-3,4-dihydrophthalazin-1-yl)phenyl)sulfamoyl)carbamate;
Compound No. 61: tert-butyl (N-(3-(3-methyl-4-oxo-3,4-dihydrophthalazin-1-yl)phenyl)sulfamoyl)carbamate;
Compound No. 62: tert-butyl (N-(3-(3-benzyl-4-oxo-3,4-dihydrophthalazin-1-yl)phenyl)sulfamoyl)carbamate;
Compound No. 63: tert-butyl (N-(3-(4-oxo-3,4-dihydrophthalazin-1-yl)benzyl)sulfamoyl)carbamate;
Compound No. 64: tert-butyl N-(3-(3-methyl-4-oxo-3,4-dihydrophthalazin-1-yl)benzyl)sulfamoyl carbamate;
Compound No. 65: tert-butyl (N-(3-(3-benzyl-4-oxo-3,4-dihydrophthalazin-1-yl)benzyl)sulfamoyl)carbamate;
Compound No. 66: tert-butyl (N-(4-(3-methyl-4-oxo-3,4-dihydrophthalazin-1-yl)benzyl)sulfamoyl)carbamate;
Compound No. 67: tert-butyl (N-(4-(3-ethyl-4-oxo-3,4-dihydrophthalazin-1-yl)benzyl)sulfamoyl)carbamate;
Compound No. 68: tert-butyl N-(4-(3-isopropyl-4-oxo-3,4-dihydrophthalazin-1-yl)benzyl)sulfamoyl carbamate;
Compound No. 69: tert-butyl (N-(4-(4-oxo-3-phenyl-3,4-dihydrophthalazin-1-yl)benzyl)sulfamoyl)carbamate;
Compound No. 70: tert-butyl (N-(4-3-benzyl-4-oxo-3,4-dihydrophthalazin-1-yl)benzyl)sulfamoyl)carbamate;
Compound No. 71: tert-butyl (N-(4-(3-(4-fluorobenzyl)-4-oxo-3,4-dihydrophthalazin-1-yl)benzyl)sulfamoyl)carbamate;
Compound No. 72: tert-butyl (4-(7-methoxy-4-oxo-3,4-dihydrophthalazin-1-yl)benzyl)sulfamoyl)carbamate;
Compound No. 73: tert-butyl N-(4-(7-methoxy-3-methyl-4-oxo-3,4-dihydrophthalazin-1-yl)benzyl)sulfamoyl carbamate;
Compound No. 74: tert-butyl N-(4-(6-methoxy-4-oxo-3,4-dihydrophthalazin-1-yl)benzyl)sulfamoyl carbamate;
Compound No. 75: tert-butyl (N-(4-(6-methoxy-3-methyl-4-oxo-3,4-dihydrophthalazin-1-yl)benzyl)sulfamoyl)carbamate;
Compound No. 76: tert-butyl (N-(4-(6,7-dimethoxy-4-oxo-3,4-dihydrophthalazin-1-yl)benzyl)sulfamoyl)carbamate;
Compound No. 77: tert-butyl (N-(4-(6,7-dimethoxy-3-methyl-4-oxo-3,4-dihydrophthalazin-1-yl)benzyl)sulfamoyl)carbamate;
Compound No. 78: tert-butyl (N-(1-(4-(4-oxo-3,4-dihydrophthalazin-1-yl)phenyl)ethyl)sulfamoyl)carbamate;
Compound No. 79: tert-butyl (R)-(N-(1-(4-(4-oxo-3,4-dihydrophthalazin-1-yl)phenyl)ethyl)sulfamoyl)carbamate;
Compound No. 80: tert-butyl (N-(2-(4-(4-oxo-3,4-dihydrophthalazin-1-yl)phenyl)propan-2-yl)sulfamoyl)carbamate;
Compound No. 81: tert-butyl N-(4-(4-oxo-3,4-dihydrophthalazin-1-yl)-2-(trifluoromethyl)benzyl)sulfamoyl carbamate;
Compound No. 82: tert-butyl (N-methyl-N-(4-(4-oxo-3,4-dihydrophthalazin-1-yl)benzyl)sulfamoyl)carbamate;
Compound No. 83: tert-butyl ((6-(4-oxo-3,4-dihydrophthalazin-1-yl)-3,4-dihydroisoquinolin-2(1H)-yl)sulfonyl)carbamate;
Compound No. 84: tert-butyl ((6-(3-methyl-4-oxo-3,4-dihydrophthalazin-1-yl)-3,4-dihydroisoquinolin-2(1H)-yl)sulfonyl)carbamate;
Compound No. 85: tert-butyl ((6-(6,7-dimethoxy-4-oxo-3,4-dihydrophthalazin-1-yl)-3,4-dihydroisoquinolin-2(1H)-yl)sulfonyl)carbamate;
Compound No. 86: tert-butyl ((6-(6,7-dimethoxy-3-methyl-4-oxo-3,4-dihydrophthalazin-1-yl)-3,4-dihydroisoquinolin-2(1H)-yl)sulfonyl)carbamate;
Compound No. 87: N-(4-(4-oxo-3,4-dihydrophthalazin-1-yl)phenyl)sulfamide;
Compound No. 88: N-(3-(3-methyl-4-oxo-3,4-dihydrophthalazin-1-yl)phenyl)sulfamide hydrochloride;
Compound No. 89: N-(3-(3-benzyl-4-oxo-3,4-dihydrophthalazin-1-yl)phenyl)sulfamide hydrochloride;
Compound No. 90: N-(3-(3-benzyl-4-oxo-3,4-dihydrophthalazin-1-yl)benzyl)sulfamide hydrochloride;
Compound No. 91: N-(4-(4-oxo-3,4-dihydrophthalazin-1-yl)phenyl)sulfamide
Compound No. 92: N-(4-(3-methyl-4-oxo-3,4-dihydrophthalazin-1-yl)phenyl)sulfamide;
Compound No. 93: N-(4-(3-benzyl-4-oxo-3,4-dihydrophthalazin-1-yl)phenyl)sulfamide;
Compound No. 94: N-(3-(3-methyl-4-oxo-3,4-dihydrophthalazin-1-yl)benzyl)sulfamide trifluoroacetic acid;
Compound No. 95: N-(3-(3-oxo-3,4-dihydrophthalazin-1-yl)benzyl)sulfamide hydrochloride;
Compound No. 96: N-(4-(4-oxo-3,4-dihydrophthalazin-1-yl)benzyl) sulfamide hydrochloride;
Compound No. 97: N-(4-(3-methyl-4-oxo-3,4-dihydrophthalazin-1-yl)benzyl)sulfamide hydrochloride;
Compound No. 98: N-(4-(3-ethyl-4-oxo-3,4-dihydrophthalazin-1-yl)benzyl)sulfamide hydrochloride;
Compound No. 99: N-(4-(3-isopropyl-4-oxo-3,4-dihydrophthalazin-1-yl)benzyl)sulfamide trifluoroacetic acid;
Compound No. 100: N-(4-(4-oxo-3-phenyl-3,4-dihydrophthalazin-1-yl)benzyl)sulfamide hydrochloride;
Compound No. 101: N-(4-(3-benzyl-4-oxo-3,4-dihydrophthalazin-1-yl)benzyl)sulfamide hydrochloride;
Compound No. 102: N-(4-(3-(4-fluorobenzyl)-4-oxo-3,4-dihydrophthalazin-1-yl)benzyl)sulfamide hydrochloride;
Compound No. 103: N-(4-(7-methoxy-4-oxo-3,4-dihydrophthalazin-1-yl)benzyl)sulfamide hydrochloride;
Compound No. 104: N-(4-(6,7-dimethoxy-4-oxo-3,4-dihydrophthalazin-1-yl)benzyl)sulfamide hydrochloride;
Compound No. 105: N-(4-(7-methoxy-3-methyl-4-oxo-3,4-dihydrophthalazin-1-yl)benzyl)sulfamide hydrochloride;
Compound No. 106: N-(4-(6-methoxy-4-oxo-3,4-dihydrophthalazin-1-yl)benzyl)sulfamide hydrochloride;
Compound No. 107: N-(4-(6-methoxy-3-methyl-4-oxo-3,4-dihydrophthalazin-1-yl)benzyl)sulfamide hydrochloride;
Compound No. 108: N-(4-(6,7-dimethoxy-3-methyl-4-oxo-3,4-dihydrophthalazin-1-yl)benzyl)sulfamide hydrochloride;
Compound No. 109: N-(3-(6,7-dimethoxy-4-oxo-3,4-dihydrophthalazin-1-yl)-benzyl) sulfamide;
Compound No. 110: N-(3-(6,7-dimethoxy-3-methyl-4-oxo-3,4-dihydrophthalazin-1-yl)-benzyl) sulfamide;
Compound No. 111: N-(4-(6,7-dimethoxy-4-oxo-3,4-dihydrophthalazin-1-yl)-2-fluorobenzyl)sulfamide hydrochloride;
Compound No. 112: N-(4-(6,7-dimethoxy-4-oxo-3,4-dihydrophthalazin-1-yl)-3-fluorobenzyl)sulfamide hydrochloride;
Compound No. 113: N-(4-(6,7-dimethoxy-4-oxo-3,4-dihydrophthalazin-1-yl)-2-fluorobenzyl)-N-methyl sulfamide hydrochloride;
Compound No. 114: N-(4-(6,7-dimethoxy-4-oxo-3,4-dihydrophthalazin-1-yl)-3-fluorobenzyl)-N-methyl sulfamide hydrochloride;
Compound No. 115: N-(4-(6,7-dimethoxy-4-oxo-3,4-dihydrophthalazin-1-yl)-2,3-difluorobenzyl)sulfamide hydrochloride;
Compound No. 116: N-(4-(6,7-dimethoxy-4-oxo-3,4-dihydrophthalazin-1-yl)-3-(trifluorobenzyl methyl)benzyl) sulfamide hydrochloride;
Compound No. 117: N-(4-(6,7-dimethoxy-4-oxo-3,4-dihydrophthalazin-1-yl)-2,3-difluorobenzyl)-N-methyl sulfamide hydrochloride;
Compound No. 118: N-(4-(6,7-dimethoxy-4-oxo-3,4-dihydrophthalazin-1-yl)-3-(trifluorobenzyl methyl)benzyl)-N-methyl sulfamide hydrochloride;
Compound No. 119: N-(4-(6,7-dimethoxy-4-oxo-3,4-dihydrophthalazin-1-yl)-2-(trifluorobenzyl methyl)benzyl)sulfamide hydrochloride;
Compound No. 120: N-(4-(6,7-dimethoxy-4-oxo-3,4-dihydrophthalazin-1-yl)-2-(trifluorobenzyl methyl)benzyl)-N-methyl sulfamide hydrochloride;
Compound No. 121: 7-(4-oxo-3,4-dihydrophthalazin-1-yl)-3,4-dihydroisoquinoline-2(1H) sulfamide hydrochloride;
Compound No. 122: 7-(6,7-dimethoxy-4-oxo-3,4-dihydrophthalazin-1-yl)-3,4-dihydroisoquinoline-2(1H)-sulfamide;
Compound No. 123: N-(1-(4-(4-oxo-3,4-dihydrophthalazin-1-yl)phenyl)ethyl)sulfamide hydrochloride;
Compound No. 124: (R)-N-(1-(4-(4-oxo-3,4-dihydrophthalazin-1-yl)phenyl)ethyl)sulfamide hydrochloride;
Compound No. 125: N-(2-(4-(4-oxo-3,4-dihydrophthalazin-1-yl)phenyl)propan-2-yl)sulfamide hydrochloride;
Compound No. 126: N-(4-(4-oxo-3,4-dihydrophthalazin-1-yl)-2-(trifluoromethyl)benzyl)sulfamide hydrochloride;
Compound No. 127: N-methyl-N-(4-(4-oxo-3,4-dihydrophthalazin-1-yl)benzyl)sulfamide;
Compound No. 128: 6-(4-oxo-3,4-dihydrophthalazin-1-yl-3,4-dihydroisoquinoline-2(1H)-sulfonamide hydrochloride;
Compound No. 129: 6-(3-methyl-4-oxo-3,4-dihydrophthalazin-1-yl)-3,4-dihydroisoquinoline-2(1H)-sulfonamide hydrochloride;
Compound No. 130: 6-(6,7-dimethoxy-4-oxo-3,4-dihydrophthalazin-1-yl)-3,4-dihydroisoquinoline-2(1H)-sulfonamide hydrochloride;
Compound No. 131: 6-(6,7-dimethoxy-3-methyl-4-oxo-3,4-dihydrophthalazin-1-yl)-3,4-dihydroisoquinoline-2(1H)-sulfonamide hydrochloride;
Compound No. 132: tert-butyl ((1-(4-oxo-3,4-dihydrophthalazin-1-yl)piperidin-4-yl)methyl)carbamate;
Compound No. 133: tert-butyl ((1-(6,7-dimethoxy-4-oxo-3,4-dihydrophthalazin-1-yl)piperidin-4-yl)methyl)carbamate;
Compound No. 134: 4-(4-(aminomethyl)piperidin-1-yl)phthalazin-1(2H)-one hydrochloride;
Compound No. 135: 4-(4-(aminomethyl)piperidin-1-yl)-6,7-dimethoxyphthalazin-1(2H)-one hydrochloride;
Compound No. 136: tert-butyl N-((1-(4-oxo-3,4-dihydrophthalazin-1-yl)piperidin-4-yl)methyl)sulfamoyl carbamate;
Compound No. 137: tert-butyl N-((1-(6,7-dimethoxy-4-oxo-3,4-dihydrophthalazin-1-yl)piperidin-4-yl)methyl)sulfamoyl carbamate;
Compound No. 138: N-((1-(4-oxo-3,4-dihydrophthalazin-1-yl)piperidin-4-yl)methyl)sulfamide hydrochloride;
Compound No. 139: N-((1-(6,7-dimethoxy-4-oxo-3,4-dihydrophthalazin-1-yl)piperidin-4-yl)methyl)sulfamide hydrochloride;
Compound No. 140: N-(4-(6,7-dimethoxy-3-methyl-4-oxo-3,4-dihydrophthalazin-1-yl)-amino)phenyl)sulfamide hydrochloride;
Compound No. 141: N-(3-(6,7-dimethoxy-3-methyl-4-oxo-3,4-dihydrophthalazin-1-yl)-amino)phenyl)sulfamide hydrochloride;
Compound No. 142: N-(2-(1-(4-oxo-3,4-dihydrophthalazin-1-yl)piperidin-4-yl)ethyl)sulfamide trifluoroacetic acid;
Compound No. 143: 4-benzylphthalazin-1(2H)-one;
Compound No. 144: 4-benzyl-6,7-dimethoxyphthalazin-1(2H)-one;
Compound No. 145: tert-butyl (3-((6,7-dimethoxy-4-oxo-3,4-dihydrophthalazin-1-yl)methyl)phenyl)carbamate;
Compound No. 146: tert-butyl (3-((6,7-dimethoxy-3-methyl-4-oxo-3,4-dihydrophthalazin-1-yl)methyl)phenyl)carbamate;
Compound No. 147: tert-butyl (4-((4-oxo-3,4-dihydrophthalazin-1-yl)methyl)phenyl)carbamate;
Compound No. 148: tert-butyl (4-((3-methyl-4-oxo-3,4-dihydrophthalazin-1-yl)methyl)phenyl)carbamate;
Compound No. 149: tert-butyl (4-((6,7-dimethoxy-4-oxo-3,4-dihydrophthalazin-1-yl)methyl)phenyl)carbamate;
Compound No. 150: tert-butyl (4-((6,7-dimethoxy-3-methyl-4-oxo-3,4-dihydrophthalazin-1-yl)methyl)phenyl)carbamate;
Compound No. 151: tert-butyl (4-((4-oxo-3,4-dihydrophthalazin-1-yl)methyl)phenyl) (methyl) carbamate;
Compound No. 152: tert-butyl (4-((6,7-dimethoxy-4-oxo-3,4-dihydrophthalazin-1-yl)methyl)phenyl)(methyl)carbamate;
Compound No. 153: tert-butyl (4-((6,7-dimethoxy-3-methyl-4-oxo-3,4-dihydrophthalazin-1-yl)methyl)phenyl) (methyl) carbamate;
Compound No. 154: tert-butyl (5-((6,7-dimethoxy-4-oxo-3,4-dihydrophthalazin-1-yl)methyl)indoline-1-carboxylate;
Compound No. 155: tert-butyl (5-((6,7-dimethoxy-3-methyl-4-oxo-3,4-dihydrophthalazin-1-yl)methyl)indoline-1-carboxylate;
Compound No. 156: 4-(3-aminobenzyl)-6,7-dimethoxyphthalazin-1(2H)-one hydrochloride;
Compound No. 157: 4-(3-aminobenzyl)-6,7-dimethoxy-2-methylphthalazin-1(2H)-one hydrochloride;
Compound No. 158: 4-(4-aminobenzyl)-phthalazin-1(2H)-one hydrochloride;
Compound No. 159: 4-(4-aminobenzyl)-2-methylphthalazin-1(2H)-one hydrochloride;
Compound No. 160: 4-(4-aminobenzyl)-6,7-dimethoxyphthalazin-1(2H)-one hydrochloride;
Compound No. 161: 4-(4-aminobenzyl)-6,7-dimethoxy-2-methylphthalazin-1(2H)-one hydrochloride;
Compound No. 162: 4-(4-(methylamino)benzyl)phthalazin-1(2H)-one hydrochloride;
Compound No. 163: 6,7-dimethoxy-4-(4-(methylamino)benzyl)phthalazin-1(2H)-one hydrochloride;
Compound No. 164: 6,7-dimethoxy-2-methyl-4-(4-(methylamino)benzyl)phthalazin-1(2H)-one hydrochloride;
Compound No. 165: 4-(indolin-5-yl-methyl)-6,7-dimethoxyphthalazin-1(2H)-one hydrochloride;
Compound No. 166: 4-(indolin-5-yl-methyl)-6,7-dimethoxy-2-methylphthalazin-1(2H)-one hydrochloride;
Compound No. 167: tert-butyl (N-(3-((6,7-dimethoxy-4-oxo-3,4-dihydrophthalazin-1-yl)methyl)phenyl)sulfamoyl)carbamate;
Compound No. 168: tert-butyl (N-(3-((6,7-dimethoxy-3-methyl-4-oxo-3,4-dihydrophthalazin-1-yl)methyl)phenyl)sulfamoyl)carbamate;
Compound No. 169: tert-butyl (N-(4-((4-oxo-3,4-dihydrophthalazin-1-yl)methyl)phenyl)sulfamoyl)carbamate;
Compound No. 170: tert-butyl (N-(4-((3-methyl-4-oxo-3,4-dihydrophthalazin-1-yl)methyl)phenyl)sulfamoyl)carbamate;
Compound No. 171: tert-butyl (N-(4-((6,7-dimethoxy-4-oxo-3,4-dihydrophthalazin-1-yl)methyl)phenyl)sulfamoyl)carbamate;
Compound No. 172: tert-butyl (N-(4-((6,7-dimethoxy-3-methyl-4-oxo-3,4-dihydrophthalazin-1-yl)methyl)phenyl)sulfamoyl)carbamate;
Compound No. 173: tert-butyl N-methyl-N-(4-((4-oxo-3,4-dihydrophthalazin-1-yl)methyl)phenyl)sulfamoyl carbamate;
Compound No. 174: tert-butyl N-(4-((6,7-dimethoxy-4-oxo-3,4-dihydrophthalazin-1-yl)methyl)phenyl)-N-methylsulfamoyl carbamate;
Compound No. 175: tert-butyl N-(4-((6,7-dimethoxy-3-methyl-4-oxo-3,4-dihydrophthalazin-1-yl)methyl)phenyl)-N-methylsulfamoyl carbamate;
Compound No. 176: tert-butyl (5-((6,7-dimethoxy-4-oxo-3,4-dihydrophthalazin-1-yl)methyl)indolin-1-yl)sulfonyl carbamate;
Compound No. 177: tert-butyl (5-((6,7-dimethoxy-3-methyl-4-oxo-3,4-dihydrophthalazin-1-yl)methyl)indolin-1-yl)sulfonyl carbamate;
Compound No. 178: N-(3-((6,7-dimethoxy-4-oxo-3,4-dihydrophthalazin-1-yl)methyl)phenyl)sulfamide hydrochloride;
Compound No. 179: N-(3-((6,7-dimethoxy-3-methyl-4-oxo-3,4-dihydrophthalazin-1-yl)methyl)phenyl)sulfamide hydrochloride;
Compound No. 180: N-(4-((4-oxo-3,4-dihydrophthalazin-1-yl)methyl)phenyl)sulfamide hydrochloride;
Compound No. 181: N-(4-((3-methyl-4-oxo-3,4-dihydrophthalazin-1-yl)methyl)phenyl)sulfamide hydrochloride;
Compound No. 182: N-(4-((6,7-dimethoxy-4-oxo-3,4-dihydrophthalazin-1-yl)methyl)phenyl)sulfamide hydrochloride;
Compound No. 183: N-(4-((6,7-dimethoxy-3-methyl-4-oxo-3,4-dihydrophthalazin-1-yl)methyl)phenyl)sulfamide hydrochloride;
Compound No. 184: N-methyl-N-(4-((4-oxo-3,4-dihydrophthalazin-1-yl)methyl)phenyl)sulfamide hydrochloride;
Compound No. 185: N-(4-((6,7-dimethoxy-4-oxo-3,4-dihydrophthalazin-1-yl)methyl)phenyl)-N-methylsulfamide hydrochloride;
Compound No. 186: N-(4-((6,7-dimethoxy-3-methyl-4-oxo-3,4-dihydrophthalazin-1-yl)methyl)phenyl)-N-methylsulfamide hydrochloride;
Compound No. 187: 5-((6,7-dimethoxy-4-oxo-3,4-dihydrophthalazin-1-yl)methyl)indoline-1-sulfonamide hydrochloride;
Compound No. 188: 5-((6,7-dimethoxy-3-methyl-4-oxo-3,4-dihydrophthalazin-1-yl)methyl)indoline-1-sulfonamide hydrochloride;
Compound No. 189: N-(3-fluoro-4-((4-oxo-3,4-dihydrophthalazin-1-yl)methyl)phenyl)sulfamide hydrochloride;
Compound No. 190: N-(2-fluoro-4-((4-oxo-3,4-dihydrophthalazin-1-yl)methyl)phenyl)sulfamide hydrochloride;
Compound No. 191: N-(3-(trifluorobenzyl methyl)-4-((4-oxo-3,4-dihydrophthalazin-1-yl)methyl)phenyl)sulfamide hydrochloride;
Compound No. 192: N-(2-(trifluorobenzyl methyl)-4-((4-oxo-3,4-dihydrophthalazin-1-yl)methyl)phenyl)sulfamide hydrochloride;
Compound No. 193: 4-(3-bromobenzyl)-6,7-dimethoxyphthalazin-1(2H)-one;
Compound No. 194: 4-(4-bromobenzyl)-6,7-dimethoxyphthalazin-1(2H)-one;
Compound No. 195: N-(4-(4-oxo-3,4-dihydrophthalazin-1-yl)benzyl)methanesulfonamide;
Compound No. 196: N-(4-(4-oxo-3,4-dihydrophthalazin-1-yl)benzyl)cyclopropanesulfonamide;
Compound No. 197: 4-methyl-N-(4-(4-oxo-3,4-dihydrophthalazin-1-yl)benzyl)benzenesulfonamide;
Compound No. 198: N-(4-(4-oxo-3,4-dihydrophthalazin-1-yl)benzyl)thiophene-2-sulfonamide;
Compound No. 199: (4-(3-methyl-4-oxo-3,4-dihydrophthalazin-1-yl)phenyl)sulfamide;
Compound No. 200: N-methyl-1-(4-(3-methyl-4-oxo-3,4-dihydrophthalazin-1-yl)phenyl)methanesulfonamide;
Compound No. 201: 2-methyl-4-(4-((morpholinosulfonyl)methyl)phenyl)phthalazin-1(2H)-one;
Compound No. 202: 2-(4-(3-methyl-4-oxo-3,4-dihydrophthalazin-1-yl)phenyl)ethane-1-sulfonamide; and
Compound No. 203: 4-(4-(aminomethyl)-3-(trifluoromethyl)phenyl-6,7-dimethoxy phthalazin-1(2H)-one hydrochloride.

6. The compound according to claim 1, wherein the pharmaceutically acceptable salt is a salt of an inorganic or organic acid selected from the group consisting of hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, hydroxymaleic acid, benzoic acid, hydroxybenzoic acid, phenylacetic acid, cinnamic acid, salicylic acid, methanesulfonic acid, benzenesulfonic acid, and toluenesulfonic acid.

7. A pharmaceutical composition for preventing, alleviating or treating cancer comprising the compound according to any one of claims 1 to 6 as an active ingredient.

8. An ENPP 1 inhibitor comprising the compound according to any one of claims 1 to 6 as an active ingredient.

9. A STING pathway activator comprising the compound according to any one of claims 1 to 6 as an active ingredient.

10. The pharmaceutical composition according to claim 7, wherein the cancer is associated with inhibition of ENPP 1.
